# EUROPEAN PATENT SPECIFICATION

(11) **EP 4 076 382 B1**
(45) Date of publication and mention of the grant of the patent: **20.03.2024**
(21) Application number: 20780762.9
(22) Date of filing: 02.10.2020
(51) Int. Cl.: A61K 9/00, A61K 9/70, A61K 31/165, A61K 47/32, A61P 25/24

(54) **TRANSDERMAL THERAPEUTIC SYSTEM CONTAINING AGOMELATINE**
TRANSDERMALES THERAPEUTISCHES SYSTEM MIT AGOMELATIN
SYSTÈME THÉRAPEUTIQUE TRANSDERMIQUE CONTENANT DE L'AGOMÉLATINE

(30) Priority: 20.12.2019 EP 19218599
(43) Date of publication of application: 26.10.2022
(73) Proprietor: LTS Lohmann Therapie-Systeme AG, 56626 Andernach (DE)
(72) Inventor: MOHR, Patrick, 53498 Bad Breisig (DE); RIETSCHER, René, 56626 Andernach (DE); EIFLER, René, 56072 Koblenz (DE); BOURQUAIN, Olga, 56307 Dürrholz (DE)
(74) Representative: Maiwald GmbH
(86) International application number: PCT/EP2020/077737
(87) International publication number: WO 2020/260727

(56) References cited:
- WO-A1-2014/024896
- CN-A- 110 151 735
- US-A1- 2016 184 246

## Description

### TECHNICAL FIELD OF THE INVENTION

The present invention relates to a transdermal therapeutic system (TTS) for the transdermal administration of agomelatine to the systemic circulation, and processes of manufacture and transdermal therapeutic systems for uses thereof.

### BACKGROUND OF THE INVENTION

The active agent agomelatine (N-(2-(7-Methoxy-1-naphthyl)ethyl)acetamide) is a melatonergic antidepressant developed by Les Laboratoires Servier. The chemical structure is very similar to that of melatonin.

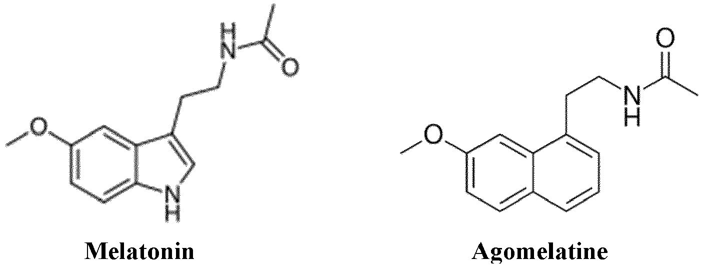

As a melatonergic agonist stimulating the MT1 and MT2 receptors, agomelatine is able to mediate synchronization of the circadian rhythm, much like melatonin. However, in addition and in contrast to melatonin, agomelatine is also a 5-HT2B / 5-HT2C antagonist, and blocking the serotonergic 5HT2C receptors causes enhanced release of dopamine and norepinephrine in the prefrontal cortex. Unexpected synergistic actions have been observed for the MT1/MT2 agonism and 5HT2C antagonism, and it is supposed that this synergy accounts for the antidepressant actions and unique clinical profile of agomelatine.

Agomelatine has been approved in Europe under the trade names Valdoxan^{®}, Melitor^{®} and Thymanax^{®} and is indicated for the treatment of major depressive disorder (MDD). The currently available form is a film tablet containing a dose of 25 mg, which is prescribed with an initial dose of one tablet to be taken at bedtime, with the option of doubling the dose if no improvement is seen. Agomelatine is the only antidepressant on the market with the mechanism of action described above

Oral agomelatine undergoes extensive first pass and systemic metabolism, mainly *via* the cytochrome CYP1A2. Although agomelatine is well absorbed orally (> 80 %), overall bioavailability is very low (less than 5 %), with pronounced inter-individual variability. Both the time to reach maximal blood plasma concentration as well as elimination half-life t_{1/2} is about 1 to 2 hours, and in steady state, the volume of distribution is 35 liters, with a plasma protein binding of 95 %.

When compared to other antidepressants, agomelatine seems to have a more rapid onset of effects (usually within a week), and major side effects commonly known for other antidepressants such as weight gain, sexual dysfunction, anticholinergic symptoms and cardiotoxicity appear to be reduced. However, agomelatine bears the risk of hepatotoxicity of which the mechanism remains unclarified, manifesting as increased alanine aminotransferase (ALAT) and/or aspartate aminotransferase (ASAT) values, and in few exceptional cases, the outcome was fatal or necessitated liver transplantation. In addition, hepatic impairment was also reported to be related to a huge increase in agomelatine exposure, with up to 140-fold AUC and cₘₐₓ values observed for patients with moderate hepatic impairment compared to healthy subjects.

Efforts were made by Servier and Novartis to establish a sublingual dosage form of agomelatine, supposedly in order to avoid the first-pass metabolism and the associated drawbacks as outlined above (low oral bioavailability and hepatotoxicity), resulting in placebo controlled randomized studies with 1 and 2 mg (Servier) or 0.5 and 1 mg agomelatine sublingual tablets (Novartis). No result is publicly available for the 2008/2009 Servier trial. In the Novartis studies, commenced in 2011/2011, the efficacy of the agomelatine sublingual tablets was not better than placebo, and there was no clear dose response relationship, though at least liver toxicity was shown to be rare. One of the reasons for such results appears to have been the pronounced irritant sensation caused by agomelatine when administered to the oral mucosa. As a consequence, the FDA decided not to grant approval of the drug in the USA, despite the advantages over other actives in the treatment of MDD.

Transdermal administration of agomelatine would not only avoid the first-pass metabolism and the associated disadvantages of oral administration, but also the irritant sensation induced by sublingual tablets. In addition, the bioavailability of an agomelatine transdermal therapeutic systems should be higher, and thus, subject to whether an increase in systemic delivery of the active when compared to the oral dosage forms can be shown in practice, it will be possible to reduce the dose, which will not only enhance cost-efficiency but also address dose-related issues such as hepatotoxicity. Transdermal delivery of agomelatine has been investigated, but it appears that formulating appropriate dosage forms for the passive transdermal delivery of agomelatine is challenging.

Passive transport of active agents from a transdermal therapeutic system (TTS) through the skin makes use of the driving force based on the concentration gradient between the concentration of active agent in the transdermal system and on the outer surface of the skin and the concentration in the blood stream. Such passive transport is advantageous in view of complexity of the TTS and the convenience of administration compared to TTS making use of active transportation such as iontophoresis or microporation. However, for creating such a driving force, a balance has to be found between a sufficiently high drug concentration in the TTS, and an excessive solubility of the drug in the active-containing layer, as the latter will work against high skin permeation rates. Agomelatine is a poorly soluble drug, and several different polymorphic forms of agomelatine are known. In order to prevent re-crystallization of the drug upon storage, which is undesirable since it may influence the TTS performance due to crystallization of the drug in unpredictable forms and thus may lead to reduced shelf-life, and also in order to achieve sufficiently high drug concentration, the formulation has to be such that solubility of agomelatine is high enough. This requirement has to be balanced against sufficient skin permeation of the drug.

In addition, since agomelatine is mainly used for re-synchronizing the circadian rhythm, the desired drug release profile is a rapid initial increase followed by an only overnight release, in contrast to the continuous and steady drug exposure sought after (and often achieved by TTS formulations) for an around-the-clock treatment of chronic diseases.

Agomelatine TTS formulations comprising isopropanol have been suggested, but isopropanol is a volatile solvent and thus, the composition of formulations comprising isopropanol are expected to change over time due to evaporation of the solvent. The overall delivered amount also seems to be low for such formulations. Yet other formulations relying on fatty acid-based ionic liquids appear to have been investigated, but the drug concentration of these formulations were very low, and as a consequence, it seems that the layer thickness had to be increased to such dimensions that TTS manufacture would be impractical on large /industrial scale. Patent document WO 2014/024896 discloses transdermal therapeutic systems comprising agomelatine.

Up to date, no commercial agomelatine TTS is available.

In summary, alternative administration forms of agomelatine are much needed, overcoming the disadvantages of the oral as well as sublingual administration routes. As outlined above, a TTS would be able to address these disadvantages.

There is thus a need in the art for an agomelatine TTS with sufficiently high skin permeation of the drug.

### OBJECTS AND SUMMARY OF THE INVENTION

It is an object of the present invention to provide an agomelatine TTS overcoming the above-mentioned disadvantages of current agomelatine administration.

Thus, it is an object of the present invention to provide a TTS for the transdermal administration of agomelatine providing a permeation rate which is sufficient for achieving a therapeutically effective dose.

It is a further object of the present invention to provide a TTS for the transdermal administration of agomelatine providing a drug release profile with a rapid initial increase and allowing an overnight application, e.g. appropriate for an administration shortly before going to bed, and removal of the TTS in the morning.

It is also an object of the present invention to provide a TTS for the transdermal administration of agomelatine with sufficient storage stability, with respect to active agent stability as well as stability of the composition and/or of the drug release profile, preventing in particular (re-)crystallization of the active.

It is another object of the present invention to provide a TTS for the transdermal administration of agomelatine, wherein hepatotoxicity and the inter-individual variability is reduced and the bioavailability is increased when compared to oral administration.

It is further an object of the present invention to provide a TTS for the transdermal administration of agomelatine which complies with the needs of a convenient application in view of size and thickness, provides for good patient compliance and/or which is easy and cost-efficient to manufacture

These objects and others are accomplished by the present invention, which according to one aspect relates to a transdermal therapeutic system for the transdermal administration of agomelatine comprising a self-adhesive layer structure containing a therapeutically effective amount of agomelatine, said self-adhesive layer structure comprising:
A) a backing layer; and
B) an agomelatine-containing layer comprising:
   i) agomelatine;
   ii) a hydrophobic polymer; and
   iii) at least 1 wt-% of a crystallization inhibitor selected from the group consisting of polyvinylpyrrolidone and polyvinylpyrrolidone-polyvinylacetate copolymer;
   wherein
   the hydrophobic polymer is selected from pressure-sensitive adhesives based on polysiloxanes.

According to another aspect, the present invention relates to a transdermal therapeutic system for the transdermal administration of agomelatine wherein the agomelatine-containing layer is of a microreservoir-type.

According to certain embodiments of the invention, the transdermal therapeutic system according to the invention is for use in a method of treatment, preferably for use in a method of treating major depression.

Disclosed, but not claimed, is the use of the transdermal therapeutic system for the manufacture of a medicament for a treatment, preferably for treating major depression.

According to yet another aspect, the invention relates to a process of manufacture of an agomelatine-containing layer comprising the steps of comprising the steps of:
i) combining at least agomelatine, a hydrophobic polymer, and a crystallization inhibitor selected from the group consisting of polyvinylpyrrolidone and polyvinylpyrrolidone-polyvinylacetate copolymer in a solvent to obtain a coating composition;
ii) coating the coating composition onto a backing layer or a release liner or any intermediate liner; and
iii) drying the coated coating composition to form the agomelatine-containing layer, wherein the hydrophobic polymer is selected from pressure-sensitive adhesives based on polysiloxanes.

Disclosed, but not claimed, is a transdermal therapeutic system for the transdermal administration of agomelatine obtainable by such a process of manufacture.

According to certain embodiments, the invention also relates to a transdermal therapeutic system for the transdermal administration of agomelatine comprising a self-adhesive layer structure containing a therapeutically effective amount of agomelatine, said self-adhesive layer structure comprising:
A) a backing layer; and
B) an agomelatine-containing layer comprising:
   i) 2 to 6 wt-% agomelatine;
   ii) a hydrophobic polymer;
   iii) from 2 to 7 wt-% polyvinylpyrrolidone; and
   iv) from 2 to 7 wt-% of a permeation enhancer selected from levulinic acid and polyethylene glycol ethers;
wherein
the hydrophobic polymer is selected from pressure-sensitive adhesives based on polysiloxanes. and
wherein the area weight of the agomelatine-containing layer ranges from 35 to 70 g/m².

According to other embodiments, the invention relates to a transdermal therapeutic system for the transdermal administration of agomelatine comprising a self-adhesive layer structure containing a therapeutically effective amount of agomelatine, said self-adhesive layer structure comprising:
A) a backing layer; and
B) an agomelatine-containing layer comprising:
   i) 2 to 6 wt-% agomelatine;
   ii) a hydrophobic polymer; and
   iii) from 7 to 15 wt-% polyvinylpyrrolidone;
wherein
the hydrophobic polymer is selected from pressure-sensitive adhesives based on polysiloxanes. and
wherein the area weight of the agomelatine-containing layer ranges from 35 to 70 g/m².

Within the meaning of this invention, the term "transdermal therapeutic system" (TTS) refers to a system by which the active agent (agomelatine) is administered to the systemic circulation *via* transdermal delivery and refers to the entire individual dosing unit that is applied to the skin of a patient, and which comprises a therapeutically effective amount of agomelatine in a self-adhesive layer structure and optionally an additional adhesive overlay on top of the agomelatine-containing self-adhesive layer structure. The self-adhesive layer structure may be located on a release liner (a detachable protective layer), thus, the TTS may further comprise a release liner. Within the meaning of this invention, the term "TTS" in particular refers to a system providing passive transdermal delivery excluding active transport as in methods including iontophoresis or microporation.

Within the meaning of this invention, the term "agomelatine-containing self-adhesive layer structure" or "self-adhesive layer structure containing a therapeutically effective amount of agomelatine" refers to the active agent-containing structure providing the area of release for agomelatine during administration. The adhesive overlay adds to the overall size of the TTS but does not add to the area of release. The agomelatine-containing self-adhesive layer structure comprises a backing layer and at least one agomelatine-containing layer.

Within the meaning of this invention, the term "therapeutically effective amount" refers to a quantity of active agent in the TTS sufficient to provide, if administered by the TTS to a patient, agomelatine blood levels of a similar range (e.g. of about 10 % to about 1000 % as measured as an AUC) when compared to blood levels obtained in a one-time administration of 25 mg oral agomelatine. A TTS usually contains more active in the system than is in fact provided to the skin and the systemic circulation. This excess amount of active agent is usually necessary to provide enough driving force for the passive transportation from the TTS to the systemic circulation.

Within the meaning of this invention, the terms "active", "active agent", and the like, as well as the term "agomelatine" refer to agomelatine in any pharmaceutically acceptable chemical and morphological form and physical state. These forms include without limitation agomelatine in its free, dissociated or any associated form such as hydrates, solvates and so on, as well as agomelatine in the form of particles which may be in micronized form, crystalline form, and in particular in one of its polymorph forms, and/or in amorphous form, and in any hybrid type form of any of the aforementioned forms or a mixture thereof. The agomelatine, where contained in a medium such as a solvent, may be dissolved or dispersed or in part dissolved and in part dispersed.

When agomelatine is mentioned to be used in a particular form in the manufacture of the TTS, this does not exclude interactions between this form of agomelatine and other ingredients of the agomelatine-containing self-adhesive layer structure so that the active is present in another form in the final TTS. This means that, even if agomelatine is included in a free, dissociated form, it may be present in the final TTS in the form of a hydrate or a solvate, or, if it is included in one of its polymorph forms, it may be present in amorphous form in the final TTS. Unless otherwise indicated, in particular the amount of agomelatine in the self-adhesive layer structure relates to the amount of agomelatine included in the TTS during manufacture of the TTS and is calculated based on agomelatine in the free form, i.e. when agomelatine is included in an amount of 0.1 mmol, the amount of agomelatine in the self-adhesive layer structure is, within the meaning of the invention, considered to be 24.3 mg (the molecular weight of agomelatine is 243 g/mol), regardless of whether the agomelatine has been included in the TTS during manufacture in its free form or in any associated form.

The agomelatine starting material included in the TTS during manufacture of the TTS may be in the form of particles. Agomelatine may e.g. be present in the self-adhesive layer structure in the form of particles and/or dissolved.

Within the meaning of this invention, the term "particles" refers to a solid, particulate material comprising individual particles, the dimensions of which are negligible compared to the material. In particular, the particles are solid, including plastic/deformable solids, including amorphous and crystalline materials.

Within the meaning of this invention, the term "dispersing" refers to a step or a combination of steps wherein a starting material (e.g. agomelatine) is not totally dissolved. Dispersing in the sense of the invention comprises the dissolution of a part of the starting material (e.g. agomelatine particles), depending on the solubility of the starting material (e.g. the solubility of agomelatine in the coating composition).

There are two main types of TTS using passive active agent delivery, i.e. matrix-type TTS and reservoir-type TTS. In matrix-type TTS the active agent is included in a matrix, while in a reservoir-type TTS the active agent is included in a liquid or semi-liquid reservoir. So-called microreservoir-type TTS are considered in the art to be a mixture between a matrix-type TTS and a reservoir-type TTS. The release of the active agent in a matrix-type TTS is mainly controlled by the matrix including the active agent itself. In contrast thereto, a reservoir-type TTS needs a rate-controlling membrane controlling the release of the active agent. Matrix-type TTS are advantageous in that, compared to reservoir type TTS, usually no rate determining membranes are necessary and no dose dumping can occur due to membrane rupture. In summary, matrix-type transdermal therapeutic systems (TTS) are less complex in manufacture and easy and convenient to use by patients. Microreservoir-type TTS also do not need rate determining membranes, but in contrast to "classical" matrix-type TTS, the drug release profile is often characterized by a faster onset and a higher utilization of active, which is often advantageous.

Within the meaning of this invention, "matrix-type TTS" refers to a system or structure wherein the active is homogeneously dissolved and/or dispersed within a polymeric carrier, i.e. the matrix, which forms with the active agent and optionally remaining ingredients a matrix layer. In such a system, the matrix layer controls the release of the active agent from the TTS. A matrix-type TTS may also include a rate-controlling membrane. Matrix-type TTS may in particular be in the form of a "drug-in-adhesive"-type TTS referring to a system wherein the active is homogeneously dissolved and/or dispersed within a pressure-sensitive adhesive matrix.

TTS with a rate-controlling membrane and a liquid or semi-liquid active agent containing reservoir, wherein the release of the active agent from the TTS is controlled by the rate-controlling membrane, are referred to by the term "reservoir-type TTS". Reservoir-type TTS are not to be understood as being of matrix-type within the meaning of the invention.

Within the meaning of this invention, "microreservoir-type TTS" refers to a system or structure wherein the active agent-containing layer is a biphasic layer having an inner active-containing phase in an outer matrix-phase. Herein, the term "biphasic" refers to a system of two distinguishable, e.g., visually distinguishable, areas, an outer phase and an inner phase, wherein the inner phase is in form of dispersed deposits within the outer phase. Such deposits are e.g., solid solution droplets. Deposits that are visually distinguishable may be identified by use of a microscope.

Within the meaning of this invention, the term "matrix layer" or "layer of a matrix-type" refers to any layer containing the active homogeneously dissolved and/or dispersed within a polymeric carrier. Typically, a matrix layer is present in a matrix-type TTS as the active agent-containing layer. A reservoir-type TTS may comprise, in addition to a reservoir layer and a rate-controlling membrane, an additional adhesive layer which serves as a skin contact layer. In such a reservoir-type TTS, the additional adhesive layer often is manufactured as an active agent-free layer. However, due to the concentration gradient, the active agent will migrate from the reservoir to the additional adhesive layer over time, until an equilibrium is reached. Therefore, in such a reservoir-type TTS, after some time of equilibration, the additional adhesive layer contains the active agent and is to be regarded as an active agent-containing layer in the sense of the present invention.

The active agent-containing layer is the final, solidified layer e.g. obtained after coating and drying the solvent-containing coating composition. The active agent-containing layer may also be manufactured by laminating two or more such solidified layers (e.g. dried layers) of the same composition to provide the desired area weight. The active agent-containing layer may be self-adhesive (in the form of a pressure sensitive adhesive layer) or the TTS may comprise an additional skin contact layer of a pressure sensitive adhesive for providing sufficient tack. In particular, the active agent-containing layer is a pressure sensitive adhesive layer.

Within the meaning of this invention, the term "biphasic layer" refers to the final biphasic layer of a microreservoir-type TTS solidified after coating the coating mixture by e.g. drying a solvent-containing coating mixture or cooling a hot-melt coating mixture. Solvent-containing coating mixtures are preferred according to the invention. The biphasic layer may also be manufactured by laminating two or more layers (e.g. dried layers) of the same composition to provide the desired area weight.

Within the meaning of this invention, the term "dried biphasic layer" refers to a biphasic layer obtained from a solvent-containing coating mixture after coating on a film and evaporating the solvents (solvent-based layer) and is to be distinguished from a biphasic layer obtained from a hot-melt coating mixture (hot-melt-based layer).

Within the meaning of this invention, the term "pressure-sensitive adhesive" refers to a material that in particular adheres with finger pressure, is permanently tacky, exerts a strong holding force and should be removable from smooth surfaces without leaving a residue. A pressure sensitive adhesive layer, when in contact with the skin, is "self-adhesive", i.e. provides adhesion to the skin so that typically no further aid for fixation on the skin is needed. A "self-adhesive" layer structure includes a pressure sensitive adhesive layer for skin contact which may be provided in the form of a pressure sensitive adhesive active agent-containing layer or in the form of an additional layer, i.e. a pressure sensitive adhesive skin contact layer. An adhesive overlay may still be employed to advance adhesion.

Within the meaning of this invention, the term "skin contact layer" refers to a layer included in the TTS to be in direct contact with the skin of the patient during administration. When the TTS comprises a skin contact layer, the other layers do not contact the skin and do not necessarily have self-adhesive properties. As outlined above, the skin contact layer may over time absorb parts of the active agent and then may be regarded as an active agent-containing layer. The area of release is provided by the area of the active agent-containing layer. A skin contact layer may be used to enhance adherence. The sizes of an additional skin contact layer and the active agent-containing layer are usually coextensive and correspond to the area of release.

Within the meaning of this invention, the term "area weight" refers to the dry weight of a specific layer, e.g. of the active agent-containing layer, provided in g/m². The area weight values are subject to a tolerance of ± 10 %, preferably ± 7.5 %, due to manufacturing variability.

If not indicated otherwise "%" refers to weight-%.

Within the meaning of this invention, the term "polymer" refers to any substance consisting of so-called repeating units obtained by polymerizing one or more monomers, and includes homopolymers which consist of one type of monomer and copolymers which consist of two or more types of monomers. Polymers may be of any architecture such as linear polymers, star polymer, comb polymers, brush polymers, of any monomer arrangements in case of copolymers, e.g. alternating, statistical, block copolymers, or graft polymers. The minimum molecular weight varies depending on the polymer type and is known to the skilled person. Polymers may e.g. have a molecular weight above 2,000, preferably above 5,000 and more preferably above 10,000 Dalton. Correspondingly, compounds with a molecular weight below 2,000, preferably below 5,000 or more preferably below 10,000 Dalton are usually referred to as oligomers.

Within the meaning of this invention, the term "cross-linking agent" refers to a substance which is able to cross-link functional groups contained within the polymer.

Within the meaning of this invention, the term "adhesive overlay" refers to a self-adhesive layer structure that is free of active agent and larger in area than the active agent-containing structure and provides additional area adhering to the skin, but no area of release of the active agent. It enhances thereby the overall adhesive properties of the TTS. The adhesive overlay comprises a backing layer and an adhesive layer.

Within the meaning of this invention, the term "backing layer" refers to a layer, which supports e.g. the agomelatine-containing layer or forms the backing of the adhesive overlay. At least one backing layer in the TTS and usually the backing layer of the agomelatine-containing layer is occlusive, i.e. substantially impermeable to the active agent contained in the layer during the period of storage and administration and thus prevents active loss or cross-contamination in accordance with regulatory requirements.

The TTS according to the present invention can be characterized by certain parameters as measured in an *in vitro* skin permeation test.

The *in vitro* permeation test is performed in a Franz diffusion cell, with human or animal skin and preferably with dermatomed split-thickness human skin with a thickness of 800 µm and an intact epidermis, and with phosphate buffer pH 5.5 or 7.4 as receptor medium (32 °C with 0.1 % saline azide) with or without addition of a maximum of 40 vol-% organic solvent e.g. ethanol, acetonitrile, isopropanol, dipropylene glycol, PEG 400 so that a receptor medium may e.g. contain 60 vol-% phosphate buffer pH 5.5, 30 vol-% dipropylene glycol and 10 vol-% acetonitrile.

Where not otherwise indicated, the *in vitro* permeation test is performed with dermatomed split-thickness human skin with a thickness of 800 µm and an intact epidermis, and with phosphate buffer pH 5.5 as receptor medium (32 °C with 0.1 % saline azide). The amount of active permeated into the receptor medium is determined in regular intervals using a validated HPLC method with a UV photometric detector by taking a sample volume. The receptor medium is completely or in part replaced by fresh medium when taking the sample volume, and the measured amount of active permeated relates to the amount permeated between the two last sampling points and not the total amount permeated so far.

Thus, within the meaning of this invention, the parameter "permeated amount" is provided in µg/cm² and relates to the amount of active permeated in a sample interval at certain elapsed time. E.g., in an *in vitro* permeation test as described above, wherein the amount of active permeated into the receptor medium has been e.g. measured at hours 0, 2, 4, 8, 12 and 24, the "permeated amount" of active can be given e.g. for the sample interval from hour 8 to hour 12 and corresponds to the measurement at hour 12.

The permeated amount can also be given as a "cumulative permeated amount", corresponding to the cumulated amount of active permeated at a certain point in time. E.g., in an *in vitro* permeation test as described above, wherein the amount of active permeated into the receptor medium has been e.g. measured at hours 0, 2, 4, 8, 12 and 24, the "cumulative permeated amount" of active at hour 12 corresponds to the sum of the permeated amounts from hour 0 to hour 2, hour 2 to hour 4, hour 4 to hour 8 and hour 8 to hour 12.

Within the meaning of this invention, the parameter "skin permeation rate" for a certain sample interval at certain elapsed time is provided in µg/(cm² h) and is calculated from the permeated amount in said sample interval as measured by *in vitro* permeation test as described above in µg/cm², divided by the hours of said sample interval. E.g. the skin permeation rate in an *in vitro* permeation test as described above, wherein the amount of active permeated into the receptor medium has been e.g. measured at hours 0, 2, 4, 8, 12 and 24, the "skin permeation rate" at hour 12 is calculated as the permeated amount in the sample interval from hour 8 to hour 12 divided by 4 hours.

A "cumulative skin permeation rate" can be calculated from the respective cumulative permeated amount by dividing the cumulative permeated amount by the elapsed time. E.g. in an *in vitro* permeation test as described above, wherein the amount of active permeated into the receptor medium has been e.g. measured at hours 0, 2, 4, 8, 12 and 24, the "cumulative skin permeation rate" at hour 12 is calculated as the cumulative permeated amount for hour 12 (see above) divided by 12 hours.

Within the meaning of this invention, the above parameters permeated amount and skin permeation rate (as well as cumulative permeated amount and cumulative skin permeation rate) refer to mean values calculated from 3 *in vitro* permeation test experiments.

The TTS according to the present invention can also be characterized by certain parameters as measured in an *in vivo* clinical study.

Within the meaning of this invention, the term "administration" refers to the application of the dosage form, i.e. the TTS, to the skin of the patient, which is then maintained on the skin for a certain period of time.

In a typical continuous treatment of MDD, the frequency of drug administration is kept sufficiently high so as to maintain a therapeutically effective blood plasma concentration. The interval between two dosage form administrations, also called dosing interval, needs to be adapted accordingly. Within the meaning of the present invention, the term "dosing interval" refers to the period of time between two consecutive TTS administrations, i.e. the interval between two consecutive points in time a TTS is applied to the skin of the patient. In order to maintain the blood plasma concentration at therapeutic level, the TTS is usually maintained on the skin of the patient for the entire dosing interval and only removed at the end of the dosing interval, at which time a new TTS is applied to the skin. E.g., if the dosing interval is 168 hours or 7 days, the TTS is applied to and maintained on the skin of the patient for 168 hours or 7 days. After 168 hours or 7 days, the TTS is removed from the skin and a new TTS is applied. Thus, a dosing interval of 168 hours or 7 days allows a once-a-week TTS exchange mode in an around-the-clock treatment.

For a continuous treatment with agomelatine, the TTS will be usually administered once daily (dosing interval of 24 hours), and preferably at bedtime. The TTS may in particular be applied to the skin of the patient shortly (e.g. 1-2 hours) before going to bed in order to account for the delay in drug onset, and maintained on the skin up to 24 hours. If the TTS is maintained on the skin for 24 hours, the TTS can be removed and a new TTS can be applied at the same time. Since it appears for agomelatine that an around-the-clock maintenance of blood plasma concentration at therapeutic level is not necessary, or may be even contraindicated for resynchronization of the circadian rhythm, it is possible to remove the TTS during daytime, so that the patient does not wear any TTS thereafter during daytime. In an overnight-only application, the TTS is maintained on the skin only during nighttime, e.g. for a time period of between 4 and 12 hours, or between 6 and 10 hours, or during sleep, depending on the patient's length of sleep, and is then removed in the morning.

Within the meaning of this invention, the term "room temperature" refers to the unmodified temperature found indoors in the laboratory where the experiments are conducted and usually lies within 15 to 35 °C, preferably about 18 to 25 °C.

Within the meaning of this invention, the term "patient" refers to a subject who has presented a clinical manifestation of a particular symptom or symptoms suggesting the need for treatment, who is treated preventatively or prophylactically for a condition, or who has been diagnosed with a condition to be treated.

Clinical studies according to the present invention refer to studies performed in full compliance with the International Conference for Harmonization of Clinical Trials (ICH) and all applicable local Good Clinical Practices (GCP) and regulations.

Within the meaning of this invention, the term "coating composition" refers to a composition comprising all components of the drug-containing layer in a solvent, which may be coated onto the backing layer or release liner to form the drug-containing layer upon drying.

Within the meaning of this invention, the term "dissolve" refers to the process of obtaining a solution, which is clear and does not contain any particles, as visible to the naked eye.

Within the meaning of this invention, the term "solvent" refers to any liquid substance, which preferably is a volatile organic liquid such as methanol, ethanol, isopropanol, acetone, ethyl acetate, methylene chloride, hexane, n-heptane, heptanes, toluene and mixtures thereof.

Within the meaning of this invention, and unless otherwise specified, the term "about" refers to an amount that is ± 10 % of the disclosed amount. In some embodiments, the term "about" refers to an amount that is ± 5 % of the disclosed amount. In some embodiments, the term "about" refers to an amount that is ± 2 % of the disclosed amount.

### BRIEF DESCRIPTION OF THE DRAWINGS

Fig. 1a depicts the agomelatine skin permeation rate of TTS prepared according to Examples 1a, 1b and 1c for hours 0 to 24.
Fig. 1b depicts the utilization of agomelatine of TTS prepared according to Examples 1a, 1b and 1c after 8 hours.
Fig. 1c depicts the utilization of agomelatine of TTS prepared according to Examples 1a, 1b and 1c after 24 hours.
Fig. 2a depicts the agomelatine skin permeation rate of TTS prepared according to Examples 2a, 2b, 2c, 2d and 2e for hours 0 to 24.
Fig. 2b depicts the utilization of agomelatine of TTS prepared according to Examples 2a, 2b, 2c, 2d and 2e after 8 hours.
Fig. 2c depicts the utilization of agomelatine of TTS prepared according to Examples 2a, 2b, 2c, 2d and 2e after 24 hours.
Fig. 3a depicts the agomelatine skin permeation rate of TTS prepared according to Examples 2e, 3a, 3b, 3c and 3d for hours 0 to 24.
Fig. 3b depicts the utilization of agomelatine of TTS prepared according to Examples 2e, 3a, 3b, 3c and 3d after 8 hours.
Fig. 3c depicts the utilization of agomelatine of TTS prepared according to Examples 2e, 3a, 3b, 3c and 3d after 24 hours.
Fig. 3d depicts a microscopic picture of the agomelatine-containing layer of a TTS prepared according to Example 3d.
Fig. 4a depicts the agomelatine skin permeation rate of TTS prepared according to Examples 4a, 4b, 4c, 4d and 4e for hours 0 to 24.
Fig. 4b depicts the utilization of agomelatine of TTS prepared according to Examples 4a, 4b, 4c, 4d and 4e after 8 hours.
Fig. 4c depicts the utilization of agomelatine of TTS prepared according to Examples 4a, 4b, 4c, 4d and 4e after 24 hours.
Fig. 4d depicts a microscopic picture of the agomelatine-containing layer of a TTS prepared according to Example 4c.
Fig. 5a depicts the agomelatine skin permeation rate of TTS prepared according to Examples 5a, 5b, 5c and 5d for hours 0 to 8.
Fig. 5b depicts the utilization of agomelatine of TTS prepared according to Examples 5a, 5b, 5c and 5d after 8 hours.
Fig. 5c depicts a microscopic picture of the agomelatine-containing layer of a TTS prepared according to Example 5b.
Fig. 5d depicts a microscopic picture of the agomelatine-containing layer of a TTS prepared according to Example 5d.
Fig. 6a depicts the agomelatine skin permeation rate of TTS prepared according to Examples 3c, 6a, 6b, 6c and 6d for hours 0 to 12.
Fig. 6b depicts the utilization of agomelatine of TTS prepared according to Examples 3c, 6a, 6b, 6c and 6d after 8 hours.
Fig. 6c depicts a microscopic picture of the agomelatine-containing layer of a TTS prepared according to Example 6a.
Fig. 6d depicts a microscopic picture of the agomelatine-containing layer of a TTS prepared according to Example 6c.
Fig. 7a depicts the agomelatine skin permeation rate of TTS prepared according to Examples 7a, 7b, 7c, 7d and 3d for hours 0 to 24.
Fig. 7b depicts the utilization of agomelatine of TTS prepared according to Examples 7a, 7b, 7c, 7d and 3d after 8 hours.
Fig. 7c depicts the utilization of agomelatine of TTS prepared according to Examples 7a, 7b, 7c, 7d and 3d after 24 hours.
Fig. 8a depicts the agomelatine skin permeation rate of TTS prepared according to Examples 8a, 8b, 8c and 8d for hours 0 to 24.
Fig. 8b depicts the utilization of agomelatine of TTS prepared according to Examples 8a, 8b, 8c and 8d after 8 hours.
Fig. 8c depicts a microscopic picture of the agomelatine-containing layer of a TTS prepared according to Example 8b.
Fig. 8d depicts a microscopic picture of the agomelatine-containing layer of a TTS prepared according to Example 8d.
Fig. 8e depicts the sum of possible degradation substances and the agomelatine amount detected in a storage stability test at 25 °C and 60 % RH, 30 °C and 75 % RH as well as 40 °C and 75 % RH at different time points for a TTS prepared according to Example 8b.
Fig. 8f depicts the adhesion force and peel force determined in a storage stability test at 25 °C and 60 % RH, 30 °C and 75 % RH as well as 40 °C and 75 % RH at different time points for a TTS prepared according to Example 8b.
Fig. 8g depicts the sum of possible degradation substances and the agomelatine amount detected in a storage stability test at 25 °C and 60 % RH, 30 °C and 75 % RH as well as 40 °C and 75 % RH at different time points for a TTS prepared according to Example 8d.
Fig. 8h depicts the adhesion force and peel force determined in a storage stability test at 25 °C and 60 % RH, 30 °C and 75 % RH as well as 40 °C and 75 % RH at different time points for a TTS prepared according to Example 8d.
Fig. 9a depicts a microscopic picture of the agomelatine-containing layer of a TTS prepared according to Example 9a.
Fig. 9b depicts a microscopic picture of the agomelatine-containing layer of a TTS prepared according to Example 9b.
Fig. 9c depicts a microscopic picture of the agomelatine-containing layer of a TTS prepared according to Example 9c.
Fig. 9d depicts a microscopic picture of the agomelatine-containing layer of a TTS prepared according to Example 9d.
Fig. 9e depicts a microscopic picture of the agomelatine-containing layer of a TTS prepared according to Example 9e.

### DETAILED DESCRIPTION

### TTS STRUCTURE

The present invention is related to a transdermal therapeutic system for the transdermal administration of agomelatine comprising a self-adhesive layer structure containing agomelatine.

The self-adhesive layer structure contains therapeutically effective amounts of agomelatine and comprises A) a backing layer, and B) an agomelatine-containing layer comprising i) agomelatine and ii) a hydrophobic polymer.

Thus, the transdermal therapeutic system according to the claims for the transdermal administration of agomelatine comprises a self-adhesive layer structure containing a therapeutically effective amount of agomelatine, said self-adhesive layer structure comprising:
A) a backing layer;
B) an agomelatine-containing layer comprising:
   i) agomelatine; and
   ii) a hydrophobic polymer.

According to the claims, the agomelatine-containing layer further comprises a crystallization inhibitor, or comprises at least 1 wt-% of a crystallization inhibitor.

Thus, in certain embodiments of the invention, the transdermal therapeutic system for the transdermal administration of agomelatine comprises a self-adhesive layer structure containing a therapeutically effective amount of agomelatine, said self-adhesive layer structure comprising:
A) a backing layer;
B) an agomelatine-containing layer comprising:
   i) agomelatine;
   ii) a hydrophobic polymer; and
   iii) at least 1 wt-% of a crystallization inhibitor.

The backing layer is in particular substantially agomelatine-impermeable.

The TTS according to the present invention may in particular be a matrix-type TTS or a microreservoir-type TTS, and more preferably is a microreservoir-type TTS.

In such a matrix-type or microreservoir-type TTS, a therapeutically effective amount of agomelatine is included in the agomelatine-containing layer. The self-adhesive layer structure in such a matrix-type or microreservoir-type TTS can include one or more further layers such as a skin contact layer. In such a further layer, the active agent may be included or may not be included. As outlined above, a skin contact layer can, even if manufactured as an active agent-free layer, after equilibration, comprise agomelatine and then may also be regarded as an additional agomelatine-containing matrix-type or microreservoir-type layer. The further layer and the agomelatine-containing layer may comprise the same hydrophobic polymer or different polymers. Any of the agomelatine-containing layer and the further layer(s) may be directly contacting each other or separated by a membrane such as a rate controlling membrane. If an agomelatine-containing layer is prepared by laminating two layers which are of substantially the same composition, the resulting double layer is to be regarded as one layer.

In a reservoir-type TTS, the active agent is included in a liquid or semi-liquid reservoir. The self-adhesive layer structure in such a reservoir-type TTS can include one or more further layers such as a skin contact layer. In such a further layer, the active agent may be included or may not be included. As outlined above, a skin contact layer can, even if manufactured as an active agent-free layer, after equilibration, comprise agomelatine and then may also be regarded as an agomelatine-containing layer in the sense of the present invention. A reservoir-type TTS further includes a rate controlling membrane separating the reservoir and skin contact layer.

Thus, in certain embodiments, the self-adhesive layer structure comprises an additional reservoir layer which is located between the backing layer and the agomelatine-containing layer, and a further rate controlling membrane which is located between the additional reservoir layer and the agomelatine-containing layer.

In specific embodiments, the self-adhesive layer structure according to the invention comprises an additional skin contact layer. The additional skin contact layer is self-adhesive and provides for adhesion between the self-adhesive layer structure and the skin of the patient during administration.

In such embodiments, the self-adhesive layer structure may or may not comprise a membrane which is located between the agomelatine-containing layer and the additional skin contact layer, wherein the membrane is preferably a rate controlling membrane.

In another embodiment, the self-adhesive layer structure according to the invention does not comprise an additional skin contact layer. Sufficient adhesion between the self-adhesive layer structure and the skin of the patient during administration is then provided for by other means, e.g. an agomelatine-containing layer and/or an adhesive layer. In particular, the self-adhesive layer structure may consist of the backing layer and the agomelatine-containing layer.

Thus, according to certain embodiments of the invention, the TTS may further comprise an adhesive overlay or does not comprise an adhesive overlay, and preferably does not comprise an adhesive overlay. This adhesive overlay is in particular larger than the agomelatine-containing self-adhesive layer structure and is attached thereto for enhancing the adhesive properties of the overall transdermal therapeutic system. Said adhesive overlay comprises also a backing layer. The area of said adhesive overlay adds to the overall size of the TTS but does not add to the area of release. The adhesive overlay comprises a self-adhesive polymer or a self-adhesive polymer mixture selected from the group of acrylic polymers, polyisobutylenes, styrene-isoprene-styrene copolymers, polysiloxanes, and mixtures thereof, which may be identical to or different from any (e.g. hydrophobic) polymer or polymer mixture included in the active agent-containing self-adhesive layer structure.

The self-adhesive layer structure according to the invention is normally located on a detachable protective layer (release liner) from which it is removed immediately before application to the surface of the patient's skin. Thus, the TTS may further comprise a release liner. A TTS protected this way is usually stored in a seam-sealed pouch. The packaging may be child resistant and/or senior friendly.

### AGOMELATINE-CONTAINING LAYER

As outlined in more detail above, the TTS according to certain embodiments of the present invention comprises a self-adhesive layer structure comprising an agomelatine-containing layer.

In these embodiments, the agomelatine-containing layer according to the claims comprises
i) agomelatine; and
ii) a hydrophobic polymer.

According to the claims, the agomelatine-containing layer comprises a crystallization inhibitor, or at least 1 wt-% of a crystallization inhibitor.

In some specific embodiments, the agomelatine-containing layer is of a microreservoir-type or of a matrix-type, and preferably is of a microreservoir-type. In such embodiments, the agomelatine may be completely dissolved or may be in dispersed form.

As already indicated, the active agent-containing layer in a microreservoir-type TTS is a biphasic layer having an inner active-containing phase in an outer matrix-phase, wherein the inner phase is in form of dispersed deposits within the outer phase.

Where the agomelatine-containing layer comprises a crystallization inhibitor, it is conjectured that the agomelatine is present with the crystallization inhibitor in a homogeneous form in the inner phase, while the hydrophobic polymer is present as a separate phase, forming the outer phase of the biphasic layer.

Thus, in certain embodiments of the invention, the agomelatine-containing layer is a dried biphasic layer having
a) an outer phase having a pressure-sensitive adhesive composition comprising the hydrophobic polymer, and
b) an inner phase having a composition comprising the agomelatine,
wherein the inner phase forms dispersed deposits in the outer phase.

As already outlined, it is assumed that the crystallization inhibitor, as comprised in the agomelatine-containing layer, is present with the agomelatine within the droplets of the inner phase, forming a homogeneous phase, which may be e.g. a viscous liquid or an amorphous phase or a so-called solid solution of agomelatine dissolved in the crystallization inhibitor and optional further excipients such as solubilizers. Thus, in such embodiments, the composition of the inner phase comprises a crystallization inhibitor, and preferably the pressure-sensitive adhesive composition of the outer phase comprises substantially no crystallization inhibitor, specifically, the pressure-sensitive adhesive composition of the outer phase may comprise less than or equal to 5 wt %, preferably less than or equal to 3 wt-%, or more preferably less than or equal to 1 wt-% crystallization inhibitor.

On the other hand, the hydrophobic polymer is supposed to be present in the outer phase. Thus, in these embodiments, the composition of the inner phase comprises substantially no hydrophobic polymer, and specifically, the composition of the inner phase comprises less than or equal to 5 wt %, preferably less than or equal to 3 wt-%, or more preferably less than or equal to 1 wt-% hydrophobic polymer.

As the term "microreservoir" indicates, the dispersed deposits are of micrometer size, i.e., in certain embodiments, the dispersed deposits have an average particle size of 0.1 to 100 µm, or of 0.5 to 50 µm.

A microreservoir system has the advantage that a sufficient amount of active can be dissolved in the inner phase, without having to increase the drug solubility of the actual adhesive layer, i.e. the outer phase, which might result in a poor drug release. Without wishing to be bound by theory, it is assumed that this balance of sufficient amount of active in the inner phase and low drug solubility in the outer phase leads to the good permeation profile of the inventive TTS concerned. Such a system also has the advantage that (re-)crystallization of the active and associated restriction on storage stability can be prevented.

Also in general and independently of whether the agomelatine-containing layer is of microreservoir-type or not, in a specific embodiment of the invention, the agomelatine-containing layer is free of agomelatine crystals.

Further, the area weight of the agomelatine-containing layer is one of the factors decisive for the amount of active. A certain thickness is required in order to obtain a sufficient amount of active, and it is also difficult to coat very thin layers in particular with sufficient accuracy. On the other hand, very thick layers are not only uncomfortable to wear and prone to detachment from the skin, but also difficult to manufacture, and tend to lead to a continuous release of the active of up to 24 hours or more, which in the case of agomelatine is not the desired release profile. On balance, it is preferred that the agomelatine-containing layer has an area weight of at least 25 g/m², more preferably at least 35 g/m², or most preferably at least 40 g/m², or has an area weight of less than or equal to 150 g/m², more preferably less than or equal to 120 g/m², or most preferably less than or equal to 90 g/m², or has an area weight of from 25 to 150 g/m², more preferably of from 35 to 120 g/m², or most preferably of from 40 to 90 g/m².

While the area of release controls the effective dose and thus a certain minimum size in terms of area of release is required, if the area of release is too large, the TTS will be huge in size, uncomfortable to wear and lead to low patient compliance. Considering this, in certain embodiments of the invention, the transdermal therapeutic system has an area of release of at least 1 cm², preferably at least 5 cm², or more preferably at least 10 cm², or has an area of release of less than or equal to 100 cm², preferably less than or equal to 60 cm², or more preferably less than or equal to 50 cm², or has an area of release of from 1 to 100 cm², preferably of from 5 to 60 cm², or more preferably of from 10 to 50 cm².

As outlined also above and without wishing to be bound by theory, it is believed that a sufficient amount of active agent contained in the TTS is necessary to achieve certain advantageous features of the TTS according to the present invention, such as good *in vitro* skin permeation. On the other hand, if the amount of active is too high, this might lead to undesirable storage stability issues such as re-crystallization of the active, but also to potential skin irritation due to the drug concentration being too high. The amount of agomelatine contained in the TTS can be controlled two-way by adjusting concentration and/or the area weight of the agomelatine-containing layer. Details concerning the area weight are outlined above. With respect to the concentration, the agomelatine-containing layer comprises at least 0.5 wt-% agomelatine, preferably at least 1 wt-% agomelatine, and more preferably at least 1.5 wt-% agomelatine, or the agomelatine-containing layer comprises less than or equal to 8 wt-% agomelatine, preferably less than or equal to 6 wt-% agomelatine, and more preferably less than or equal to 5 wt-% agomelatine, or the agomelatine-containing layer comprises from 0.5 to less than or equal to 8 wt-% agomelatine, preferably from 1 to less than or equal to 6 wt-% agomelatine, and more preferably from 1.5 to less than or equal to 5 wt-% agomelatine.

Thus, in certain embodiments of the invention, the agomelatine-containing layer comprises at least 0.04 mg/cm², preferably at least 0.06 mg/cm², more preferably at least 0.08 mg/cm², or most preferably at least 0.1 mg/cm² agomelatine, or wherein the agomelatine-containing layer comprises less than or equal to 0.4 mg/cm², preferably less than or equal to 0.3 mg/cm², more preferably less than or equal to 0.25 mg/cm², or most preferably less than or equal to 0.2 mg/cm² agomelatine per area of release.

In terms of active amount per TTS, the amount of agomelatine contained in the transdermal therapeutic system is at least 0.5 mg, preferably at least 1 mg, or more preferably at least 2 mg, or the amount of agomelatine contained in the transdermal therapeutic system is less than or equal to 15 mg, preferably less than or equal to 10 mg, or more preferably less than or equal to 8 mg, or the amount of agomelatine contained in the transdermal therapeutic system ranges from 0.5 to 15 mg, or preferably from 1 to 10 mg, or more preferably from 2 to 8 mg.

In certain embodiments of the invention, the agomelatine-containing layer is a pressure-sensitive adhesive layer.

As will be appreciated further below in more detail, in certain embodiments, it is preferable that the coating composition for preparing the agomelatine-containing layer makes use of ethanol as solvent, but no water. Thus, the transdermal therapeutic system according to the present invention is preferably obtainable (and/or is obtained) by drying a coated coating composition comprising the agomelatine, the hydrophobic polymer, optionally the crystallization inhibitor, the polyvinylpyrrolidone, and ethanol. It is also preferred that the transdermal therapeutic system according to the present invention is obtainable (and/or is obtained) by drying a coated coating composition comprising substantially no water, e.g. less than 1 wt-%, preferably less than 0.5 wt-% or or more preferably less than 0.1 wt-% water

Considering the stability of the agomelatine-containing layer with respect to its composition, it is preferable that the agomelatine-containing layer does not comprise any volatile constituents, which bear the risk of evaporating and changing the composition upon storage. Thus, in certain embodiments, the agomelatine-containing layer comprises substantially no volatile solvent. A volatile solvent in this sense may be selected from the group consisting of C1 to C3 linear and branched alcohols, ethyl acetate, hexane, n-heptane, and any mixtures thereof. In particular, the agomelatine-containing layer comprises less than or equal to 5 wt-%, preferably less than or equal to 3 wt-%, and more preferably less than or equal to 1 wt-% volatile solvent. In particular, the agomelatine-containing layer may comprise substantially no isopropanol, e.g. comprises less than or equal to 5 wt-%, preferably less than or equal to 3 wt-%, and more preferably less than or equal to 1 wt-% isopropanol.

As the solubility of agomelatine may be too high, and also with regard to the reduced tendency of forming microresevoir-type layers, it is preferred that the agomelatine-containing layer contains only a limited amount of acrylic polymer. Thus, in certain embodiments, the agomelatine-containing layer does not comprise an acrylic polymer in an amount of more than 70 wt-%, preferably more than 50 wt-%, and more preferably more than 30 wt-% of the agomelatine-containing layer.

### AGOMELATINE

In accordance with the invention, the self-adhesive layer structure contains agomelatine in a therapeutically effective amount, and the self-adhesive layer structure comprises an agomelatine-containing layer.

While in accordance with the present invention, the active agent agomelatine may be present in the TTS, and in particular in the agomelatine-containing layer in any form, i.e. in its free, dissociated or any associated form such as hydrates, solvates and so on, as well as in the form of particles which may be in micronized form, crystalline form, and in particular in one of its polymorph forms, and/or in amorphous form, and in any hybrid type form of any of the aforementioned forms or a mixture thereof, it is preferred that the agomelatine is present in the free, dissociated form.

Further, in certain embodiments, the agomelatine is included in the agomelatine-containing layer in dissolved form, in dispersed form, in crystalline form, in particular in one of its polymorph forms, in an amorphous form, as a hydrate, a solvate, a hybrid type form of any of the foregoing forms or a mixture thereof.

In certain embodiments, the m agomelatine-containing layer composition is obtainable (and/or is obtained) by incorporating the agomelatine dissolved form, in dispersed form, in crystalline form, in particular in one of its polymorph forms, in an amorphous form, as a hydrate, a solvate, a hybrid type form of any of the foregoing forms or a mixture thereof.

The agomelatine in the agomelatine-containing layer may be (completely) dissolved, or the agomelatine-containing layer may comprise agomelatine particles, preferably constituted of agomelatine in its free, dissociated form, so that the agomelatine is present in dispersed form. Needless to say, if the agomelatine is present in dispersed form, the agomelatine-containing layer nonetheless may comprise agomelatine also in dissolved form, depending on the solubility of the active in the agomelatine-containing layer (which is e.g. saturated or super-saturated).

In a preferred embodiment, the agomelatine is completely dissolved, e.g. at least 90 mol%, preferably at least 95 mol%, more preferably at least 98 mol% or most preferably at least 99 mol% of the agomelatine in the agomelatine-containing layer is present in dissolved form.

As outlined above, the amount of agomelatine in the TTS is believed to be important for a good release of the active, and can be e.g. adjusted by the agomelatine concentration. Thus, in certain embodiments, the concentration of agomelatine in the agomelatine-containing layer ranges from 0.5 to 8 wt-%, preferably from 1 to 6 wt-% and more preferably from 1.5 to 5 wt-% of the agomelatine-containing layer.

In certain embodiments, the agomelatine has a purity of at least 95 %, preferably of at least 98 % and more preferably of at least 99 % as determined by quantitative HPLC. Quantitative HPLC may be performed with Reversed-Phase-HPLC with UV detection. In particular, the following conditions can be used if HPLC is performed isocratically:

| | |
|---|---|
| Column: | RP Octadecyl phase |
| | XTerra RP18 100 mm × 3.9 mm; 3.5 µm or equivalent |
| Mobile phase: | 0.06 molar KH₂PO₄ Buffer/acetonitril (60:40; v:v); pH 2.5 |
| Gradient: | isocratic |
| Flux: | 1.0 ml |
| Injection volume: | 20 µl |
| Column temperature: | 23 °C |
| Wavelength: | 229 nm and 275 nm |
| Run time: | 5 min |

The TTS according to the present invention advantageously show an improved stability in terms of the agomelatine content as well as agomelatine degradation.

Thus, in certain embodiments, the agomelatine-containing layer contains initially (i.e. shortly after manufacture e.g. within one week) an amount of agomelatine of at least 95 %, preferably of at least 97 %, more preferably of at least 98 % and even more preferably of at least 99 % of the theoretical amount of agomelatine included in the agomelatine-containing layer. The theoretical amount of agomelatine is calculated from the agomelatine amount used for the coating composition and the (actual) area weight of the coated and dried agomelatine-containing layer of the tested TTS.

The agomelatine-containing layer may also contain initially a total amount of agomelatine-related degradation substances of less than 0.5 %, preferably of less than 0.3 %, more preferably of less than 0.2 % and even more preferably of less than 0.1 %.

In certain other embodiments, the TTS according to the present invention are stable upon storage, i.e. they may maintain the initial agomelatine content values or present low amounts of degradation products, as follows:

In one of such embodiments, the agomelatine-containing layer contains, after having been stored at 25 °C and 60 % relative humidity for at least 3 months, preferably at least 6 months, more preferably at least 9 months and most preferably at least 12 months, an amount of agomelatine of at least 95 %, preferably of at least 97 %, more preferably of at least 98 % and even more preferably of at least 99 % of the theoretical amount of agomelatine included in the agomelatine-containing layer.

The agomelatine-containing layer may also contain, after having been stored at 25 °C and 60 % relative humidity for at least 3 months, preferably at least 6 months, more preferably at least 9 months and most preferably at least 12 months, a total amount of agomelatine-related degradation substances of less than 1.0 %, preferably of less than 0.5 %, more preferably of less than 0.2 % and even more preferably of less than 0.1 %.

In one of such embodiments, the agomelatine-containing layer contains, after having been stored at 30 °C / 75 % RH for at least 3 months, preferably at least 6 months, more preferably at least 9 months and most preferably at least 12 months, an amount of agomelatine of at least 95 %, preferably of at least 97 %, more preferably of at least 98 % and even more preferably of at least 99 % of the theoretical amount of agomelatine included in the agomelatine-containing layer.

The agomelatine-containing layer may also contain, after having been stored at 30 °C / 75 % RH for at least 3 months, preferably at least 6 months, more preferably at least 9 months and most preferably at least 12 months, a total amount of agomelatine -related degradation substances of less than 1.0 %, preferably of less than 0.5 %, more preferably of less than 0.2 % and even more preferably of less than 0.1 %.

In one of such embodiments, the agomelatine-containing layer contains, after having been stored at 40 °C / 75 % RH for at least 3 months and preferably at least 6 months, an amount of agomelatine of at least 95 %, preferably of at least 96 %, more preferably of at least 97 % and even more preferably of at least 98 % of the theoretical amount of agomelatine included in the agomelatine-containing layer.

The agomelatine-containing layer may also contain, after having been stored at 40 °C / 75 % RH for at least 3 months and preferably at least 6 months, a total amount of agomelatine - related degradation substances of less than 1.0 %, preferably of less than 0.7 %, more preferably of less than 0.5 % and even more preferably of less than 0.4 %.

The TTS according to the present invention are also advantageously stable upon storage in terms of adhesion and removability of the agomelatine-containing layer from the release liner, i.e. they may maintain the adhesion force and peel force over time.

Thus, in certain embodiments, the adhesion force of the agomelatine-containing layer decreases by less than 25 %, preferably less than 10 % and more preferably by less than 5 % after having been stored at 25 °C and 60 % relative humidity for at least 3 months, preferably at least 6 months, more preferably at least 9 months and most preferably at least 12 months.

In certain embodiments, the adhesion force of the agomelatine-containing layer decreases by less than 25 %, preferably less than 10 % and more preferably by less than 5 % after having been stored at 30 °C and 75 % relative humidity for at least 3 months, preferably at least 6 months, more preferably at least 9 months and most preferably at least 12 months.

The adhesion force of the agomelatine-containing layer may also decrease by less than 20 %, preferably less than 10 % and more preferably by less than 3 % after having been stored at 40 °C / 75 % RH for at least 3 months and preferably at least 6 months.

In certain embodiments, the peel force of the agomelatine-containing layer increases by less than 150 %, preferably less than 100 % and more preferably by less than 30 % after having been stored at 25 °C and 60 % relative humidity for at least 3 months, preferably at least 6 months, more preferably at least 9 months and most preferably at least 12 months.

In certain embodiments, the peel force of the agomelatine-containing layer increases by less than 250 %, preferably less than 150 % and more preferably by less than 100 % after having been stored at 30 °C and 75 % relative humidity for at least 3 months, preferably at least 6 months, more preferably at least 9 months and most preferably at least 12 months.

The peel force of the agomelatine-containing layer may also increase by less than 250 %, preferably less than 100 % and more preferably by less than 60 % after having been stored at 40 °C / 75 % RH for at least 3 months and preferably at least 6 months.

The method for determining the agomelatine content and the total amount of agomelatine-related degradation substances, as well as the adhesion force and the peel force is preferably conducted as described for Examples 8b and 8d.

### HYDROPHOBIC POLYMER

As outlined above, the TTS according to the present invention comprises a self-adhesive layer structure comprising an agomelatine-containing layer comprising a hydrophobic polymer.

This hydrophobic polymer provides for sufficient cohesion of the agomelatine-containing layer. According to certain embodiments, the hydrophobic polymer may also provide for sufficient adhesion. In such embodiments, but also in general, the hydrophobic polymer may be selected from pressure sensitive adhesive polymers.

In a preferred embodiment, the amount of the hydrophobic polymer is at least 75 wt-%, preferably at least 80 wt-% and more preferably at least 75 wt-%, and/or the amount of the hydrophobic polymer is less than or equal to 98 wt-%, preferably less than or equal to 94 wt-% and more preferably less than or equal to 90 wt-%, and in particular, the amount of the hydrophobic polymer ranges from 75 to 98 wt-%, preferably from 80 to 94 wt-%, and more preferably from 85 to 90 wt-% of the agomelatine-containing layer.

Polymers which are suitable as the hydrophobic polymer are selected from the group consisting of polyisobutylenes, styrene-isoprene-styrene block copolymers, silicone acrylic hybrid polymers, pressure-sensitive adhesives based on polysiloxanes, and any mixtures thereof. The claims disclose pressure-sensitive adhesives based on polysiloxanes as the hydrophobic polymer.

The hydrophobic polymer is present in the agomelatine-containing layer, but may also be contained in an optional adhesive overlay.

The hydrophobic polymer is usually supplied and used in solvents like n-heptane and ethyl acetate. The solids content of the pressure-sensitive adhesives is usually between 30 % and 80 %.

Suitable hydrophobic polymers are commercially available e.g. under the brand names Oppanol^{™} (polyisobutylenes), JSR-SIS (a styrene-isoprene-styrene copolymer), SilAc Hybrid PSAs (silicone acrylic hybrid polymers) or BIO-PSAs (pressure sensitive adhesives based on polysiloxanes). The claims disclose pressure-sensitive adhesives based on polysiloxanes as the hydrophobic polymer.

According to the claims, the hydrophobic polymer is a pressure-sensitive adhesive based on polysiloxanes.

Pressure-sensitive adhesives based on polysiloxanes may also be referred to as silicone-based pressure-sensitive adhesives, or silicone pressure sensitive adhesives. They are advantageous in terms of the utilization of the active as well as the overall release profile.

These pressure-sensitive adhesives based on polysiloxanes provide for suitable tack and for quick bonding to various skin types, including wet skin, suitable adhesive and cohesive qualities, long lasting adhesion to the skin, a high degree of flexibility, a permeability to moisture, and compatibility to many actives and film-substrates. It is possible to provide them with sufficient amine resistance and therefore enhanced stability in the presence of amines. Such pressure-sensitive adhesives are based on a resin-in-polymer concept wherein, by condensation reaction of silanol end blocked polydimethylsiloxane with a silica resin (also referred to as silicate resin), a pressure-sensitive adhesive based on polysiloxane is prepared wherein for amine stability the residual silanol functionality is additionally capped with trimethylsiloxy groups. The silanol end blocked polydimethylsiloxane content contributes to the viscous component of the visco-elastic behavior, and impacts the wetting and the spreadability properties of the adhesive. The resin acts as a tackifying and reinforcing agent, and participates in the elastic component. The correct balance between silanol end blocked polydimethylsiloxane and resin provides for the correct adhesive properties.

In view of the above, silicone-based pressure sensitive adhesives are generally obtainable by polycondensation of silanol endblocked polydimethylsiloxane with a silicate resin. In other words, in a preferred embodiment, the pressure-sensitive adhesive based on polysiloxanes is a soluble silicate resin polycondensed with silanol-terminated polydimethylsiloxanes. Amine-compatible silicone-based polymers, and in particular amine-compatible silicone-based pressure sensitive adhesives, can be obtained by reacting the silicone-based polymer, in particular the silicone-based pressure sensitive adhesive, with trimethylsilyl (e.g. hexamethyldisilazane) in order to reduce the silanol content of the polymer. As a result, the residual silanol functionality is at least partly, preferably mostly or fully capped with trimethylsiloxy groups. Thus, in certain embodiments, the pressure-sensitive adhesive based on polysiloxanes is a soluble silicate resin polycondensed with silanol-terminated polydimethylsiloxanes, wherein those silanol groups of the polydimethylsiloxane, which are not linked to the soluble silicate resin, are either free silanol groups or are trimethylsilylated.

As indicated above, the tackiness of the silicone-based polymer may be modified by the resin-to-polymer ratio, i.e. the ratio of the silanol endblocked polydimethylsiloxane to the silicate resin, which is preferably in the range of from 70:30 to 50:50, preferably from 65:35 to 55:45. The tackiness will be increased with increasing amounts of the polydimethylsiloxane relative to the resin. High tack silicone-based polymers preferably have a resin-to-polymer ratio of 55:45, medium tack silicone-based polymers preferably have a resin-to-polymer ratio of 60:40, and low tack silicone-based polymers preferably have a resin-to-polymer ratio of 65:35. In a preferred embodiment, the pressure-sensitive adhesive based on polysiloxanes is a soluble silicate resin polycondensed with silanol-terminated polydimethylsiloxanes having a resin-to-polymer ratio of 65:35, of 60:40 or of 55:45. High tack silicone-based polymers preferably have a complex viscosity at 0.01 rad/s and 30 °C of about 5 × 10⁶ Poise, medium tack silicone-based polymers preferably have a complex viscosity at 0.01 rad/s and 30 °C of about 5 × 10⁷ Poise, and low tack silicone-based polymers preferably have a complex viscosity at 0.01 rad/s and 30 °C of about 5 × 10⁸ Poise. High tack amine-compatible silicone-based polymers preferably have a complex viscosity at 0.01 rad/s and 30 °C of about 5 × 10⁶ Poise, medium tack amine-compatible silicone-based polymers preferably have a complex viscosity at 0.01 rad/s and 30 °C of about 5 × 10⁸ Poise, and low tack amine-compatible silicone-based polymers preferably have a complex viscosity at 0.01 rad/s and 30 °C of about 5 × 10⁹ Poise.

Examples of silicone-based PSA compositions which are commercially available include the standard BIO-PSA series (7-4400,7-4500 and 7-4600 series), the amine compatible (endcapped) BIO-PSA series (7-4100, 7-4200 and 7-4300 series) and the Soft Skin Adhesives series (7-9800) manufactured and typically supplied in n-heptane or ethyl acetate by Dow Corning. For example, BIO-PSA 7-4201 is characterized by a solution viscosity at 25 °C and about 60 % solids content in heptane of 450 mPa s and a complex viscosity at 0.01 rad/s at 30 °C of 1×10⁸ Poise. BIO-PSA 7-4301 has a solution viscosity at 25 °C and about 60 % solids content in heptane of 500 mPa s and a complex viscosity at 0.01 rad/s at 30 °C of 5×10⁶ Poise.

The pressure-sensitive adhesives based on polysiloxanes are supplied and used in solvents like n-heptane, ethyl acetate or other volatile silicone fluids. The solids content of pressure-sensitive adhesives based on polysiloxanes in solvents is usually between 60 and 85 %, preferably between 70 and 80 % or between 60 and 75 %. The skilled person is aware that the solids content may be modified by adding a suitable amount of solvent.

Pressure-sensitive adhesives based on polysiloxanes, which are, e.g., available from Dow Corning, may be obtained according to the following scheme: 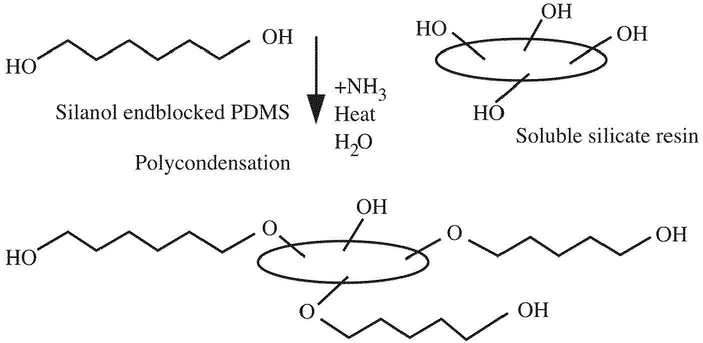 Such pressure-sensitive adhesives based on polysiloxanes are available from Dow Corning, e.g., under the tradenames BIO-PSA 7-4401, BIO-PSA-7-4501, or BIO-PSA 7-4601, which are provided in the solvent n-heptane (indicated by the code "01"), or under the tradenames BIO-PSA 7-4402, BIO-PSA 7-4502, and BIO 7-4602, which are provided in the solvent ethyl acetate (indicated by the code "02"). Typical solids contents in the solvent are in the range of from 60 to 75 %. The code "44" indicates a resin-to-polymer ratio of 65:35 resulting in a low tackiness, the code "45" indicates a resin-to-polymer ratio of 60:40 resulting in medium tackiness, the code "46" indicates a resin-to-polymer ratio of 55:45 resulting in high tackiness.

Amine-compatible pressure-sensitive adhesives based on polysiloxanes, which are, e.g., available from Dow Corning may be obtained according to the following scheme: 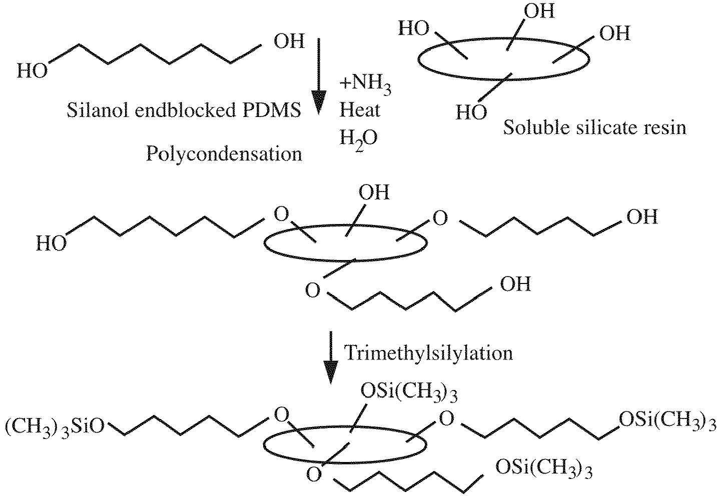 Such amine-compatible pressure-sensitive adhesives based on polysiloxanes are available from Dow Corning, e.g., under the tradenames BIO-PSA 7-4101, BIO-PSA-7-4201, or BIO-PSA 7-4301, which are provided in the solvent n-heptane (indicated by the code "01"), or under the tradenames BIO-PSA 7-4102, BIO-PSA 7-4202, and BIO 7-4302, which are provided in the solvent ethyl acetate (indicated by the code "02"). Typical solids contents in the solvent are in the range of from 60 to 75 %. The code "41" indicates a resin-to-polymer ratio of 65:35 resulting in a low tackiness, the code "42" indicates a resin-to-polymer ratio of 60:40 resulting in medium tackiness, the code "43" indicates a resin-to-polymer ratio of 55:45 resulting in high tackiness.

In a certain embodiment, pressure-sensitive adhesives based on polysiloxanes in accordance with the invention are characterized by a solution viscosity at 25 °C and 60 % solids content in n-heptane of more than about 150 mPa s, or from about 200 mPa s to about 700 mPa s, preferably as measured using a Brookfield RVT viscometer equipped with a spindle number 5 at 50 rpm. These may also be characterized by a complex viscosity at 0.01 rad/s at 30 °C of less than about 1 × 10⁹ Poise or from about 1 × 10⁵ to about 9 × 10⁸ Poise. Also in certain embodiments, pressure-sensitive adhesives based on polysiloxanes are characterized by a solution viscosity at 25 °C and 60 % solids content in ethyl acetate of more than about 350 mPa s, or from about 400 mPa s to about 1500 mPa s, preferably as measured using a Brookfield RVT viscometer equipped with a spindle number 5 at 50 rpm, or are characterized by a complex viscosity at 0.01 rad/s at 30 °C of from about 1 × 10⁵ to about 1 × 10⁷ Poise or about 5 × 10⁶ Poise.

Disclosed, but not claimed, is that the hydrophobic polymer is a polyisobutylene.

Suitable polyisobutylenes according to the invention are available under the tradename Oppanol^{®}. Combinations of high-molecular weight polyisobutylenes (B100/B80) and low-molecular weight polyisobutylenes (B10, B11, B12, B13) may be used. Suitable ratios of low-molecular weight polyisobutylene to high-molecular weight polyisobutylene are in the range of from 100:1 to 1:100, in particular from 95:5 to 40:60, preferably from 90:10 to 80:20 or from 60:40 to 20:80, and preferably from 50:50 to 30:70. A preferred example for a polyisobutylene combination is B10/B100 in a ratio of 85/15, or in a ratio of 40/60. Oppanol^{®} B100 has a viscosity average molecular weight Mᵥ of 1,110,000, and a weight average molecular weight M_{w} of 1,550,000, and an average molecular weight distribution M_{w}/Mₙ of 2.9. Oppanol^{®} B10 has a viscosity average molecular weight Mᵥ of 40,000, and a weight average molecular weight M_{w} of 53,000, and an average molecular weight distribution M_{w}/Mₙ of 3.2. In certain embodiments, polybutene may be added to the polyisobutylenes. The solids content of polyisobutylenes in solvents is usually between 30 and 50 %, preferably between 35 and 40 %. The skilled person is aware that the solids content may be modified by adding a suitable amount of solvent.

Disclosed, but not claimed, is that the hydrophobic polymer is a styrene-isoprene-styrene block copolymer.

The hydrophobic polymer may also be a silicone acrylic hybrid polymer, which is not claimed.

Silicone acrylic hybrid pressure-sensitive adhesives are usually supplied and used in solvents like n-heptane and ethyl acetate. The solids content of the pressure-sensitive adhesives is usually between 30 % and 80 %. The skilled person is aware that the solids content may be modified by adding a suitable amount of solvent.

Preferably, the weight ratio of silicone to acrylate in the silicone acrylic hybrid pressure-sensitive adhesive is from 5:95 to 95:5, or from 20:80 to 80:20, more preferably from 40:60 to 60:40, and most preferably the ratio of silicone to acrylate is about 50:50.

Suitable silicone acrylic hybrid pressure-sensitive adhesives which are commercially available include the PSA series 7-6100 and 7-6300 manufactured and supplied in n-heptane or ethyl acetate by Dow Corning (7-610X and 7-630X; X=1 n-heptane-based / X=2 ethyl acetate-based). For example, the 7-6102 silicone acrylic hybrid PSA having a silicone/acrylate ratio of 50/50 is characterized by a solution viscosity at 25 °C and about 50 % solids content in ethyl acetate of 2,500 cP and a complex viscosity at 0.1 rad/s at 30 °C of 1.0e7 Poise. The 7-6302 silicone acrylic hybrid PSA having a silicone/acrylate ratio of 50/50 has a solution viscosity at 25 °C and about 50 % solids content in ethyl acetate of 1,500 cP and a complex viscosity at 0.1 rad/s at 30 °C of 4.0e6 Poise.

Depending on the solvent in which the silicone acrylic hybrid pressure-sensitive adhesive is supplied, the arrangement of the silicone phase and the acrylic phase providing a silicone or acrylic continuous external phase and a corresponding discontinuous internal phase is different. If the silicone acrylic hybrid pressure-sensitive adhesive is provided in n-heptane, the composition contains a continuous, silicone external phase and a discontinuous, acrylic internal phase. If the silicone acrylic hybrid pressure-sensitive adhesive is provided in ethyl acetate, the composition contains a continuous, acrylic external phase and a discontinuous, silicone internal phase. After evaporating the solvent in which the silicone acrylic hybrid pressure-sensitive adhesive is provided, the phase arrangement of the resulting pressure-sensitive adhesive film or layer corresponds to the phase arrangement of the solvent-containing adhesive coating composition. For example, in the absence of any substance that may induce an inversion of the phase arrangement in a silicone acrylic hybrid pressure sensitive adhesive composition, a pressure-sensitive adhesive layer prepared from a silicone acrylic hybrid pressure-sensitive adhesive in n-heptane provides a continuous, silicone external phase and a discontinuous, acrylic internal phase, a pressure-sensitive adhesive layer prepared from a silicone acrylic hybrid pressure-sensitive adhesive in ethyl acetate provides a continuous, acrylic external phase and a discontinuous, silicone internal phase. The phase arrangement of the compositions can, for example, be determined in peel force tests with pressure-sensitive adhesive films or layers prepared from the silicone acrylic hybrid PSA compositions which are attached to a siliconized release liner. The pressure-sensitive adhesive film contains a continuous, silicone external phase if the siliconized release liner cannot or can only hardly be removed from the pressure-sensitive adhesive film (laminated to a backing film) due to the blocking of the two silicone surfaces. Blocking results from the adherence of two silicone layers which comprise a similar surface energy. The silicone adhesive shows a good spreading on the siliconized liner and therefore can create a good adhesion to the liner. If the siliconized release liner can easily be removed the pressure-sensitive adhesive film contains a continuous, acrylic external phase. The acrylic adhesive has no good spreading due to the different surface energies and thus has a low or almost no adhesion to the siliconized liner.

Disclosed, but not claimed, is that the silicone acrylic hybrid polymer is a silicone acrylic hybrid pressure-sensitive adhesive obtainable from a silicon-containing pressure-sensitive adhesive composition comprising acrylate or methacrylate functionality. It is to be understood that the silicon-containing pressure-sensitive adhesive composition comprising acrylate or methacrylate functionality can include only acrylate functionality, only methacrylate functionality, or both acrylate functionality and methacrylate functionality.

Disclosed, but not claimed, is that the silicone acrylic hybrid pressure-sensitive adhesive comprises the reaction product of (a) a silicon-containing pressure-sensitive adhesive composition comprising acrylate or methacrylate functionality, (b) an ethylenically unsaturated monomer, and (c) an initiator. That is, the silicone acrylic hybrid pressure-sensitive adhesive is the product of the chemical reaction between these reactants ((a), (b), and (c)). In particular, the silicone acrylic hybrid pressure-sensitive adhesive includes the reaction product of (a) a silicon-containing pressure-sensitive adhesive composition comprising acrylate or methacrylate functionality, (b) a (meth)acrylate monomer, and (c) an initiator (i.e., in the presence of the initiator). That is, the silicone acrylic hybrid pressure-sensitive adhesive includes the product of the chemical reaction between these reactants ((a), (b), and (c)).

The reaction product of (a) a silicon-containing pressure-sensitive adhesive composition comprising acrylate or methacrylate functionality, (b) an ethylenically unsaturated monomer, and (c) an initiator may contain a continuous, silicone external phase and a discontinuous, acrylic internal phase or the reaction product of (a), (b), and (c) may contain a continuous, acrylic external phase and a discontinuous, silicone internal phase.

Disclosed, but not claimed, is that the silicone acrylic hybrid polymer comprises a reaction product of a silicone polymer, a silicone resin and an acrylic polymer, wherein the acrylic polymer is covalently self-crosslinked and covalently bound to the silicone polymer and/or the silicone resin.

Disclosed, but not claimed, is that the silicone acrylic hybrid polymer comprises a reaction product of a silicone polymer, a silicone resin and an acrylic polymer, wherein the silicone resin contains triorganosiloxy units R₃SiO_{1/2} where R is an organic group, and tetrafunctional siloxy units SiO_{4/2} in a mole ratio of from 0.1 to 0.9 R₃SiO_{1/2} units for each SiO_{4/2}.

The acrylic polymer may comprise at least an alkoxysilyl functional monomer, polysiloxane-containing monomer, halosilyl functional monomer or alkoxy halosilyl functional monomer. Preferably, the acrylic polymer is prepared from alkoxysilyl functional monomers selected from the group consisting of trialkoxylsilyl (meth)acrylates, dialkoxyalkylsilyl (meth)acrylates, and mixtures thereof, or comprises end-capped alkoxysilyl functional groups. The alkoxysilyl functional groups may preferably be selected from the group consisting of trimethoxylsilyl groups, dimethoxymethylsilyl groups, triethoxylsilyl, diethoxymethylsilyl groups and mixtures thereof.

The acrylic polymer may also be prepared from a mixture comprising polysiloxane-containing monomers, preferably from a mixture comprising polydimethylsiloxane mono (meth)acrylate.

Disclosed, but not claimed, is that the silicone acrylic hybrid polymer may be prepared by a) reacting silicone polymer with silicone resin to form a resultant product, b) reacting the resultant product of a) with an acrylic polymer containing reactive functionality, wherein the components are reacted in an organic solvent.

Disclosed, but not claimed, is that the silicone acrylic hybrid polymer may be prepared by a) reacting a silicone resin with an acrylic polymer containing reactive functionality to form a resultant product, b) reacting the resultant product of a) with silicone polymer, wherein the components are reacted in an organic solvent.

Disclosed, but not claimed, is that the silicone acrylic hybrid polymer may be prepared by a) reacting a silicone polymer with an acrylic polymer containing reactive functionality to form a resultant product, b) reacting the resultant product of a) with silicone resin, wherein the components are reacted in an organic solvent.

Further suitable acrylic polymers, silicone resins, and silicone polymers that can be used for chemically reacting together a silicone polymer, a silicone resin and an acrylic polymer to provide a silicone acrylic hybrid polymer in accordance with the previous paragraphs are detailed in WO 2010/124187.

### CRYSTALLIZATION INHIBITORS AND SOLUBILIZERS

As outlined above, according to the present invention, the agomelatine-containing layer comprises a crystallization inhibitor.

Within the meaning of this invention, a "crystallization inhibitor" is any substance which is able to prevent re-crystallization of the active agent agomelatine in the TTS and in particular in the agomelatine-containing layer of the present invention. The skilled person is aware of suitable crystallization inhibitors.

For the above-mentioned effect of re-crystallization prevention, but also for an advantageous effect on the permeation profile (see also below), a certain amount of the crystallization inhibitor is required. On the other hand, if incorporated in high amounts, the permeation rate may be adversely affected due to too high drug solubility. Thus, the agomelatine-containing layer preferably comprises at least 1 wt-% of a crystallization inhibitor. In preferred embodiments, the agomelatine-containing layer comprises at least 1.5 wt-%, preferably at least 2.5 wt-%, more preferably at least 4 wt-%, and most preferably at least 5 wt-% of such a crystallization inhibitor, and on the other hand comprises equal to or less than 30 wt-%, preferably equal to or less than 25 wt-%, more preferably equal to or less than 20 wt-% and most preferably equal to or less than 15 wt-% of a crystallization inhibitor, e.g. the agomelatine-containing layer may comprise from 1.5 to 30 wt-%, preferably from 2.5 to 25 wt-%, more preferably from 4 to 20 wt-%, and most preferably from 5 to 15 wt-% of a crystallization inhibitor

Of particular interest as crystallization inhibitors are polymers with an enhanced ability to absorb water, as higher water and/or moisture absorption assists in maintaining / improving the adhesive properties of the agomelatine-containing layer, and since such substances are believed to contribute to the good skin permeation behavior of microreservoir system. That is, without wishing to be bound to any theory, it is believed that in a microreservoir system, the crystallization inhibitor which is present in the inner phase with the active agent, has a higher affinity to water than to the active agent so that cutaneous water taken up during application of the TTS to the skin of a patient displaces dissolved active agent. The displaced molecules of active agent are subject to a high driving force for the diffusion out of the TTS to the skin and into the skin

Thus, in certain embodiments, the agomelatine-containing layer comprises a crystallization inhibitor selected from polymers, which provide for an improved water and/or moisture absorption of the agomelatine-containing layer. Such polymers are well known in the art. Of those, particularly suitable and claimed are polyvinylpyrrolidone and polyvinylpyrrolidone-polyvinylacetate copolymer. Polyvinylpyrrolidone-polyvinylacetate copolymers are commercially available, e.g. under the brand name Kollidon^{®} VA64 supplied by BASF

According to the claims, the crystallization inhibitor is selected from the group consisting of polyvinylpyrrolidone and polyvinylpyrrolidone-polyvinylacetate copolymer. Preferably, the crystallization inhibitor is polyvinylpyrrolidone, and in particular the crystallization inhibitor is selected from soluble polyvinylpyrrolidones.

The term "soluble polyvinylpyrrolidone" refers to polyvinylpyrrolidone, also known as povidone, which is soluble with more than 10 % in at least ethanol, preferably also in water, diethylene glycol, methanol, n-propanol, 2-propanol, n-butanol, chloroform, methylene chloride, 2-pyrrolidone, macrogol 400, 1,2 propylene glycol, 1,4 butanediol, glycerol, triethanolamine, propionic acid and acetic acid. Examples of polyvinylpyrrolidones which are commercially available include Kollidon^{®} 12 PF, Kollidon^{®} 17 PF, Kollidon^{®} 25, Kollidon^{®} 30 and Kollidon^{®} 90 F supplied by BASF, or povidone K90F. The different grades of Kollidon^{®} are defined in terms of the K-Value reflecting the average molecular weight of the polyvinylpyrrolidone grades. Kollidon^{®} 12 PF is characterized by a K-Value range of 10.2 to 13.8, corresponding to a nominal K-Value of 12. Kollidon^{®} 17 PF is characterized by a K-Value range of 15.3 to 18.4, corresponding to a nominal K-Value of 17. Kollidon^{®} 25 is characterized by a K-Value range of 22.5 to 27.0, corresponding to a nominal K-Value of 25, Kollidon^{®} 30 is characterized by a K-Value range of 27.0 to 32.4, corresponding to a nominal K-Value of 30. Kollidon^{®} 90 F is characterized by a K-Value range of 81.0 to 97.2, corresponding to a nominal K-Value of 90. Preferred Kollidon^{®} grades are Kollidon^{®} 12 PF, Kollidon^{®} 30 and Kollidon^{®} 90 F. For all grades and types of polyvinylpyrrolidone, it is preferred that the amount of peroxides is within certain limits, in particular, the peroxide amount is equal to or less than 500 ppm, more preferably equal to or less than 150 ppm, and most preferably equal to or less than 100 ppm.

Within the meaning of this invention, the term "K-Value" refers to a value calculated from the relative viscosity of polyvinylpyrrolidone in water according to the European Pharmacopoeia (Ph.Eur.) and USP monographs for "Povidone".

Thus, in certain embodiments, the crystallization inhibitor selected from polyvinylpyrrolidones having a K-Value within a range selected from the group of ranges consisting of
9 to 15, and preferably 10.2 to 13.8,
15 to 20, and preferably 15.3 to 18.4,
20 to 27, and preferably 22.5 to 27.0,
27 to 35, and preferably 27.0 to 32.4, and
75 to 110, and preferably 81.0 to 97.2,
or any mixtures thereof, and more preferably is a polyvinylpyrrolidone having a K-Value within a range of 27.0 to 32.4 or of 81.0 to 97.2, and any mixtures thereof, and most preferably is a polyvinylpyrrolidone having a K-Value within range of 81.0 to 97.2.

In certain embodiments of the present invention, the agomelatine-containing layer comprises a solubilizer.

Within the meaning of the present invention, the term "solubilizer" refers to any substance that is able to increase the solubility of agomelatine in the agomelatine-containing layer substantially, e.g. by at least 1 percentage point (with respect to the amount of agomelatine in wt-% in the agomelatine-containing layer) per 10 wt-%, preferably per 5 wt-%, more preferably per 1 wt-% and most preferably per 0.5 wt-% solubilizer added to the agomelatine-containing layer.

In certain preferred embodiments, the solubilizer is selected from the group consisting of dipropylene glycol, lauryl lactate, mixtures of propylene glycol monoesters and diesters of fatty acids, which are commercially available e.g. under the brand name Capryol, which is a propylene glycol monocaprylate (type II), a mixture of propylene glycol monoesters and diesters of fatty acids with a ratio of > 90 % monoesters and < 10 % diesters, wherein the fatty acids mainly consist of caprylic acid (commercially available as Capryol^{™} 90 supplied by Gattefossé), levulinic acid, polyethylene glycol ethers, in particular polyethylene glycol fatty alcohol ethers (such as those commercially available as Brij^{®}), e.g. polyethylene glycol dodecyl ether with an average molecular weight Mn of about 362 commercially available as Brij^{®} L4, and diethylene glycol monoethyl ether (commercially available as transcutol^{®}).

A certain minimum amount of solubilizer is advantageous in that it will help preventing recrystallization of the active agent in the agomelatine-containing layer. On the other hand, if the amount of solubilizer is too high, cohesion of the agomelatine-containing layer may be impaired, thus, a balance has to be found. In view of these factors, in a certain preferred embodiment, the agomelatine-containing layer comprises at least 1.5 wt-%, preferably at least 2.5 wt-%, more preferably at least 4 wt-% and most preferably at least 5 wt-% of the solubilizer, and on the other hand comprises equal to or less than 30 wt-%, preferably equal to or less than 25 wt-%, more preferably equal to or less than 20 wt-% and most preferably equal to or less than 15 wt-% of a solubilizer, e.g. the agomelatine-containing layer may comprise from 1.5 to 30 wt-%, preferably from 2.5 to 25 wt-%, more preferably from 4 to 20 wt-%, and most preferably from 5 to 15 wt-% of a solubilizer. In another embodiment, the agomelatine-containing layer does not comprise a solubilizer selected from the group consisting of dipropylene glycol, lauryl lactate, mixtures of propylene glycol monoesters and diesters of fatty acids, levulinic acid, polyethylene glycol ethers and diethylene glycol monoethyl ether.

In addition, with respect to the advantageous effect of preventing agomelatine recrystallization, a crystallization inhibitor and a solubilizer may complement each other. Thus, in certain embodiments, and in particular where a crystallization inhibitor is present in sufficient amounts, the agomelatine-containing layer does not comprise a solubilizer selected from the group consisting of dipropylene glycol, lauryl lactate, mixtures of propylene glycol monoesters and diesters of fatty acids, levulinic acid and polyethylene glycol ethers. Also, preferably, the total amount of crystallization inhibitor and solubilizer present in the agomelatine-containing layer is at least 2.5 wt-%, preferably at least 3.5 wt-%, more preferably at least 4 wt-% and most preferably at least 5 wt-%, and/or is equal to or less than 30 wt-%, preferably equal to or less than 25 wt-%, more preferably equal to or less than 20 wt-% and most preferably equal to or less than 15 wt-%, e.g. is from 1.5 to 30 wt-%, preferably from 2.5 to 25 wt-%, more preferably from 4 to 20 wt-%, and most preferably from 5 to 15 wt-%.

Finally, the amount of crystallization inhibitor and/or solubilizer needed for effective prevention of agomelatine re-crystallization also depends on the amount of agomelatine present in the agomelatine-containing layer. Thus, the ratio of the total amount of crystallization inhibitor and solubilizer present in the agomelatine-containing layer to the amount of agomelatine present in the agomelatine-containing layer preferably is at least 1 : 2, more preferably at least 1 : 1, and most preferably at least 2:1. This total amount will refer to the amount of crystallization inhibitor in case no solubilizer is present.

### FURTHER ADDITIVES

The agomelatine-containing layer of the TTS according to the invention may comprise further excipients or additives selected from the group consisting of cross-linking agents, further solubilizers, fillers, tackifiers, plasticizers, stabilizers, softeners, substances for skincare, permeation enhancers, i.e. substances which influence the barrier properties of the stratum corneum in the sense of increasing the active agent permeability, pH regulators, and preservatives.

Particularly preferred additives are stabilizers. Such additives may be present in the agomelatine-containing layer in an amount of from 0.001 to 15 wt-% of the agomelatine-containing layer per additive. In a certain embodiment, the total amount of all additives is from 0.001 to 25 wt-% of the agomelatine-containing layer. Hereinafter, where a range for an amount of a specific additive is given, such a range refers to the amount per individual additive.

It should be noted that in pharmaceutical formulations, the formulation components are categorized according to their physicochemical and physiological properties, and in accordance with their function. This means in particular that a substance or a compound falling into one category is not excluded from falling into another category of formulation component. E.g. a certain polymer can be a crystallization inhibitor but also a tackifier. Some substances may e.g. be a typical softener but at the same time act as a permeation enhancer. The skilled person is able to determine based on his general knowledge in which category or categories of formulation component a certain substance or compound belongs to. In the following, details on the excipients and additives are provided which are, however, not to be understood as being exclusive. Other substances not explicitly listed in the present description may be as well used in accordance with the present invention, and substances and/or compounds explicitly listed for one category of formulation component are not excluded from being used as another formulation component in the sense of the present invention.

The cross-linking agent may be selected from the group consisting of aluminium and titanium cross-linking agents such as aluminium acetylacetonate, titanium acetylacetonate or polybutyltitanate. The amount of cross-linking agent may range from 0.005 to 1 wt-%, and preferably from 0.01 to 0.1 wt-% of the agomelatine-containing layer. The agomelatine-containing layer may also comprise a polymer which is self-crosslinking, i.e. comprises a cross-linking functional group such as glycidyl groups, which reacts upon heating. According to a further specific embodiment, the agomelatine-containing layer comprises a cross-linking agent as above and a self-crosslinking polymer.

The agomelatine-containing layer may comprise a further solubilizer in addition to the solubilizer(s) referred to previously. Preferred further solubilizers include, e.g., glycerol-, polyglycerol-, propylene glycol- and polyoxyethylene-esters of medium chain and/or long chain fatty acids, such as glyceryl monolinoleate, medium chain glycerides and medium chain triglycerides, non-ionic solubilizers made by reacting castor oil with ethylene oxide, and any mixtures thereof which may further contain fatty acids or fatty alcohols; cellulose and methyl cellulose and derivatives thereof such as hydroxypropyl cellulose and hypromellose acetate succinate; various cyclodextrins and derivatives thereof; non-ionic tri-block copolymers having a central hydrophobic chain of polyoxypropylene flanked by two hydrophilic chains of polyoxyethylene known as poloxamers; a polyethylene glycol, polyvinyl acetate and polyvinylcaprolactame-based graft copolymer, also abbreviated as PVAc-PVCap- PEG and known as Soluplus^{®}; and purified grades of naturally derived castor oil, of polyethylene glycol 400, of polyoxyethylene sorbitan monooleate (such as polysorbate 80) or of propylene glycols; as well as insoluble / cross-linked polyvinylpyrrolidones also known as crospovidones such as Kollidon^{®} CL, Kollidon^{®} CL-M and Kollidon^{®} CL-SF.

However, also the permeation enhancers mentioned below can act as further solubilizers.

Fillers such as silica gels, titanium dioxide and zinc oxide may be used in conjunction with the polymer in order to influence certain physical parameters, such as cohesion and bond strength, in the desired way.

In case the agomelatine-containing layer is required to have self-adhesive properties and a hydrophobic polymer is selected which does not provide sufficient self-adhesive properties, a tackifier is added. The tackifier may be selected from triglycerides, polyethylene glycols, dipropylene glycol, resins, resin esters, terpenes and derivatives thereof, ethylene vinyl acetate adhesives, dimethylpolysiloxanes and polybutenes, and mixtures thereof. In certain embodiments, the agomelatine-containing layer comprises a tackifier in an amount of from 5 to 15 % of the agomelatine-containing layer.

In certain embodiments, the agomelatine-containing layer comprises a stabilizer selected from sodium metabisulfite, ascorbic acid and ester derivatives thereof, butylated hydroxytoluene, tocopherol and ester derivatives thereof such as tocopheryl acetate and tocopheryl linoleate, preferably from tocopherol and ester derivatives thereof and ascorbic acid and ester derivatives thereof, in particular ascorbyl esters of fatty acids such as ascorbyl palmitate, and α-tocopherol. Where the agomelatine-containing layer comprises a stabilizer, the amount of the stabilizer is from 0.001 to 2 wt-% of the agomelatine-containing layer.

In one embodiment, the agomelatine-containing layer further comprises a softener / plasticizer. Exemplary softeners / plasticizers include linear or branched, saturated or unsaturated alcohols having 6 to 20 carbon atoms, triglycerides and polyethylene glycols.

In certain embodiments, the agomelatine-containing layer comprises a permeation enhancer selected from caprylic acid, glycerol, 2,5-dimethylisosorbid, dimethyl ethylene urea, N,N-diethyl-meta-toluamide, polyethylene glycol, propylene glycol monocaprylat, 2-methoxy-4-(prop-2-en-1-yl)phenol, lactic acid and laurocapram.

In certain other embodiments, the agomelatine-containing layer does not comprise a permeation enhancer selected from diethylene glycol monoethyl ether, diisopropyl adipate, isopropyl myristate, isopropyl palmitate, lactic acid, dimethylethylene urea and dimethylpropylene urea.

The agomelatine-containing layer according to the invention may comprise a pH regulator. Preferably, the pH regulator is selected from amine derivatives, inorganic alkali derivatives, polymers with basic and acidic functionality, respectively.

### RELEASE CHARACTERISTICS

The TTS in accordance with the invention are designed for transdermally administering a certain amount of agomelatine to the systemic circulation in particular during night time.

An administration of the inventive TTS in general and preferably consists of applying the transdermal therapeutic system to the skin of a human patient and maintaining the same on the skin for at least 2 hours, preferably at least 4 hours and more preferably at least 6 hours, and/or for less than or equal to 24 hours, preferably less than or equal to 18 hours, and more preferably less than or equal to 14 hours, and/or for 2 to 24 hours, preferably for 4 to 18 hours, and more preferably for 6 to 14 hours.

In specific embodiments of the invention, the TTS according to the invention as described above provides a skin permeation rate of agomelatine as measured in a Franz diffusion cell with dermatomed human skin of
0.5 µg/cm²-hr to 15 µg/cm²-hr at hour 2,
1 µg/cm²-hr to 20 µg/cm²-hr at hour 4,
2 µg/cm²-hr to 25 µg/cm²-hr at hour 8, and
1 µg/cm²-hr to 15 µg/cm²-hr at hour 16.

In certain embodiments, the transdermal therapeutic system according to the invention provides a cumulative permeated amount of agomelatine as measured in a Franz diffusion cell with dermatomed human skin of at least 0.01 mg/cm², preferably at least 0.015 mg/cm², and more preferably at least 0.02 mg/cm², and/or less than or equal to 0.2 mg/cm², preferably less than or equal to 0.15 mg/cm², and more preferably less than or equal to 0.1 mg/cm², and/or of from 0.01 mg/cm² to 0.2 mg/cm², preferably from 0.015 mg/cm² to 0.15 mg/cm², and more preferably from 0.02 mg/cm² to 0.1 mg/cm² at hour 8.

In certain embodiments, the transdermal therapeutic system according to the invention provides a utilization of agomelatine as measured in a Franz diffusion cell with dermatomed human skin after 8 hours of at least 10%, preferably at least 15 %, and more preferably at least 20 %

### MEDICAL USE

In accordance with a specific aspect of the present invention, the TTS according to the invention is for use in a method of treatment, and in particular in a method of treating a human patient. In accordance with another aspect, the present invention is related to a TTS for use in a method of treatment, wherein the transdermal therapeutic system according to the invention is applied to the skin, in particular to the skin of a human patient. Disclosed, but not claimed, is the use of the inventive transdermal therapeutic system for the manufacture of a medicament for a treatment, preferably for the treatment of a human patient.

The majority of patients, more than 80% who suffer from classical mood disorders (major depression, depressive phases of bipolar disorder, or generalized anxiety disorder) show disruptions of the sleep-wake cycle and of the sleep architecture. Characteristically, difficulty falling asleep (increased sleep latency) followed by fitful sleep results in a pronounced daytime sleepiness that further compromises their ability to function properly in daily life, establishing a vicious cycle. As concerns depression, although there are no specific treatment guidelines, available options generally base themselves on the premise that depression and sleep disturbances share a bidirectional relationship and so, the successful treatment of one of the conditions will reciprocally benefit the other.

In recent years it has become increasingly clear that circadian rhythm disruption - maladjustment and dysfunction of the "body clock" that locks core physiological functions such as body temperature and blood pressure, but also very complex neurotransmitter responses, to the time of the day - is a major factor in major depressive disorder that would warrant therapeutic attention. Malfunctioning of the link between the suprachiasmatic nucleus, the pineal gland, and the neurohormone it produces - melatonin - has been suggested as the main cause for these phenomena. Melatonin has been heavily advocated (and marketed) as a "non-photic circadian rhythm resynchronizing agent" to treat jet lag and insomnia associated with shift working, and a lot has been published on the antidepressant and anxiolytic effects of melatonin. Because the oral bioavailability is low for melatonin, synthetic melatonin receptor agonists have been investigated; the most prominent of which is agomelatine.

While agomelatine is approved for the treatment of depression, treatment of other indications such as bipolar disorders, generalized anxiety disorder, Smith-Magenis syndrome, periventricular leukomalacia and OCD has been suggested.

Thus, in certain embodiments, the TTS according to the invention is preferably for use in a method of treating major depression. Disclosed, but not claimed, is a method of treating major depression, wherein the transdermal therapeutic system according to the invention is applied to the skin, in particular to the skin of a human patient. Disclosed, but not claimed, is the use of the inventive transdermal therapeutic system for the manufacture of a medicament for treating major depression.

The treatment of depression, or major depression, also called major depressive disorder, may include treatment of conditions such as major depressive episodes, anxiety symptoms, sleep-wake cycle disturbances, daytime sleepiness and insomnia (the majority of MDD patients, i.e. over 80 % suffer from depression combined with insomnia) in depressive / MDD patients. In other embodiments, the treatment in general may also refer to treating bipolar disorder, generalized anxiety disorder, Smith-Magenis syndrome, periventricular leukomalacia, or OCD.

As outlined above, transdermal delivery avoids the first-pass effect and thus, the TTS according to the invention has a low risk of hepatotoxicity, unlike oral agomelatine dosage forms. Thus, there is no limitation with respect to the patient group to be treated. Thus, the treatment includes the treatment of human patients with or without hepatic impairment, including those patients with at least mild or at least moderate hepatic impairment.

Also, in a certain embodiment, treatment with the inventive transdermal therapeutic system provides a reduction in at least one agomelatine-related side effect relative to an equivalent oral dose of agomelatine. As outlined above, in certain specific embodiments, such an agomelatine-related side effect is hepatotoxicity. Relative to an equivalent oral dose of agomelatine should be understood as a comparison in the incidence and intensity of side effects in a clinical study when using a dose of transdermal and oral agomelatine that leads substantially to the same blood plasma exposure of agomelatine. The incidence of the at least one agomelatine-related side effect relative to an equivalent oral dose of agomelatine may be reduced by at least about 30 %, preferably at least about 40 %, more preferably at least about 70 % and most preferably at least about 80 %, and/or the intensity of the at least one agomelatine-related side effect relative to an equivalent oral dose of agomelatine may be reduced. The intensity of a side effect can be determined e.g. by classifying the side effects on a scale indicating "mild", "moderate" or "severe" intensity, and a reduction of the intensity can be quantified by comparing the median intensity.

In any of the treatments outlined for the above aspects and embodiments, the transdermal therapeutic system is preferably applied to the skin of a human patient and maintained on the skin for at least 2 hours, preferably at least 4 hours and more preferably at least 6 hours, and/or for less than or equal to 24 hours, preferably less than or equal to 18 hours, and more preferably less than or equal to 14 hours, and/or for 2 to 24 hours, preferably for 4 to 18 hours, and more preferably for 6 to 14 hours.

Disclosed, but not claimed, is that the TTS according to the invention may also be for use in a method of reducing, in a patient, at least one agomelatine-related side effect relative to an equivalent oral dose of agomelatine.

Disclosed, but not claimed, is a method of reducing at least one agomelatine-related side effect in a patient being treated with oral agomelatine therapy, the method comprising
a) discontinuing oral agomelatine therapy; and
b) administering a transdermal therapeutic system according to the invention to the skin of the patient, wherein the transdermal therapeutic system provides a reduction in at least one agomelatine-related side effect relative to an equivalent oral dose of agomelatine.

In such a method, the transdermal therapeutic system may deliver an amount of agomelatine equivalent to the amount of agomelatine originally provided by the oral agomelatine therapy.

### PROCESS OF MANUFACTURE

The invention further relates to a process of manufacture of an agomelatine-containing layer for use in a transdermal therapeutic system and a corresponding self-adhesive layer structure comprising the agomelatine-containing layer and a corresponding TTS.

In accordance with one aspect of the invention, the process of manufacture of an agomelatine-containing layer according to the claims for use in a transdermal therapeutic system comprises the steps of
i) combining at least agomelatine, a hydrophobic polymer, and a crystallization inhibitor selected from the group consisting of polyvinylpyrrolidone and polyvinylpyrrolidone-polyvinylacetate copolymer in a solvent to obtain a coating composition;
ii) coating the coating composition onto a backing layer or a release liner or any intermediate liner; and
iii) drying the coated coating composition to form the agomelatine-containing layer.

In accordance with another aspect of the invention, the process of manufacture of an agomelatine-containing layer according to the claims for use in a transdermal therapeutic system comprises the steps of
i) combining at least agomelatine and a hydrophobic polymer in a solvent to obtain a coating composition;
ii) coating the coating composition onto a backing layer or a release liner or any intermediate liner; and
iii) drying the coated coating composition to form an agomelatine-containing layer of microreservoir-type.

In such a process, the hydrophobic polymer is selected from pressure-sensitive adhesives based on polysiloxanes.

In this process of manufacture, preferably in step i) the agomelatine is dissolved or is dispersed, and more preferably is dissolved to obtain a coating composition.

Since agomelatine is poorly soluble in water and thus would be at risk of recrystallizing, it is preferred that not water is used.

Thus, in the above described process, the solvent may be selected from alcoholic solvents, in particular methanol, ethanol, isopropanol and mixtures thereof, and from non-alcoholic solvents, in particular ethyl acetate, hexane, n-heptane, heptanes, petroleum ether, toluene, and mixtures thereof. Preferably, the solvent comprises an alcoholic solvent selected from methanol, ethanol, isopropanol and mixtures thereof, and more preferably, the solvent comprises ethanol, or consists of ethanol.

In addition, the solvent comprises substantially no water, e.g. the solvent comprises less than 1 wt-%, preferably less than 0.5 wt-% and more preferably less than 0.1 wt-% water.

The preferences for the hydrophobic polymer, the crystallization inhibitor, and other constituents of the agomelatine-containing layer are as outlined above. Thus, in some embodiments, step i) consists of combining at least agomelatine, a hydrophobic polymer, a polyvinylpyrrolidone, and a solubilizer selected from the group consisting of dipropylene glycol, lauryl lactate, mixtures of propylene glycol monoesters and diesters of fatty acids, levulinic acid, polyethylene glycol ethers and diethylene glycol monoethyl ether, in a solvent to obtain a coating composition. Also, in step i), the agomelatine may be combined in dissolved form, in dispersed form, in crystalline form, in particular in one of its polymorph forms, in an amorphous form, as a hydrate, a solvate, a hybrid type form of any of the foregoing forms or a mixture thereof.

In step iii), drying is performed preferably in one or more cycles at room temperature and/or at a temperature of from 40 to 90 °C, more preferably from 50 to 70 °C.

A self-adhesive layer structure comprising the agomelatine-containing layer and a corresponding TTS can be manufactured using the above-outlined process, using further manufacturing steps such as laminating with a backing layer in order to obtain a self-adhesive layer structure, and punching out individual TTS and packaging, e.g. by sealing in a pouch of a primary packaging material, as known to the skilled person. Such further steps preferably lead to a self-adhesive layer structure or a TTS as described in the previous chapters.

The present invention in particular also relates to agomelatine-containing layers as well as self-adhesive layer structures and transdermal therapeutic systems obtainable (and/or obtained) by the above-described processes.

### EXAMPLES

The present invention will now be more fully described with reference to the accompanying examples. It should be understood, however, that the following description is illustrative only and should not be taken in any way as a restriction of the invention. Numerical values provided in the examples regarding the amount of ingredients in the composition or the area weight may vary slightly due to manufacturing variability.

### EXAMPLES 1A-1C

### Coating composition

The formulations of the agomelatine-containing coating compositions of Examples 1a to 1c are summarized in Table 1.1 below. The formulations are based on weight percent, as also indicated in Table 1.1.

**Table 1.1 (Examples 1a and 1b are not according to the claims)**

| **Ingredient (Trade Name)** | **Ex.1a** | | **Ex.1b** | | **Ex. 1c** | |
|---|---|---|---|---|---|---|
| | **Amt [g]** | **Solids [%]** | **Amt [g]** | **Solids [%]** | **Amt [g]** | **Solids [%]** |
| Agomelatine | 0.4 | 8.0 | 0.4 | 7.9 | 0.4 | 8.4 |
| Acrylic adhesive comprising no functional groups in ethyl acetate. Solids content of about 38.4 % by weight (Duro-Tak^{™} (3)87-4098) | 12.0 | 92.0 | - | - | - | - |
| Acrylic adhesive copolymer comprising carboxylic acid groups in ethyl acetate and hexane. Solids content of about 35.9 % by weight (Duro-Tak^{™} (3)87-2353) | - | - | 12.9 | 92.1 | - | - |
| Silicone-based PSA in ethyl acetate. Solids content of about 60.0 % by weight (Bio-PSA Q7-4302) | - | - | - | - | 6.8 | 86.3 |
| Polyvinylpyrrolidone (Povidone K90) | - | - | - | - | 0.25 | 5.3 |
| Ethylene acetate | - | - | 2.0 | - | - | - |
| Ethanol denat. (1 % (v/v) methyl ethyl ketone) | - | - | - | - | 0.8 | - |
| Total | 12.4 | 100.0 | 15.3 | 100.0 | 8.25 | 100.0 |
| Area weight [g/m²] | 46.6 | | 49.5 | | 50.7 | |
| Agomelatine content [µg/cm²] | 373.4 | | 393.4 | | 426.5 | |

### Preparation of the coating composition

For Example 1a, a beaker was loaded with agomelatine. The acrylic pressure sensitive adhesive Duro-Tak^{™} (3)87-4098 was added and the mixture was then stirred until a clear solution was obtained (about 30 min).

For Examples 1b, a beaker was loaded with the agomelatine. The acrylic pressure sensitive adhesive Duro-Tak^{™} (3)87-2353 was added and the mixture was then stirred until a viscose mixture was obtained (about 14 min). Then, ethyl acetate was added under stirring and the homogeneous, clear mixture was stirred for about 2.5 hours.

For Example 1c, a beaker was loaded with agomelatine. The silicone pressure sensitive adhesive Q7-4302 was added and the mixture was then stirred (about 10 min). Initial 0.6 g of ethanol was added under stirring, followed by another 0.2 g of ethanol about 10 minutes later. Then, Povidone K90 was added under stirring, thereby increasing the viscosity. A slightly opaque, homogeneous mixture was obtained.

### Coating of the coating composition

The resulting agomelatine-containing coating composition was coated on a polyester film (polyethylene terephthalate film, one side siliconized, 100 µm thickness, which may function as release liner for Ex. 1a and 1b and 74 µm thickness, which may function as fluoropolymer coated release liner for Ex. 1c) and dried for approx. 10 min at room temperature and 10 min at 60 °C (Ex. 1a) and 70 °C (Ex. 1b and 1c), respectively. The coating thickness gave an area weight of 46.6 g/m² (Ex. 1a), 49.5 (Ex. 1b) g/m², and 50.7 g/m² (Ex. 1c), respectively. The dried film was laminated with a polyethylene terephthalate backing layer (23 µm thickness) to provide an agomelatine-containing self-adhesive layer structure.

### Preparation of the TTS (concerning all examples)

The individual systems (TTS) were then punched out from the agomelatine-containing self-adhesive layer structure. In specific embodiments, a TTS as described above can be provided with a further self-adhesive layer of larger surface area, preferably with rounded corners, comprising a pressure-sensitive adhesive matrix layer which is free of active agent. This is of advantage when the TTS, on the basis of its physical properties alone, does not adhere sufficiently to the skin and/or when the agomelatine-containing matrix layer, for the purpose of avoiding waste, has pronounced corners (square or rectangular shapes). The TTS are then punched out and sealed into pouches of the primary packaging material as conventional in the art, i.e. under protective atmosphere, by flushing with nitrogen gas.

All TTS described herein as examples were free of crystals as observed by the naked eye directly after preparation, unless explicitly indicated.

### Measurement of skin permeation rate

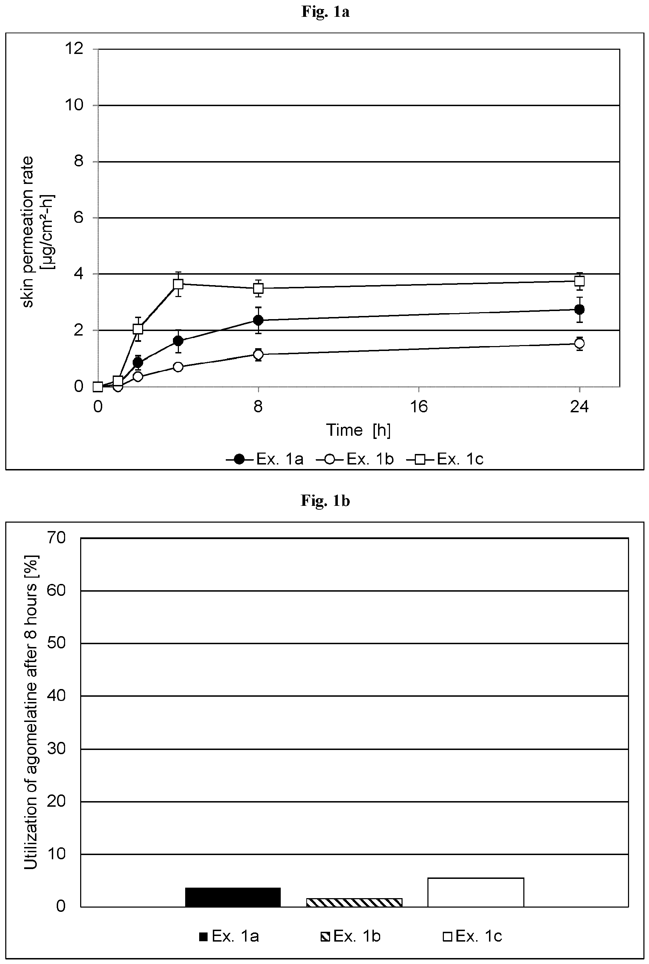

The permeated amount and the corresponding skin permeation rates of TTS prepared according to Examples 1a to 1c were determined by *in vitro* experiments in accordance with the OECD Guideline (adopted April 13, 2004) carried out with a 7.0 ml Franz diffusion cell. Split thickness human skin from cosmetic surgeries (abdomen) was used. A dermatome was used to prepare skin to a thickness of 800 µm, with an intact epidermis for all TTS. Diecuts with an area of 1.156 cm² were punched from the TTS. The agomelatine permeated amount in the receptor medium of the Franz cell (phosphate buffer solution pH 5.5 with 0.1 % saline azide as antibacteriological agent) at a temperature of 32 ± 1°C was measured and the corresponding skin permeation rate calculated. The results are shown in Table 1.2 and Figure 1a.

**Table 1.2**

| **Skin permeation rate with SD [µg/(cm² h)]** | | | | | | |
|---|---|---|---|---|---|---|
| | **Ex. 1a (n = 3)** | | **Ex. 1b (n = 2)** | | **Ex. 1c (n = 3)** | |
| **Elapsed time [h]** | **Rate** | **SD** | **Rate** | **SD** | **Rate** | **SD** |
| **1** | 0.09 | 0.13 | - | - | 0.22 | 0.05 |
| **2** | 0.86 | 0.24 | 0.35 | 0.06 | 2.04 | 0.42 |
| **4** | 1.62 | 0.41 | 0.71 | 0.14 | 3.64 | 0.44 |
| **8** | 2.35 | 0.46 | 1.14 | 0.20 | 3.49 | 0.30 |
| **24** | 2.73 | 0.45 | 1.52 | 0.23 | 3.75 | 0.31 |

| | | | | | | |
|---|---|---|---|---|---|---|
| *: Standard deviation in this Example was, as in all other Examples, calculated based on the n-method. | | | | | | |

### Utilization of agomelatine

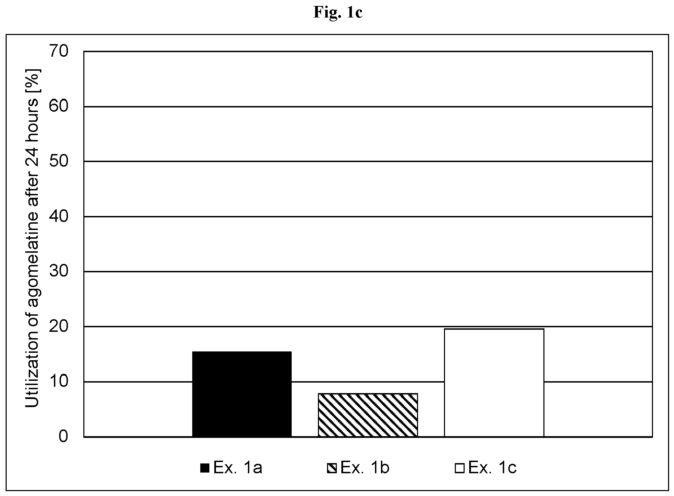

The utilization of agomelatine at 8 hours was calculated based on the cumulative permeated amount at 8 and 24 hours and the initial agomelatine content. The results are shown in Tables 1.3 and 1.4 and in Figures 1b and 1c.

**Table 1.3**

| **Utilization of agomelatine after 8 hours [%]** | | |
|---|---|---|
| **Example 1a (n = 3)** | **Example 1b (n = 3)** | **Example 1c (n = 3)** |
| **3.6** | **1.6** | **5.5** |

**Table 1.4**

| **Utilization of agomelatine after 24 hours [%]** | | |
|---|---|---|
| **Example 1a (n = 3)** | **Example 1b (n = 3)** | **Example 1c (n = 3)** |
| **15.4** | **7.8** | **19.6** |

The *in vitro* experiments show that Example 1c, wherein the agomelatine-containing layer comprises a silicone-based PSA as a hydrophobic polymer and PVP as crystallization inhibitor in accordance with certain aspects and embodiments of the invention, provides a superior skin permeation rate when compared to Examples 1a and 1b, which are based on an acrylic polymer as adhesive.

### EXAMPLES 2A-2E

### Coating composition

The formulations of the agomelatine-containing coating compositions of Examples 2a to 2e are summarized in Table 2.1 below. The formulations are based on weight percent, as also indicated in Table 2.1.

**Table 2.1 (examples 2a to 2d are not according to the claims)**

| **Ingredient (Trade Name)** | **Ex. 2a** | | **Ex. 2b** | | **Ex. 2c** | | **Ex. 2d** | | **Ex. 2e** | |
|---|---|---|---|---|---|---|---|---|---|---|
| | **Amt [g]** | **Solids [%]** | **Amt [g]** | **Solids [%]** | **Amt [g]** | **Solids [%]** | **Amt [g]** | **Solids [%]** | **Amt [g]** | **Solids [%]** |
| Agomelatine | 0.2 | 3.9 | 0.2 | 4.0 | 0.2 | 4.1 | 0.2 | 4.1 | 0.2 | 4.0 |
| Silicone acrylic hybrid PSA adhesives in n-heptane. Solid content of about 51.0 % by weight (SilAc PSA 7-6301) | 7.9 | 77.8 | 9.1 | 79.4 | - | - | - | - | - | - |
| Acrylic adhesive in ethyl acetate, ethanol, heptanes and methanol. Solids content of about 41.5 % by weight (Duro-Tak^{™} (3)87-2516) | - | - | - | - | 9.8 | 80.9 | 9.8 | 80.8 | - | - |
| Silicone-based PSA in ethyl acetate. Solids content of about 60.0 % by weight (Bio-PSA Q7-4302) | - | - | - | - | - | - | - | - | 5.9 | 71.0 |
| Polyvinylpyrrolidone (Povidone K90) | 0.2 | 3.9 | 0.2 | 3.8 | - | - | - | - | 0.5 | 10.0 |
| Capryol 90 | - | - | 0.7 | 12.8 | - | - | 0.8 | 15.1 | - | - |
| Lauryl lactate | 0.8 | 14.4 | - | - | 0.8 | 15.0 | - | - | 0.7 | 15.0 |
| Ethanol denat. (1 % (v/v) methyl ethyl ketone) | 2.1 | - | 2.4 | - | - | - | - | - | 1.4 | - |
| Ethyl acetate | - | - | - | - | 0.8 | - | 0.8 | - | - | - |
| Total | 11.2 | 100.0 | 12.6 | 100.0 | 11.6 | 100.0 | 11.6 | 100.0 | 8.7 | 100.0 |
| Area weight [g/m²] | 47.9 | | 45.7 | | 49.1 | | 47.8 | | 55.9 | |
| Agomelatine content [µg/cm²] | 186.1 | | 181.4 | | 201.6 | | 194.1 | | 223.5 | |

### Preparation of the coating composition

For Examples 2a to 2d, a beaker was loaded with agomelatine. The solvent (ethanol for Ex. 2a and 2b and ethyl acetate for Ex. 2c and 2d), the pressure sensitive adhesive (SilAc Hybrid PSA 7-6301 for Ex. 2a and 2b and Duro-Tak^{™} (3)87-2516 for Ex. 2c and 2d) and the lauryl lactate (Ex. 2a and 2c) or the Capryol 90 (Ex. 2b and 2d), respectively, were added. The mixture was then stirred. If used, the polyvinylpyrrolidone was added (Ex. 2a and 2b) under stirring to obtain a white, homogeneous mixture after about 2 hours stirring.

For Example 2e, a beaker was loaded with agomelatine. The pressure sensitive silicone adhesive Q7-4302, the lauryl lactate and the ethanol were added followed by stirring. The polyvinylpyrrolidone was added under stirring to obtain an opaque, homogeneous mixture after about 2 hours stirring.

### Coating of the coating composition

See Example 1c for Examples 2a, 2b, and 2e for the coating process. The coating thickness gave an area weight of 47.9 g/m² (Ex. 2a), 45.7 g/m² (Ex. 2b), and 55.9 g/m² (Ex. 2e), respectively. The dried film was laminated with a polyethylene terephthalate backing layer (23 µm thickness) to provide an agomelatine-containing self-adhesive layer structure.

The resulting agomelatine-containing coating composition for Example 2c and 2d, was coated on a polyethylene terephthalate film (one side siliconized, 75 µm thickness, which may function as release liner) and dried for approx. 10 min at room temperature and 10 min at 70 °C. The coating thickness gave an area weight of 49.1 g/m² (Ex. 2c) and 47.8 g/m² (Ex. 2d), respectively. The dried film was laminated with a polyethylene terephthalate backing layer (23 µm thickness) to provide an agomelatine-containing self-adhesive layer structure.

### Preparation of the TTS

See Example 1.

### Measurement of skin permeation rate

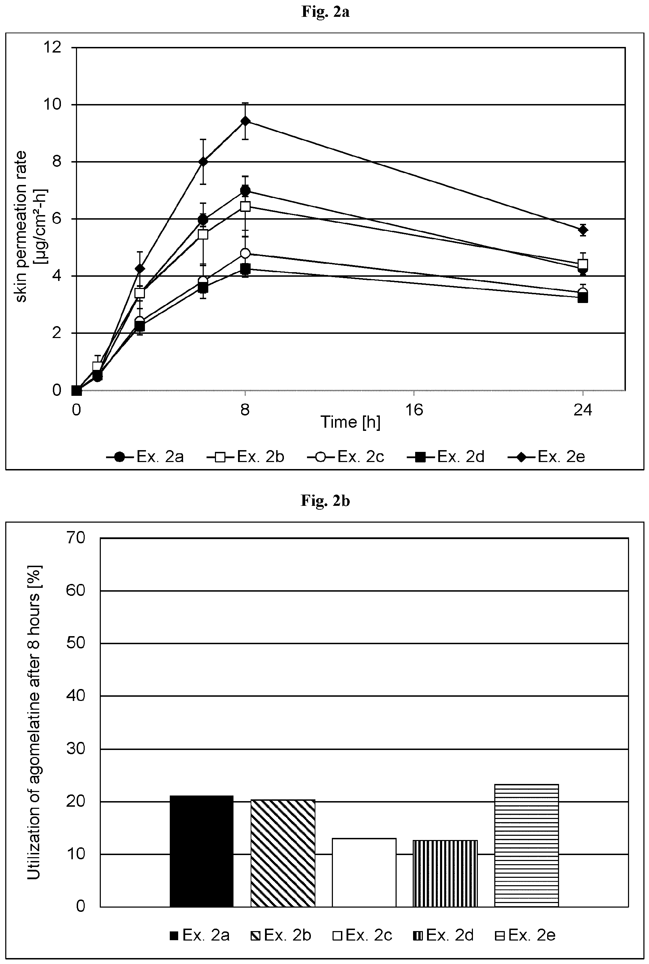

The permeated amount and the corresponding skin permeation rates of TTS prepared according to Examples 2a to 2e were determined as in Example 1 above. Split thickness human skin from cosmetic surgeries (abdomen) was used. Diecuts with an area of 1.154 cm² were punched from the TTS. The results are shown in Table 2.2 and Figure 2a.

**Table 2.2**

| **Skin permeation rate with SD [µg/(cm² h)]** | | | | | | | | | | |
|---|---|---|---|---|---|---|---|---|---|---|
| **Elapsed time [h]** | **Ex. 2a (n = 3)** | | **Ex. 2b (n = 3)** | | **Ex. 2c (n = 3)** | | **Ex. 2d (n = 3)** | | **Ex. 2e (n = 3)** | |
| | **Rate** | **SD** | **Rate** | **SD** | **Rate** | **SD** | **Rate** | **SD** | **Rate** | **SD** |
| **1** | 0.53 | 0.07 | 0.83 | 0.38 | 0.48 | 0.10 | 0.54 | 0.08 | 0.5 | 0.15 |
| **3** | 3.39 | 0.26 | 3.39 | 0.88 | 2.40 | 0.46 | 2.25 | 0.18 | 4.25 | 0.60 |
| **6** | 5.96 | 0.22 | 5.46 | 1.09 | 3.82 | 0.60 | 3.60 | 0.18 | 8.00 | 0.79 |
| **8** | 6.98 | 0.20 | 6.44 | 1.05 | 4.78 | 0.81 | 4.25 | 0.19 | 9.42 | 0.63 |
| **24** | 4.25 | 0.20 | 4.41 | 0.40 | 3.41 | 0.30 | 3.24 | 0.12 | 5.61 | 0.19 |

| | | | | | | | | | | |
|---|---|---|---|---|---|---|---|---|---|---|
| *: Standard deviation in this Example was, as in all other Examples, calculated based on the n-method. | | | | | | | | | | |

### Utilization of agomelatine

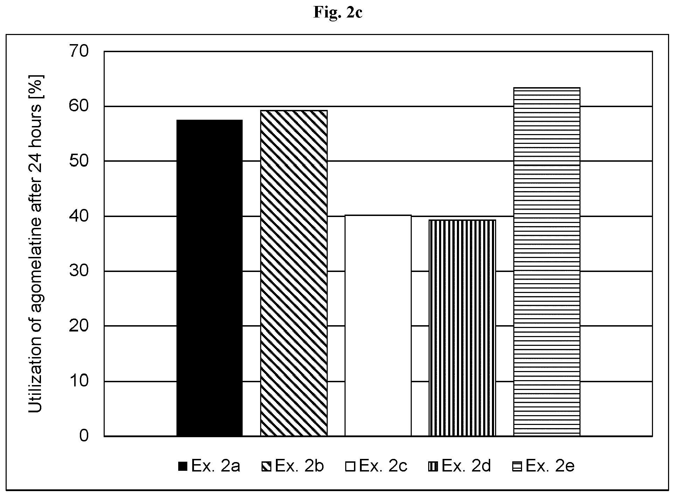

The utilization of agomelatine at 8 hours was calculated based on the cumulative permeated amount at 8 and 24 hours and the initial agomelatine content. The results are shown in Tables 2.3 and 2.4 and in Figures 2b and 2c.

**Table 2.3**

| **Utilization of agomelatine after 8 hours [%]** | | | | |
|---|---|---|---|---|
| **Example 2a (n = 3)** | **Example 2b (n = 3)** | **Example 2c (n = 3)** | **Example 2d (n = 3)** | **Example 2e (n = 3)** |
| **21.1** | **20.3** | **13.0** | **12.6** | **23.2** |

**Table2.4**

| **Utilization of agomelatine after 24 hours [%]** | | | | |
|---|---|---|---|---|
| **Example 2a (n = 3)** | **Example 2b (n = 3)** | **Example 2c (n = 3)** | **Example 2d (n = 3)** | **Example 2e (n = 3)** |
| **57.5** | **59.2** | **40.2** | **39.3** | **63.4** |

The *in vitro* experiments show that Examples 2a and 2b, wherein the agomelatine-containing layer comprises a silicone acrylic hybrid polymer as a hydrophobic polymer and PVP as crystallization inhibitor as well as Capryol 90 or lauryl lactate as solubilizer in accordance with certain aspects and embodiments of the invention, provide a superior skin permeation rate when compared to Examples 2c and 2d, which are based on an acrylic polymer as adhesive. Example 2e, based on a silicone-based PSA as a hydrophobic polymer and PVP as crystallization inhibitor and Lauryl lactate as solubilizer, provides even better skin permeation rates.

All Examples 2a to 2e are based on 4 wt-% active concentration instead of 8 wt-% of Examples 1a to 1c with the area weight being maintained at the same level, which is why the total active content per area of release is lower in Examples 2a to 2e (about half of that of Examples 1a to 1c), which may be the reason for the advantageous release profile of Examples 2a to 2e showing a decrease in the permeation rate from hour 8 to hour 24, in contrast to Examples 1a to 1c.

### EXAMPLES 3A-3D

### Coating composition

The formulations of the agomelatine-containing coating compositions of Examples 3a to 3d are summarized in Table 3.1 below. The formulations are based on weight percent, as also indicated in Table 3.1.

**Table 3.1**

| **Ingredient (Trade Name)** | **Ex. 3a** | | **Ex. 3b** | | **Ex. 3c** | | **Ex. 3d** | |
|---|---|---|---|---|---|---|---|---|
| | **Amt [g]** | **Solids [%]** | **Amt [g]** | **Solids [%]** | **Amt [g]** | **Solids [%]** | **Amt [g]** | **Solids [%]** |
| Agomelatine | 0.2 | 4.0 | 0.2 | 4.0 | 0.2 | 4.0 | 0.2 | 4.0 |
| Silicone-based PSA in ethyl acetate. Solids content of about 60.0 % by weight (Bio-PSA Q7-4302) | 7.6 | 91.0 | 6.8 | 80.8 | 7.2 | 85.8 | 7.2 | 85.7 |
| Polyvinylpyrrolidone (Povidone K90) | 0.3 | 5.0 | 0.3 | 5.0 | 0.3 | 5.0 | 0.3 | 5.0 |
| Lauryl lactate | - | - | - | - | - | - | - | - |
| Transcutol (diethylene glycol monoethyl ether) | - | - | 0.5 | 10.2 | - | - | - | - |
| Dipropylene glycol | - | - | - | - | 0.3 | 5.2 | - | - |
| Levulinic acid | - | - | - | - | - | - | 0.3 | 5.3 |
| Ethanol denat. (1 % (v/v) methyl ethyl ketone) | 0.8 | - | 0.8 | - | 0.8 | - | 0.8 | - |
| Total | 8.9 | 100.0 | 8.6 | 100.0 | 8.8 | 100.0 | 8.8 | 100.0 |
| Area weight [g/m²] | 63.4 | | 63.5 | | 63.3 | | 76.8 | |
| Agomelatine content [µg/cm²] | 252.6 | | 251.3 | | 253.6 | | 307.0 | |

### Preparation of the coating composition

For Examples 3a to 3d, a beaker was loaded with agomelatine. The ethanol, the transcutol (Ex. 3b) or the dipropylene glycol (Ex. 3c) or the levulinic acid (Ex. 3d), and the pressure sensitive adhesive (Q7-4302) were added. The mixture was then stirred. The polyvinylpyrrolidone was added under stirring to obtain an opaque, homogeneous mixture after about 3 hours stirring.

### Coating of the coating composition

The resulting agomelatine-containing coating composition of Examples 3a to 3d was coated on a polyester film (74 µm thickness, which may function as fluoropolymer coated release liner) and dried for approx. 10 min at room temperature and 10 min at 70 °C. The coating thickness gave an area weight of 63.4 g/m² (Ex. 3a), 63.5 g/m² (Ex. 3b), 63.3 g/m² (Ex. 3c), and 76.8 g/m² (Ex. 3d), respectively. The dried film was laminated with a polyethylene terephthalate backing layer (23 µm thickness) to provide an agomelatine-containing self-adhesive layer structure.

### Microscopic observation

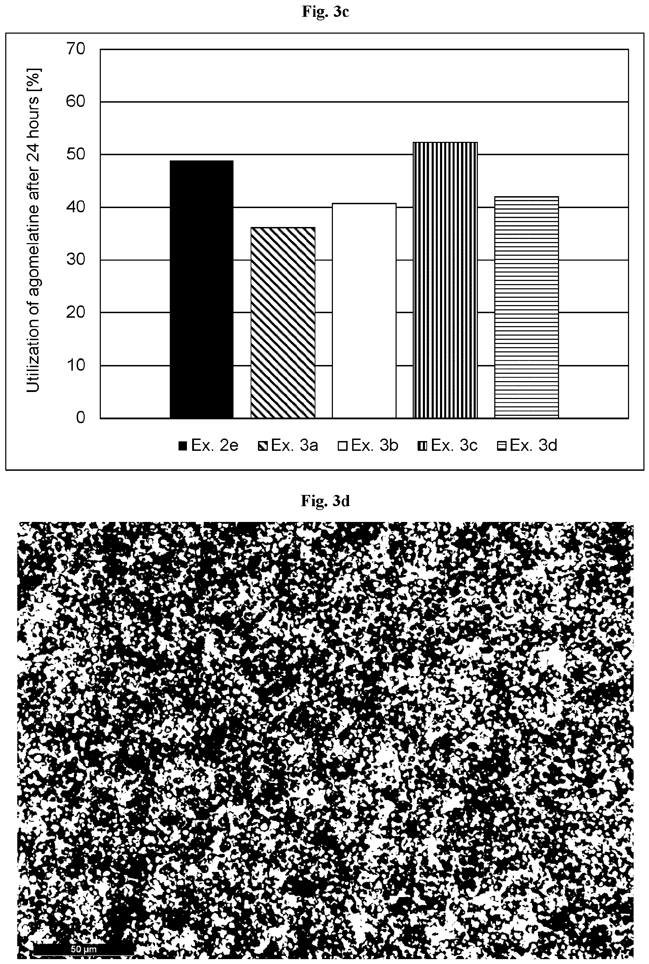

The agomelatine-containing layers of Examples 3a to 3d were observed using a microscope (Leica DM6000M microscope with digital camera DFC450 and Leica Application Suite Version 4.5) shortly after manufacture and after 4 weeks of storage. The layers of all observed examples showed droplets (approximate maximum droplet size of about 5 µm) and thus were determined to be of microreservoir-type. Fig. 3d shows a microscopic picture of the agomelatine-containing layer of Example 3d after 4 weeks of storage. Example 3d was also observed after 2 years of storage and still presented a microreservoir free of crystals with slightly larger droplets (approximate maximum droplet size of about 10 µm).

### Preparation of the TTS

See Example 1.

### Measurement of skin permeation rate

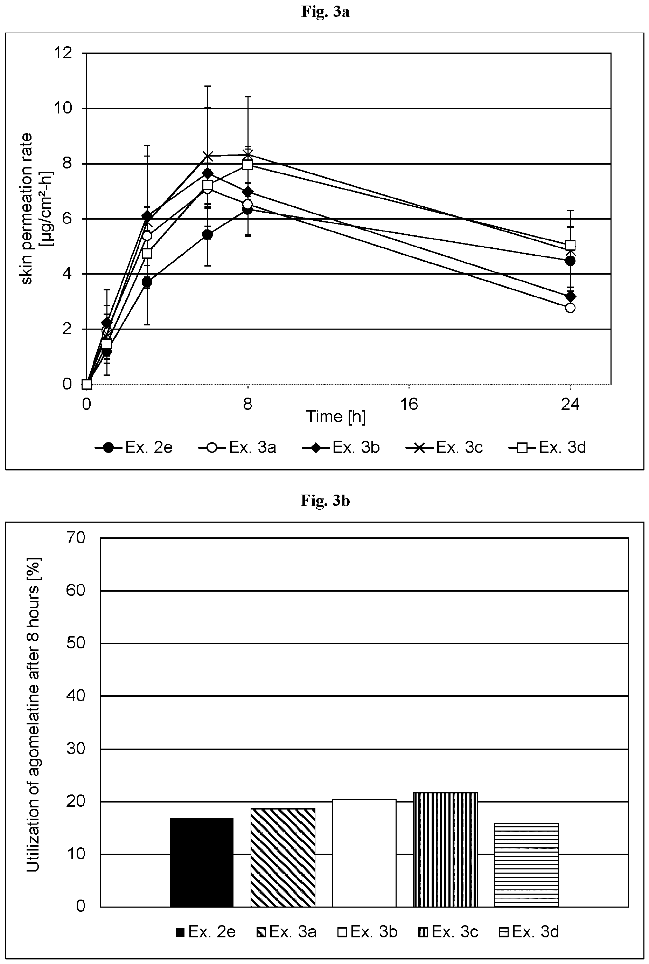

The permeated amount and the corresponding skin permeation rates of TTS prepared according to Examples 2e and 3a to 3d were determined by *in vitro* experiments in accordance with the OECD Guideline (adopted April 13, 2004) carried out with a 7.0 ml Franz diffusion cell. Split thickness minipig skin (female abdomen) was used. A dermatome was used to prepare skin to a thickness of 800 µm, with an intact epidermis for all TTS. Diecuts with an area of 1.154 cm² were punched from the TTS. The agomelatine permeated amount in the receptor medium of the Franz cell (phosphate buffer solution pH 5.5 with 0.1 % saline azide as antibacteriological agent) at a temperature of 32 ± 1°C was measured and the corresponding skin permeation rate calculated. The results are shown in Table 3.2 and Figure 3a.

**Table 3.2**

| **Skin permeation rate with SD [µg/(cm² h)]** | | | | | | | | | | |
|---|---|---|---|---|---|---|---|---|---|---|
| **Elapsed time [h]** | **Ex. 2e (n = 3)** | | **Ex. 3a (n = 3)** | | **Ex. 3b (n = 3)** | | **Ex. 3c (n = 3)** | | **Ex. 3d (n = 3)** | |
| | **Rate** | **SD** | **Rate** | **SD** | **Rate** | **SD** | **Rate** | **SD** | **Rate** | **SD** |
| **1** | 1.20 | 0.86 | 1.94 | 0.60 | 2.23 | 1.20 | 1.82 | 1.05 | 1.47 | 0.54 |
| **3** | 3.70 | 1.54 | 5.37 | 1.06 | 6.10 | 2.56 | 5.88 | 2.40 | 4.73 | 0.84 |
| **6** | 5.42 | 1.12 | 7.08 | 0.69 | 7.65 | 2.38 | 8.27 | 2.54 | 7.22 | 0.80 |
| **8** | 6.34 | 0.96 | 6.52 | 0.30 | 6.98 | 1.55 | 8.32 | 2.11 | 7.95 | 0.67 |
| **24** | 4.48 | 0.14 | 2.77 | 0.09 | 3.18 | 0.35 | 4.85 | 1.46 | 5.03 | 0.68 |

| | | | | | | | | | | |
|---|---|---|---|---|---|---|---|---|---|---|
| *: Standard deviation in this Example was, as in all other Examples, calculated based on the n-method. | | | | | | | | | | |

### Utilization of agomelatine

The utilization of agomelatine at 8 hours was calculated based on the cumulative permeated amount at 8 and 24 hours and the initial agomelatine content. The results are shown in Tables 3.3 and 3.4 and in Figures 3b and 3c.

**Table 3.3**

| **Utilization of agomelatine after 8 hours [%]** | | | | |
|---|---|---|---|---|
| **Example 2e (n = 3)** | **Example 3a (n = 3)** | **Example 3b (n = 3)** | **Example 3c (n = 3)** | **Example 3 (n = 3)** |
| **16.8** | **18.6** | **20.4** | **21.7** | **15.8** |

**Table 3.4**

| **Utilization of agomelatine after 24 hours [%]** | | | | |
|---|---|---|---|---|
| **Example 2e (n = 3)** | **Example 3a (n = 3)** | **Example 3b (n = 3)** | **Example 3c (n = 3)** | **Example 3 (n = 3)** |
| **48.8** | **36.1** | **40.7** | **52.3** | **42.0** |

The *in vitro* experiments show a good skin permeation rate, with an advantageous release profile showing a decrease in the permeation rate from hour 8 to hour 24, as well as satisfying utilizations for all tested examples, which exemplify formulations including PVP as crystallization inhibitor as well as various substances as solubilizer.

### EXAMPLES 4A-4E

### Coating composition

The formulations of the agomelatine-containing coating compositions of Examples 4a to 4e are summarized in Table 4.1 below. The formulations are based on weight percent, as also indicated in Table 4.1.

**Table 4.1 (Examples 4a and 4b are not according to the claims)**

| **Ingredient (Trade Name)** | **Ex. 4a** | | **Ex. 4b** | | **Ex. 4c** | | **Ex. 4d** | | **Ex. 4e** | |
|---|---|---|---|---|---|---|---|---|---|---|
| | **Amt [g]** | **Solids [%]** | **Amt [g]** | **Solids [%]** | **Amt [g]** | **Solids [%]** | **Amt [g]** | **Solids [%]** | **Amt [g]** | **Solids [%]** |
| Agomelatine | 0.1 | 2.0 | 0.1 | 2.0 | 0.1 | 2.0 | 0.1 | 2.0 | 0.1 | 2.0 |
| Silicone-based PSA in ethyl acetate. Solids content of about 60.0 % by weight (Bio-PSA Q7-4302) | 8.2 | 98.0 | 7.8 | 93.0 | 8.1 | 95.5 | 7.8 | 92.6 | 7.7 | 90.5 |
| Polyvinylpyrrolidone (Povidone K90) | - | - | - | - | 0.1 | 2.6 | 0.1 | 2.6 | 0.1 | 2.6 |
| Dipropylene glycol | - | - | 0.3 | 5.1 | - | - | 0.1 | 2.8 | - | - |
| Levulinic acid | - | - | - | - | - | - | - | - | 0.3 | 5.0 |
| Ethanol denat. (1 % (v/v) methyl ethyl ketone) | - | - | - | - | 0.6 | - | 0.6 | - | 0.7 | - |
| Ethyl acetate | 0.7 | - | 0.6 | - | - | - | - | - | - | - |
| Total | 9.0 | 100.0 | 8.8 | 100.1 | 8.9 | 100.1 | 8.7 | 100.0 | 8.9 | 100.1 |
| Area weight [g/m²] | 41.9 | | 41.9 | | 45.3 | | 52.2 | | 46.7 | |
| Agomelatine content [µg/cm²] | 83.6 | | 82.8 | | 88.7 | | 104.4 | | 91.4 | |

### Preparation of the coating composition

For example 4a, a beaker was loaded with agomelatine. The ethyl acetate and the pressure sensitive adhesive (Q7-4302) were added. The mixture was then stirred to obtain a clear mixture after about 2 hours.

For Examples 4b to 4e, a beaker was loaded with agomelatine. The solvent (ethyl acetate for Ex. 4b and ethanol for Ex. 4c to 4e), the dipropylene glycol (Ex. 4b and 4d) or the levulinic acid (Ex. 4e), and the pressure sensitive adhesive (Q7-4302) were added. The mixture was then stirred. If used, the polyvinylpyrrolidone was added (Ex. 4c to 4e) under stirring to obtain an opaque, homogeneous mixture after about 1 to 2 hours stirring.

### Coating of the coating composition

The resulting agomelatine-containing coating composition of Examples 4a to 4e was coated on a polyester film (74 µm thickness, which may function as fluoropolymer coated release liner) and dried for approx. 10 min at room temperature and 10 min at 60 °C (Ex. 4a and 4b) and 10 min at 70 °C (Ex. 4c to 4e), respectively. Where necessary, the coating and drying was repeated to achieve the desired coating thickness The coating thickness gave an area weight of 41.9 g/m² (Ex. 4a), 41.9 g/m² (Ex. 4b), 45.3 g/m² (Ex. 4c), 52.2 g/m² (Ex. 4d), and 46.7 g/m² (Ex. 4e), respectively. The dried film was laminated with a polyethylene terephthalate backing layer (23 µm thickness) to provide an agomelatine-containing self-adhesive layer structure.

### Microscopic observation

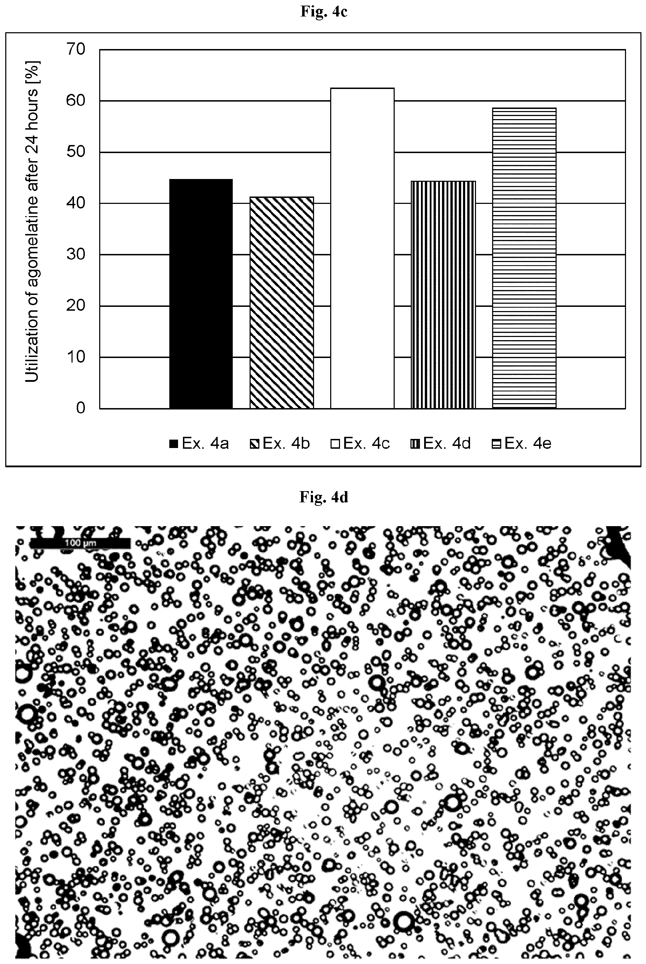

The agomelatine-containing layers of Examples 4b and 4c were observed using a microscope (Leica DM6000M microscope with digital camera DFC450 and Leica Application Suite Version 4.5) shortly after manufacture. These layers showed droplets (approximate maximum droplet size of about 10 and 15 µm) and thus were determined to be of microreservoir-type. Fig. 4d shows a microscopic picture of the agomelatine-containing layer of Example 4c 1 day after manufacture.

### Preparation of the TTS

See Example 1.

### Measurement of skin permeation rate

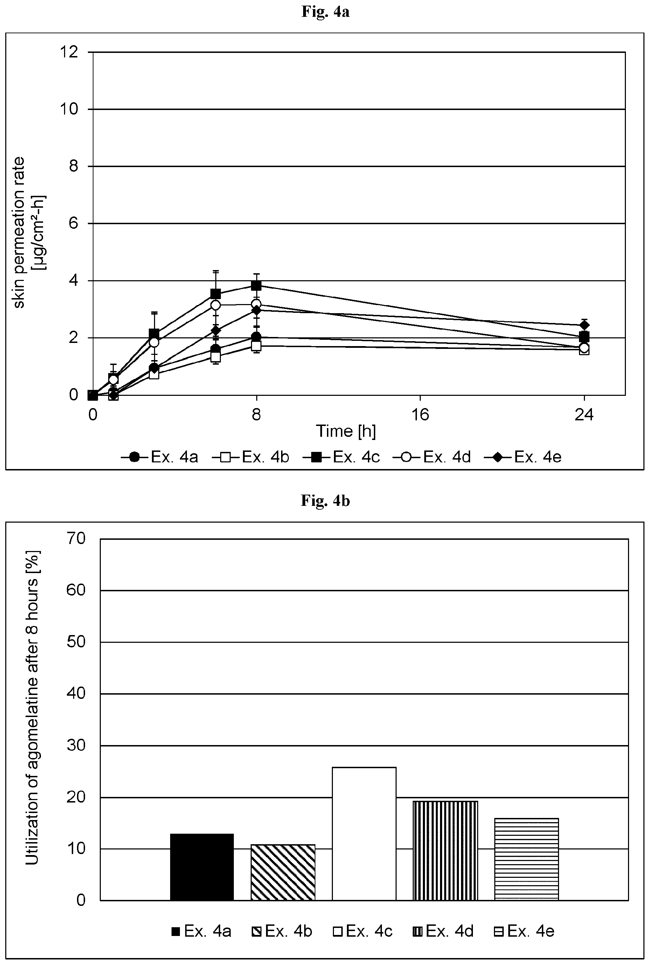

The permeated amount and the corresponding skin permeation rates of TTS prepared according to Example 4a to 4e were determined as in Example 3 above. The results are shown in Table 4.2 and Figure 4a.

**Table 4.2**

| **Skin permeation rate with SD [µg/(cm² h)]** | | | | | | | | | | |
|---|---|---|---|---|---|---|---|---|---|---|
| **Elapsed time [h]** | **Ex. 4a (n = 3)** | | **Ex. 4b (n = 3)** | | **Ex. 4c (n = 3)** | | **Ex. 4d (n = 3)** | | **Ex. 4e (n = 3)** | |
| | **Rate** | **SD** | **Rate** | **SD** | **Rate** | **SD** | **Rate** | **SD** | **Rate** | **SD** |
| **1** | 0.10 | 0.15 | - | - | 0.58 | 0.25 | 0.54 | 0.54 | - | - |
| **3** | 0.94 | 0.27 | 0.74 | 0.14 | 2.13 | 0.71 | 1.83 | 1.07 | 0.94 | 0.14 |
| **6** | 1.60 | 0.38 | 1.34 | 0.25 | 3.53 | 0.75 | 3.14 | 1.21 | 2.26 | 0.20 |
| **8** | 2.03 | 0.38 | 1.71 | 0.23 | 3.83 | 0.41 | 3.17 | 0.79 | 2.96 | 0.26 |
| **24** | 1.66 | 0.10 | 1.57 | 0.13 | 2.03 | 0.14 | 1.65 | 0.09 | 2.44 | 0.21 |

| | | | | | | | | | | |
|---|---|---|---|---|---|---|---|---|---|---|
| *: Standard deviation in this Example was, as in all other Examples, calculated based on the n-method. | | | | | | | | | | |

### Utilization of agomelatine

The utilization of agomelatine at 8 hours was calculated based on the cumulative permeated amount at 8 and 24 hours and the initial agomelatine content. The results are shown in Tables 4.3 and 4.4 and in Figures 4b and 4c.

**Table 4.3**

| **Utilization of agomelatine after 8 hours [%]** | | | | |
|---|---|---|---|---|
| **Example 4a (n = 3)** | **Example 4b (n = 3)** | **Example 4c (n = 3)** | **Example 4d (n = 3)** | **Example 4e (n = 3)** |
| **12.9** | **10.8** | **25.8** | **19.1** | **15.9** |

**Table 4.4**

| **Utilization of agomelatine after 24 hours [%]** | | | | |
|---|---|---|---|---|
| **Example 4a (n = 3)** | **Example 4b (n = 3)** | **Example 4c (n = 3)** | **Example 4d (n = 3)** | **Example 4e (n = 3)** |
| **44.7** | **41.2** | **62.5** | **44.3** | **58.6** |

These Examples show that the agomelatine utilization and the release profile as determined by *in vitro* experiments is much better, both in terms of fast onset of permeation, as well as absolute permeation rates and decrease of the permeation from hour 8 to hour 24, for Examples 4c, 4d and 4e, which include PVP as crystallization inhibitor in the agomelatine-containing layer when compared to Examples 4a and 4b.

### EXAMPLES 5A-5D

### Coating composition

The formulations of the agomelatine-containing coating compositions of Examples 5a to 5d are summarized in Table 5.1 below. The formulations are based on weight percent, as also indicated in Table 5.1.

**Table 5.1 (Examples 5a to 5c are not according to the claims)**

| **Ingredient (Trade Name)** | **Ex. 5a** | | **Ex. 5b** | | **Ex. 5c** | | **Ex. 5d** | |
|---|---|---|---|---|---|---|---|---|
| | **Amt [g]** | **Solids [%]** | **Amt [g]** | **Solids [%]** | **Amt [g]** | **Solids [%]** | **Amt [g]** | **Solids [%]** |
| Agomelatine | 0.2 | 4.0 | 0.2 | 4.0 | 0.2 | 4.0 | 0.2 | 4.0 |
| Silicone acrylic hybrid PSA adhesives in heptane. Solid content of about 51.0 % by weight (SilAc PSA 7-6301) | 8.4 | 85.9 | - | - | 8.4 | 85.9 | - | - |
| Polyisobutylene adhesive in petroleum benzine, bp 80-110 °C. Solid content of about 41.0 % by weight (Oppanol B10/B100 85/15) | - | - | 10.5 | 85.3 | - | - | - | - |
| Silicone-based PSA in ethyl acetate. Solids content of about 60.0 % by weight (Bio-PSA Q7-4302) | - | - | - | - | - | - | 7.6 | 91.0 |
| Polyvinylpyrrolidone, dissolved in ethanol (Povidone K90F). Solid content of about 25.0 % by weight. | - | - | 1.1 | 5.6 | - | - | - | - |
| Polyvinylpyrrolidone (Povidone 12PF) | - | - | - | - | - | - | 0.2 | 5.0 |
| Polyvinylpyrrolidone (Povidone K64) | - | - | - | - | 0.3 | 5.0 | - | - |
| Dipropylene glycol | 0.5 | 10.1 | 0.3 | 5.2 | 0.3 | 5.1 | - | - |
| Ethanol denat. (1 % (v/v) methyl ethyl ketone) | 0.7 | - | - | - | 0.5 | - | 0.4 | - |
| Total | 9.8 | 100.0 | 12.1 | 100.1 | 9.7 | 100.0 | 8.4 | 100.0 |
| Area weight [g/m²] | 37.4 | | 64.6 | | 52.8 | | 60.6 | |
| Agomelatine content [µg/cm²] | 149.2 | | 255.8 | | 211.5 | | 242.1 | |

### Preparation of the coating composition

For Examples 5a and 5c, a beaker was loaded with agomelatine. The dipropylene glycol, the pressure sensitive adhesive (SilAc PSA 7-6301), and, if used, the polyvinylpyrrolidone (Ex. 5c) were added. The mixture was then stirred. The ethanol was added under stirring. A white, homogeneous mixture (Ex. 5a and 5c) was obtained after about 1 to 2 hours.

For Example 5b, a beaker was loaded with agomelatine. The polyvinylpyrrolidone, the dipropylene glycol, and the adhesive (Oppanol B10/B100 85/15) were added and then mixed to obtain after about 3 hours an opaque, homogeneous mixture.

For Example 5d, a beaker was loaded with agomelatine. The pressure sensitive adhesive (Q7-4302) was added. The mixture was then stirred. The polyvinylpyrrolidone and the ethanol were added under stirring to obtain after about 1 hour an opaque, homogeneous mixture.

### Coating of the coating composition

The resulting agomelatine-containing coating composition of Examples 5a to 5d was coated on a polyester film (74 µm thickness, which may function as fluoropolymer coated release liner for Ex. 5a, 5c, and 5d and polyethylene terephthalate film, one side siliconized, 75 µm thickness, which may function as release liner for Ex. 5b) and dried for approx. 10 min at room temperature and 10 min at 70 °C (Ex. 5a, 5c, and 5d) and 10 min at 90 °C (Ex. 5b), respectively. The coating thickness gave an area weight of 37.4 g/m² (Ex. 5a), 64.6 g/m² (Ex. 5b), 52.8 g/m² (Ex. 5c), and 60.6 g/m² (Ex. 5d), respectively. The dried film was laminated with a polyethylene terephthalate backing layer (23 µm thickness) to provide an agomelatine-containing self-adhesive layer structure.

### Microscopic observation

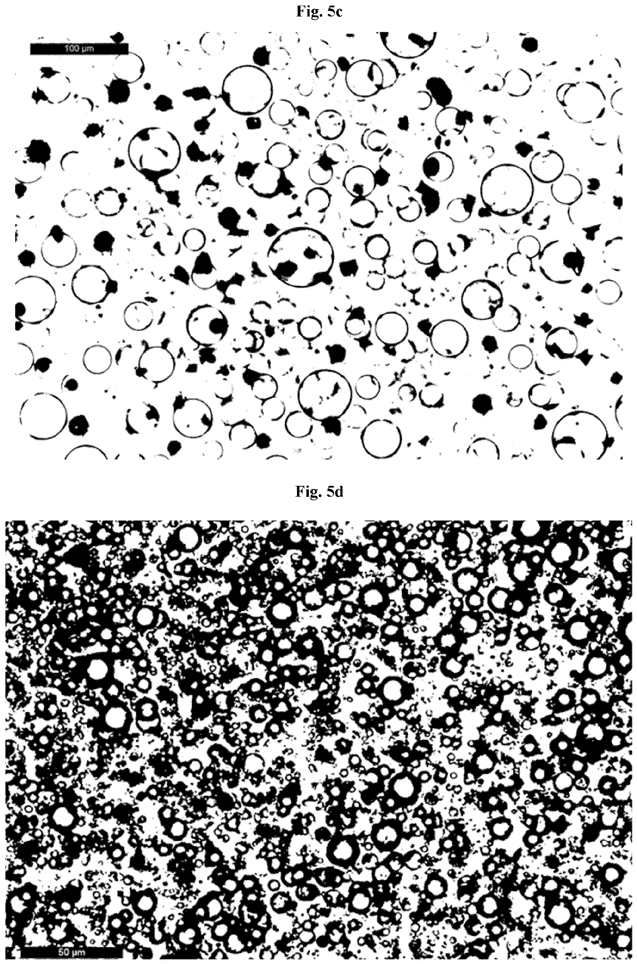

The agomelatine-containing layers of Examples 5a to 5d were observed using a microscope (Leica DM6000M microscope with digital camera DFC450 and Leica Application Suite Version 4.5) shortly after manufacture (3-4 days, Examples 5a and 5d) and after about 32 months (Examples 5b and 5c). The layers of all examples showed droplets and thus were determined to be of microreservoir-type. Example 5d (based on a silicone-based PSA as hydrophobic polymer) showed a maximum droplet size of about 15 µm, while the droplet size of Example 5b (based on polyisobutylene as hydrophobic polymer) was relatively large (maximum droplet size of about 60 µm). The droplet size in Examples 5a and 5c (based on a silicone acrylic hybrid PSA as hydrophobic polymer) was so small that the droplets were difficult to distinguish to the naked eye under the microscope. Fig. 5c and 5d show a microscopic picture of the agomelatine-containing layers of Examples 5b and 5d, respectively.

### Preparation of the TTS

See Example 1.

### Measurement of skin permeation rate

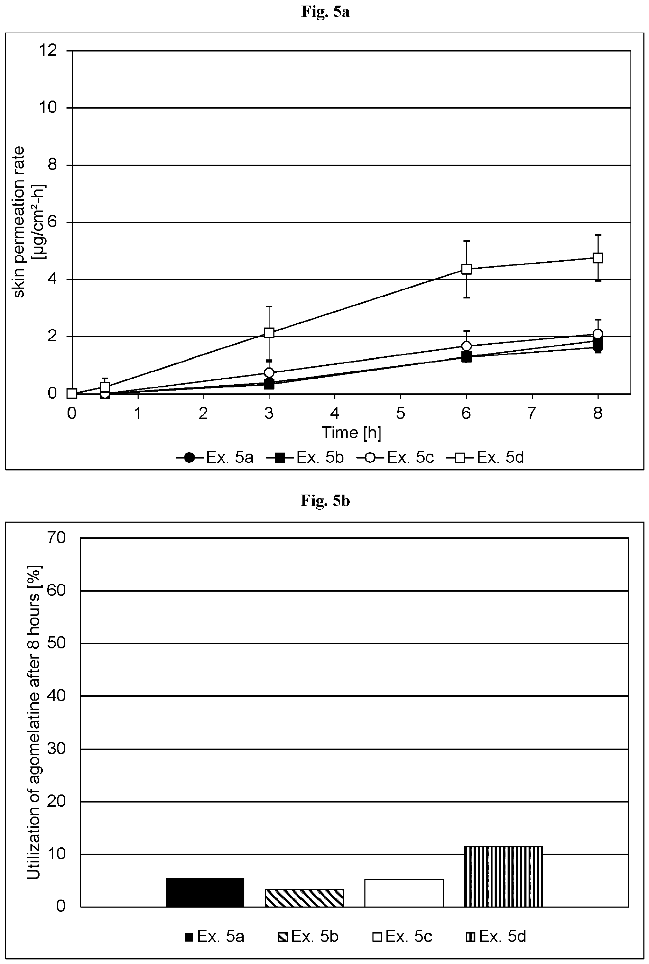

The permeated amount and the corresponding skin permeation rates of TTS prepared according to Example 5a to 5d were determined as in Example 3 above. The results are shown in Table 5.2 and Figure 5a.

**Table 5.2**

| **Skin permeation rate with SD [µg/(cm² h)]** | | | | | | | | |
|---|---|---|---|---|---|---|---|---|
| **Elapsed time [h]** | **Ex. 5a (n = 3)** | | **Ex. 5b (n = 3)** | | **Ex. 5c (n = 3)** | | **Ex. 5d (n = 3)** | |
| | **Rate** | **SD** | **Rate** | **SD** | **Rate** | **SD** | **Rate** | **SD** |
| **0.5** | - | - | - | - | - | - | 0.23 | 0.32 |
| **3** | 0.39 | 0.04 | 0.33 | 0.11 | 0.73 | 0.39 | 2.12 | 0.93 |
| **6** | 1.27 | 0.14 | 1.28 | 0.15 | 1.66 | 0.52 | 4.35 | 1.00 |
| **8** | 1.61 | 0.19 | 1.84 | 0.17 | 2.08 | 0.51 | 4.75 | 0.81 |

| | | | | | | | | |
|---|---|---|---|---|---|---|---|---|
| *: Standard deviation in this Example was, as in all other Examples, calculated based on the n-method. | | | | | | | | |

### Utilization of agomelatine

The utilization of agomelatine at 8 hours was calculated based on the cumulative permeated amount at 8 hours and the initial agomelatine content. The results are shown in Table 5.3 and in Figure 5b.

**Table 5.3**

| **Utilization of agomelatine after 8 hours [%]** | | | |
|---|---|---|---|
| **Example 5a (n = 3)** | **Example 5b (n = 3)** | **Example 5c (n = 3)** | **Example 5d (n = 3)** |
| **5.4** | **3.3** | **5.2** | **11.5** |

These Examples show that the agomelatine utilization and the release profile as determined by *in vitro* experiments is much better, both in terms of fast onset of permeation as well as absolute permeation rates, for Example 5d, which is based on a silicone-based PSA as a hydrophobic polymer, when compared to Examples 5a to 5c, which are based on a silicone acrylic hybrid polymer or polyisobutylene as a hydrophobic polymer.

### EXAMPLES 6A-6D

### Coating composition

The formulations of the agomelatine-containing coating compositions of Examples 6a to 6d are summarized in Table 6.1 below. The formulations are based on weight percent, as also indicated in Table 6.1.

**Table 6.1 (Examples 6b to 6d are not according to the claims)**

| **Ingredient (Trade Name)** | **Ex. 6a** | | **Ex. 6b** | | **Ex. 6c** | | **Ex. 6d** | |
|---|---|---|---|---|---|---|---|---|
| | **Amt [g]** | **Solids [%]** | **Amt [g]** | **Solids [%]** | **Amt [g]** | **Solids [%]** | **Amt [g]** | **Solids [%]** |
| Agomelatine | 0.2 | 2.0 | 1.1 | 10.4 | 0.2 | 2.0 | 0.2 | 2.0 |
| Silicone adhesive in ethyl acetate. Solids content of about 62.1 % by weight (Bio-PSA Q7-4202) | 15.1 | 93.0 | - | - | - | - | - | - |
| Silicone-based PSA in ethyl acetate. Solids content of about 59.0 % by weight (Bio-PSA Q7-4402) | - | - | 4.3 | 24.9 | - | - | - | - |
| Acrylic adhesive in ethyl acetate. Solids content of about 38.6 % by weight (Duro-Tak^{™} (3)87-4287) | - | - | 16.9 | 64.7 | - | - | - | - |
| Styrene isoprene styrene adhesive in n-heptane. Solid content of about 35 % (SIS-Arkon) | - | - | - | - | 26.6 | 93.0 | 25.3 | 88.1 |
| Hydroxyethyl cellulose | - | - | - | - | - | - | - | - |
| Polyvinylpyrrolidone (Povidone K90) | 0.3 | 2.5 | - | - | 0.5 | 5.0 | - | - |
| Dipropylene glycol | 0.3 | 2.5 | - | - | - | - | 1.0 | 10.0 |
| Ethanol denat. (1 % (v/v) methyl ethyl ketone) | 0.8 | - | - | - | 1.7 | - | 1.4 | - |
| Isopropyl alcohol | - | - | 1.4 | - | - | - | - | - |
| n-Heptane | - | - | - | - | 1.2 | - | 1.1 | - |
| Water VE | - | - | - | - | - | - | - | - |
| Total | 16.7 | 100.0 | 23.7 | 100.0 | 30.2 | 100.0 | 29.0 | 100.1 |
| Area weight [g/m²] | 61.2 | | 57.5 | | 58.6 | | 65.6 | |
| Agomelatine content [µg/cm²] | 121.9 | | 598.9 | | 117.3 | | 130.7 | |

### Preparation of the coating composition

For Example 6a, a beaker was loaded with agomelatine and dissolved in ethanol. The pressure sensitive adhesive (Q7-4202), the polyvinylpyrrolidone and the dipropylene glycol were added under stirring. An opaque mixture was obtained after about 3 hour.

For Example 6b, a beaker was loaded with agomelatine. The isopropyl alcohol and pressure sensitive adhesive (Q7-4402 and Duro-Tak^{™} (3)87-4287) were added. The mixture was then stirred to obtain after about 2 hours an opaque mixture.

For Examples 6c and 6d, a beaker was loaded with agomelatine. The ethanol and the pressure sensitive adhesive (SIS-Arkon) were added. The mixture was then stirred. Heptane and the polyvinylpyrrolidone (Ex. 6c) and dipropylene glycol (Ex. 6d), respectively, were added under stirring to obtain after about 1 hour an opaque, homogeneous mixture (Ex. 6c and 6d).

In addition, a new batch of Example 3c was prepared, with negligible differences in the amounts of the formulation constituents used, in accordance with the formulation of the agomelatine-containing coating composition as summarized in Table 3.1 above.

### Coating of the coating composition

The resulting agomelatine-containing coating composition of Examples 6a to 6d was coated on a polyester film (74 µm thickness, which may function as fluoropolymer coated release liner for Ex. 6a and 6b and polyethylene terephthalate film, one side siliconized, 75 µm thickness, which may function as release liner for Ex. 6c and 6d) and dried for approx. 10 min at room temperature and 10 min at 70 °C. The coating thickness gave an area weight of 61.2 /m² (Ex. 6a), 57.5 g/m² (Ex. 6b), 58.6 g/m² (Ex. 6c), and 65.6 g/m² (Ex. 6d), respectively. The dried film was laminated with a polyethylene terephthalate backing layer (23 µm thickness) to provide an agomelatine-containing self-adhesive layer structure.

For the coating of the new batch of Example 3c, see procedure for the coating of the initial batch of Example 3c as outlined above. The coating thickness gave an area weight of 64.5 g/m², resulting in an agomelatine content of 257.5 µg/cm².

### Microscopic observation

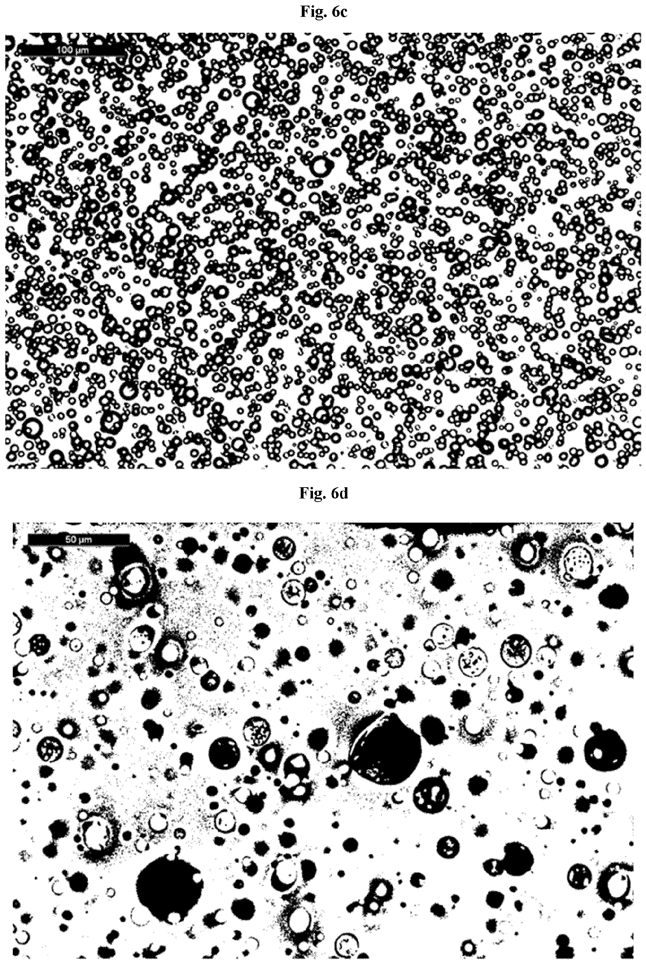

The agomelatine-containing layers of Examples 6a, 6c and 6d observed using a microscope (Leica DM6000M microscope with digital camera DFC450 and Leica Application Suite Version 4.5) shortly (several days up to 2 weeks) after manufacture. The layers of all examples showed droplets and thus were determined to be of microreservoir-type. Examples 6c and 6d (based on a styrene isoprene styrene adhesive as hydrophobic polymer) showed relatively large droplets with a maximum droplet size of about 30 and 60 µm, respectively, while the droplet size of Example 6a (based on silicone-based PSA as hydrophobic polymer) was small in comparison thereto (maximum droplet size of about 15 µm). Fig. 6c and 6d show a microscopic picture of the agomelatine-containing layers of Examples 6a and 6c, respectively.

### Preparation of the TTS

See Example 1.

### Measurement of skin permeation rate

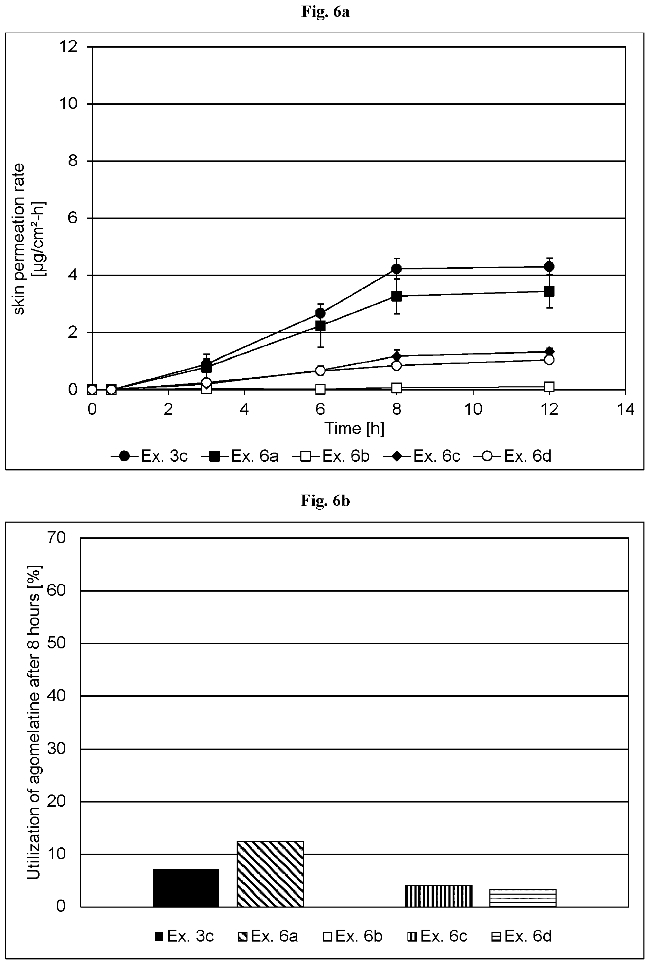

The permeated amount and the corresponding skin permeation rates of TTS prepared according to Example 6a to 6d and the new batch of Example 3c were determined as in Example 3 above. Diecuts with an area of 1.151 cm² were punched from the TTS. The results are shown in Table 6.2 and Figure 6a.

**Table 6.2**

| **Skin permeation rate with SD [µg/(cm² h)]** | | | | | | | | | | |
|---|---|---|---|---|---|---|---|---|---|---|
| **Elapsed time [h]** | **Ex. 3c (n = 3)** | | **Ex. 6a (n = 3)** | | **Ex. 6b (n = 3)** | | **Ex. 6c (n = 3)** | | **Ex. 6d (n = 3)** | |
| | **Rate** | **SD** | **Rate** | **SD** | **Rate** | **SD** | **Rate** | **SD** | **Rate** | **SD** |
| **0.5** | - | - | - | - | - | - | - | - | - | - |
| **3** | 0.89 | 0.19 | 0.79 | 0.45 | 0.04 | 0.05 | 0.20 | 0.06 | 0.25 | 0.03 |
| **6** | 2.67 | 0.33 | 2.23 | 0.75 | 0.01 | 0.02 | 0.68 | 0.15 | 0.66 | 0.09 |
| **8** | 4.22 | 0.37 | 3.26 | 0.61 | 0.07 | 0.05 | 1.16 | 0.23 | 0.84 | 0.12 |
| **12** | 4.30 | 0.30 | 3.44 | 0.58 | 0.10 | 0.01 | 1.32 | 0.12 | 1.05 | 0.11 |

| | | | | | | | | | | |
|---|---|---|---|---|---|---|---|---|---|---|
| *: Standard deviation in this Example was, as in all other Examples, calculated based on the n-method. | | | | | | | | | | |

### Utilization of agomelatine

The utilization of agomelatine at 8 hours was calculated based on the cumulative permeated amount at 8 and 12 hours and the initial agomelatine content. The results are shown in Tables 6.3 and 6.4 and in Figure 6b.

**Table 6.3**

| **Utilization of agomelatine after 8 hours [%]** | | | | |
|---|---|---|---|---|
| **Example 3c (n = 3)** | **Example 6a (n = 3)** | **Example 6b (n = 3)** | **Example 6c (n = 3)** | **Example 6d (n = 3)** |
| **7.2** | **12.5** | **0.0** | **4.1** | **3.3** |

**Table 6.4**

| **Utilization of agomelatine after 12 hours [%]** | | | | |
|---|---|---|---|---|
| **Example 3c (n = 3)** | **Example 6a (n = 3)** | **Example 6b (n = 3)** | **Example 6c (n = 3)** | **Example 6d (n = 3)** |
| 13.9 | **23.7** | **0.1** | **8.7** | **6.5** |

In Example 6b, the effect of isopropyl alcohol as a permeation enhancer in combination with a mixture of acrylate polymer and silicone-based PSA was investigated. Surprisingly, Examples 6a and 3c, which are based on a silicone-based PSA as hydrophobic polymer and include PVP as crystallization inhibitor and dipropylene glycol as a solubilizer in accordance with certain embodiments of the invention, showed a superior permeation behavior by far. Also Examples 6c and 6d, which are based on a styrene isoprene styrene adhesive as hydrophobic polymer, demonstrated much better permeation behavior and utilization, albeit not as much as Examples 6a and 3c.

### EXAMPLES 7A-7D

### Coating composition

The formulations of the agomelatine-containing coating compositions of Examples 7a to 7d and 3d' are summarized in Table 7.1 below. The formulations are based on weight percent, as also indicated in Table 7.1.

**Table 7.1**

| **Ingredient (Trade Name)** | **Ex.7a** | | **Ex. 7b** | | **Ex. 7c** | | **Ex. 7d** | |
|---|---|---|---|---|---|---|---|---|
| | **Amt [g]** | **Solids [%]** | **Amt [g]** | **Solids [%]** | **Amt [g]** | **Solids [%]** | **Amt [g]** | **Solids [%]** |
| Agomelatine | 0.2 | 2.0 | 0.2 | 2.0 | 0.1 | 2.0 | 0.2 | 4.0 |
| Silicone-based PSA in ethyl acetate. Solids content of about 60.0 % by weight (Bio-PSA Q7-4302) | 14.8 | 88.1 | 14.7 | 88.0 | 7.3 | 88.0 | 7.2 | 86.0 |
| Polyvinylpyrrolidone (Povidone K90F) | 0.5 | 4.9 | 0.5 | 5.0 | 0.3 | 5.0 | 0.5 | 10.0 |
| Brij L4 | 0.5 | 5.0 | - | - | - | - | - | - |
| Capryol 90 | - | - | - | - | 0.3 | 5.0 | - | - |
| Levulinic acid | - | - | 0.5 | 5.0 | - | - | - | - |
| Ethanol denat. (1 % (v/v) methyl ethyl ketone) | 1.8 | - | 1.8 | - | 0.8 | - | 1.4 | - |
| Total | 17.8 | 100.0 | 17.7 | 100.0 | 8.8 | 100.0 | 9.3 | 100.0 |
| Area weight [g/m²] | 51.4 | | 51.6 | | 42.7 | | 53.4 | |
| Agomelatine content [µg/cm²] | 101.7 | | 102.7 | | 85.7 | | 213.5 | |

### Preparation of the coating composition

For Example 7a, 7b, a beaker was loaded with agomelatine. The pressure sensitive adhesive (Q7-4302) and Brij L4 (Ex. 7a) and levulinic acid (Ex. 7b), respectively, were added. The mixture was then stirred. The polyvinylpyrrolidone and the ethanol were added under stirring. A clear mixture was obtained after about 1 to 2 hours of stirring.

For Example 7c, a beaker was loaded with agomelatine. The ethanol, the Capryol 90, and the pressure sensitive adhesive (Q7-4302) were added. The mixture was then stirred. The polyvinylpyrrolidone was added under stirring. An opaque mixture was obtained after about 1 hour of stirring.

For Example 7d, a beaker was loaded with agomelatine. The pressure sensitive adhesive and the ethanol (Q7-4302) were added. The mixture was then stirred. The polyvinylpyrrolidone was added under stirring. An opaque, homogeneous mixture was obtained after about 1 hour of stirring.

In addition, a new batch of Example 3d was prepared, with negligible differences in the amounts of the formulation constituents used, in accordance with the formulation of the agomelatine-containing coating composition as summarized in Table 3.1 above.

### Coating of the coating composition

The resulting agomelatine-containing coating composition of Examples 7a to 7d was coated on a polyester film (74 µm thickness, which may function as fluoropolymer coated release liner for Ex. 7a to 7d) and dried for approx. 10 min at room temperature and 10 min at 70 °C (Ex. 7a to 7d). The coating thickness gave an area weight of 51.4 /m² (Ex. 7a), 51.6 g/m² (Ex. 7b), 42.7 g/m² (Ex. 7c), and 53.4 g/m² (Ex. 7d), respectively. The dried film was laminated with a polyethylene terephthalate backing layer (19 µm thickness for Ex. 7a and 7b and 23 µm for Ex. 7c and 7d) to provide an agomelatine-containing self-adhesive layer structure.

For the coating of the new batch of Example 3d, see procedure for the coating of the initial batch of Example 3d as outlined above. The coating thickness gave an area weight of 65.3 g/m², resulting in an agomelatine content of 260.5 µg/cm².

### Microscopic observation

The agomelatine-containing layer of Example 7d as well as agomelatine-containing layers prepared analogously to Examples 7a and 7b (new batches) were observed using a microscope (Leica DM6000M microscope with digital camera DFC450 and Leica Application Suite Version 4.5) after 2 weeks to 4 weeks (new batch of Example 7a and Example 7d) and after 21 months (new batch of Example 7b) of storage. These layers showed droplets and thus were determined to be of microreservoir-type. The droplet size was relatively homogeneous and of few micrometers in size.

### Preparation of the TTS

See Example 1.

### Measurement of skin permeation rate

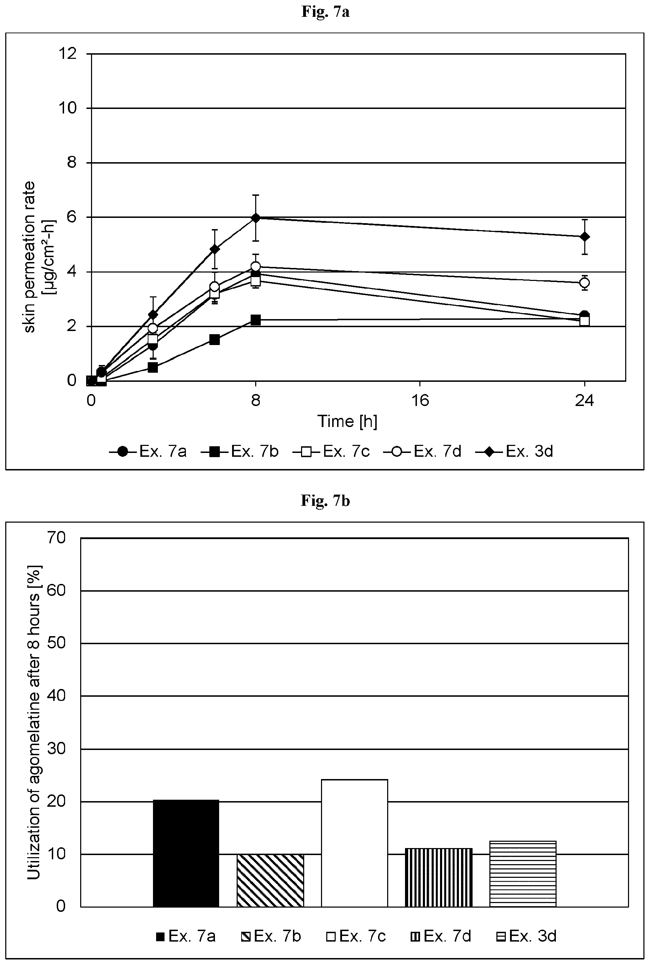

The permeated amount and the corresponding skin permeation rates of TTS prepared according to Example 7a to 7d and the new batch of Example 3d were determined as in Example 3 above. Diecuts with an area of 1.188 cm² were punched from the TTS. The results are shown in Table 7.2 and Figure 7a.

**Table 7.2**

| **Skin permeation rate with SD [µg/(cm² h)]** | | | | | | | | | | |
|---|---|---|---|---|---|---|---|---|---|---|
| **Elapsed time [h]** | **Ex. 3d (n = 3)** | | **Ex. 7a (n = 3)** | | **Ex. 7b (n = 3)** | | **Ex. 7c (n = 3)** | | **Ex. 7d (n = 3)** | |
| | **Rate** | **SD** | **Rate** | **SD** | **Rate** | **SD** | **Rate** | **SD** | **Rate** | **SD** |
| **0.5** | 0.34 | 0.22 | 0.07 | 0.09 | - | - | 0.12 | 0.11 | 0.32 | 0.10 |
| **3** | 2.42 | 0.66 | 1.30 | 0.48 | 0.50 | 0.15 | 1.52 | 0.33 | 1.91 | 0.42 |
| **6** | 4.83 | 0.72 | 3.16 | 0.27 | 1.51 | 0.17 | 3.19 | 0.36 | 3.45 | 0.54 |
| **8** | 5.97 | 0.85 | 3.92 | 0.25 | 2.23 | 0.12 | 3.66 | 0.24 | 4.18 | 0.47 |
| **24** | 5.29 | 0.64 | 2.39 | 0.13 | 2.28 | 0.05 | 2.19 | 0.11 | 3.59 | 0.26 |

| | | | | | | | | | | |
|---|---|---|---|---|---|---|---|---|---|---|
| *: Standard deviation in this Example was, as in all other Examples, calculated based on the n-method. | | | | | | | | | | |

### Utilization of agomelatine

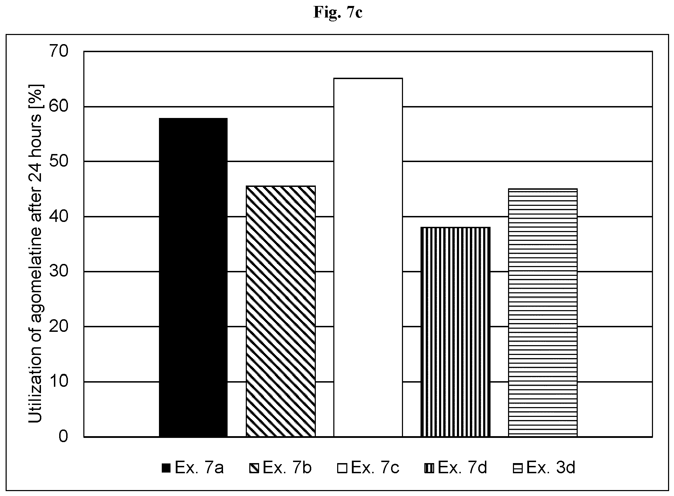

The utilization of agomelatine at 8 hours was calculated based on the cumulative permeated amount at 8 and 24 hours and the initial agomelatine content. The results are shown in Tables 7.3 and 7.4 and in Figures 7b and 7c.

**Table 7.3**

| **Utilization of agomelatine after 8 hours [%]** | | | | |
|---|---|---|---|---|
| **Example 3d (n = 3)** | **Example 7a (n = 3)** | **Example 7b (n = 3)** | **Example 7c (n = 3)** | **Example 7d (n = 3)** |
| **12.5** | **20.3** | **10.0** | **24.2** | **11.1** |

**Table 7.4**

| **Utilization of agomelatine after24 hours [%]** | | | | |
|---|---|---|---|---|
| **Example 3d (n = 3)** | **Example 7a (n = 3)** | **Example 7b (n = 3)** | **Example 7c (n = 3)** | **Example 7d (n = 3)** |
| **45.0** | **57.9** | **45.5** | **65.1** | **38.0** |

The *in vitro* experiments show a good skin permeation rate and release profile for all examples, wherein, however, the skin permeation rate and the utilization of the new batch of Example 3d using 4 wt-% agomelatine in combination with PVP as crystallization inhibitor and levulinic acid as solubilizer is much better when compared to Example 7b using the same formulation but only 2 wt-% agomelatine. The skin permeation rate and release profile is comparable for Examples 7a, 7c and 7d and in between those of Examples 7b and the new batch of Example 3d, though Example 7d, not including a solubilizer, uses 4 wt-% agomelatine and Examples 7a and 7c comprises only 2 wt-% agomelatine but include Brij or Capryol as solubilizer.

### In vivo study using Goettingen minipigs

In order to evaluate the local tolerance of agomelatine, *in vivo* experiments using *Goettingen* minipigs (female, about 6 to 7 months, body weight was 13.8-14.3 kg at the start of the study) were conducted. Diecuts with an area of 10 cm² were punched from the TTS prepared according to Examples 3d (new batch), 7a, 7b and 7c above, as well as from two further agomelatine-TTS and from six corresponding placebo TTS. One diecut of the six drug containing and of the six placebo TTS (each 10 cm²) each, totaling to twelve TTS, were used per minipig. The TTS were secured in place by a 40 cm² (6.3*6.3 cm) cover patch and the application sites were additionally covered with gauze dressing to keep the patches in place and to prevent animals from interfering with the patches. Four minipigs were used. The total wear time of all 12 patches per minipig (6 active and 6 placebo) was 12 h for animals No. 1 and 2, and 24 h for animals No. 3 and 4.

During the study, the minipigs were kept at 21 ± 3 °C, lighted from 6 am to 6 pm, SDS minipig diet (SMP (E) SQC) from Special Diets Services twice daily of about 175 g per animal, and with water *ad libitum.*

After removal of the TTS in accordance with the total wear time as outlined above, the detachment was quantified in percentage (0% to 100 %) and the general adhesion (good adhesion, easy, complete detachment) was determined qualitatively.

In addition, the skin condition was macroscopically determined and a Draize score obtained based on the score scheme below which is in accordance with the OECD Guideline for Testing of Chemicals No. 404, adopted 28th July, 2015: "Acute Dermal Irritation/Corrosion" directly after removal of the TTS as well as 12 hours post-removal.

The skin from the dose sites (complete site covered by the patches) were collected (sites 1-12), and one skin sample per animal from an untreated area was taken as a reference for the histopathological examination. Samples from all animals were trimmed and representative specimens were taken for histological processing. The specimens were embedded in paraffin and cut at a nominal thickness of 5 µm, stained with haematoxylin and eosin, and examined under a light microscope. Histopathological examination of the epidermis and the dermis revealed no agomelatine- or TTS-related findings.

The TTS adhesion (in percent adhesion area) after 12/24 hours was between 34 % and 76 % for all formulations (see Table 7.5). The reason for the lower patch adhesion area of 34 % for the TTS according to Example 3d is believed to be the TTS application site, which was located next to the minipig extremities. This is why the adhesion seems to be lower, compared to the other formulations. The adhesion of Examples 7a to 7c ranges between 60 and 76 %.

None of the formulations showed skin irritation 12 hours after TTS removal. Only directly after removal of TTS and cleaning of the application sites, the skin at the application site of the TTS according to Examples 7a and 7b show a very slight skin irritation with a Draize Score of 1 (Table 7.5). The subsequent histo-pathological assessment (12 hours after TTS removal) of the drug containing administration areas showed no API/TTS related finding. Findings recorded were considered to be within the background changes seen in the skin of Göttingen minipigs of this age and as such not to be treatment related. These results demonstrate that the risk of skin irritation potential of the inventive TTS are low.

**Table 7.5**

| Examples | **Ex. 3d'** | **Ex. 7a** | **Ex. 7b** | **Ex. 7c** |
|---|---|---|---|---|
| Ø Adhesion [%] | 34 (30/85/20/0*) | 60 (100/100/0/40*) | 60 (100/20/70/50*) | 76 (100/5/100/100*) |
| Draize** Score (after removal of TTS) | 0 / 0 / 0 / 0* | 1 0 / 0 / 0* | 0 / 0 / 1 / 0* | 0 / 0 / 0 / 0* |
| Draize** Score (12 h after removal of TTS) | 0 / 0 / 0 / 0* | 0 / 0 / 0 / 0* | 0 / 0 / 0 / 0* | 0 / 0 / 0 / 0* |
| Histo-pathological assesment | no TTS related finding | no TTS related finding | no TTS related finding | no TTS related finding |

| | | | | |
|---|---|---|---|---|
| *: Values given for minipigs No. 1 / 2 / 3 / 4, respectively. **: Score schemes for the evaluation of skin irritation potential according to Draize: 0 = No erythema, no edema, 1 = Very slight erythema (barely perceptible), very slight edema (barely perceptible), 2 = Well-defined erythema, Slight edema, 3 = Moderate to severe erythema, moderate edema, 4 = Severe erythema, severe edema. | | | | |

### EXAMPLES 8A-8D

### Coating composition

The formulations of the agomelatine-containing coating compositions of Examples 8a to 8d are summarized in Table 8.1 below. The formulations are based on weight percent, as also indicated in Table 8.1.

**Table 8.1**

| **Ingredient (Trade Name)** | **Ex.8a** | | **Ex. 8b** | | **Ex. 8c** | | **Ex. 8d** | |
|---|---|---|---|---|---|---|---|---|
| | **Amt [g]** | **Solids [%]** | **Amt [g]** | **Solids [%]** | **Amt [g]** | **Solids [%]** | **Amt [g]** | **Solids [%]** |
| Agomelatine | 2.4 | 4.0 | 10.0 | 4.0 | 2.4 | 4.0 | 12.0 | 4.0 |
| Silicone-based PSA in ethyl acetate. Solids content of about 60.0 % by weight (Bio-PSA Q7-4302) | 86.0 | 85.7 | 358.3 | 86.0 | 86.0 | 86.0 | 430.1 | 86.0 |
| Polyvinylpyrrolidone (Povidone K90F) | 3.0 | 5.0 | 12.5 | 5.0 | 6.0 | 10.0 | 30.0 | 10.0 |
| Levulinic acid | 3.2 | 5.4 | 12.5 | 5.0 | - | - | - | - |
| Ethanol denat. (1 % (v/v) methyl ethyl ketone) | 10.8 | - | 46.2 | - | 21.7 | - | 119.0 | - |
| Total | 105.4 | 100.1 | 439.5 | 100.0 | 116.1 | 100.0 | 591.1 | 100.0 |
| Area weight [g/m²] | 50.1 | | 50.0 | | 49.2 | | 52.0 | |
| Agomelatine content [µg/cm²] | 199.6 | | 200.0 | | 196.8 | | 208.0 | |

### Preparation of the coating composition

For Example 8a, a beaker was loaded with agomelatine. The ethanol, the levulinic acid and the pressure sensitive adhesive (Q7-4302) were added. The mixture was then stirred. The polyvinylpyrrolidone was added under stirring. A clear mixture was obtained after about 4 hours (Ex. 8a) of stirring.

For Example 8b, a stainless steel vessel was loaded with agomelatine. The ethanol, the levulinic acid and the pressure sensitive adhesive (Q7-4302) were added. The mixture was then stirred. The polyvinylpyrrolidone was added under stirring. A clear mixture was obtained after about 3 hours (Ex. 8b) of stirring.

For Examples 8c and 8d, a beaker was loaded with agomelatine. The pressure sensitive adhesive (Q7-4302) and the ethanol were added. The mixture was then stirred. The polyvinylpyrrolidone was added under stirring. A clear mixture was obtained after about 2 hours (Ex. 8c) and 5 hours (Ex. 8d), respectively, of stirring.

### Coating of the coating composition

The resulting agomelatine-containing coating composition of Examples 8a to 8d was coated on a polyester film (74 µm thickness, which may function as fluoropolymer coated release liner) and dried for approx. 10 min at room temperature and 10 min at 70 °C. The coating thickness gave an area weight of 50.1 /m² (Ex. 8a), 50.0 g/m² (Ex. 8b), 49.2 g/m² (Ex. 8c), and 52.0 g/m² (Ex. 8d), respectively. The dried film was laminated with a polyethylene terephthalate backing layer (19 µm thickness for Ex. 8a to 8d) to provide an agomelatine-containing self-adhesive layer structure.

### Microscopic observation

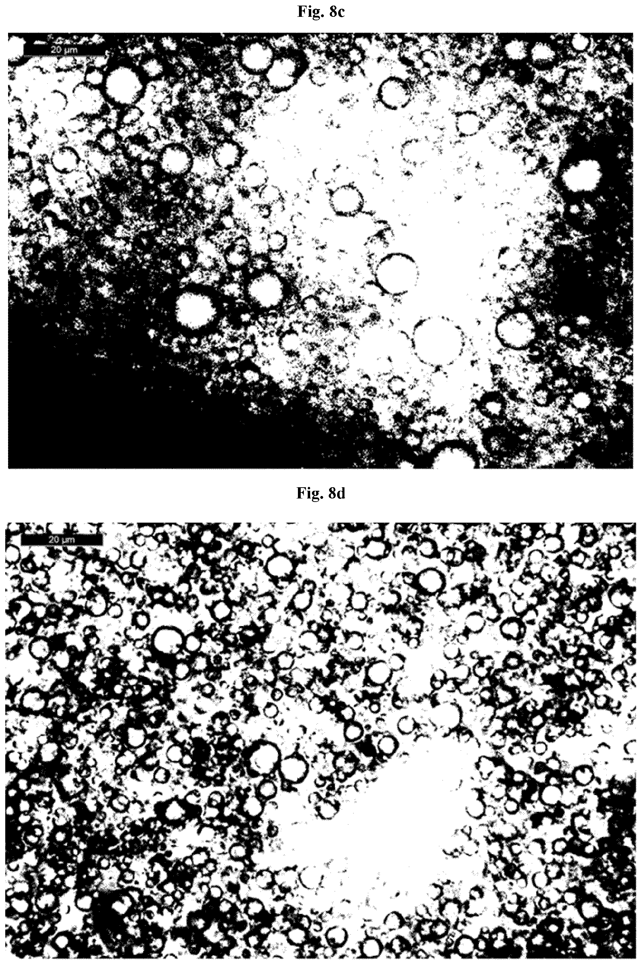

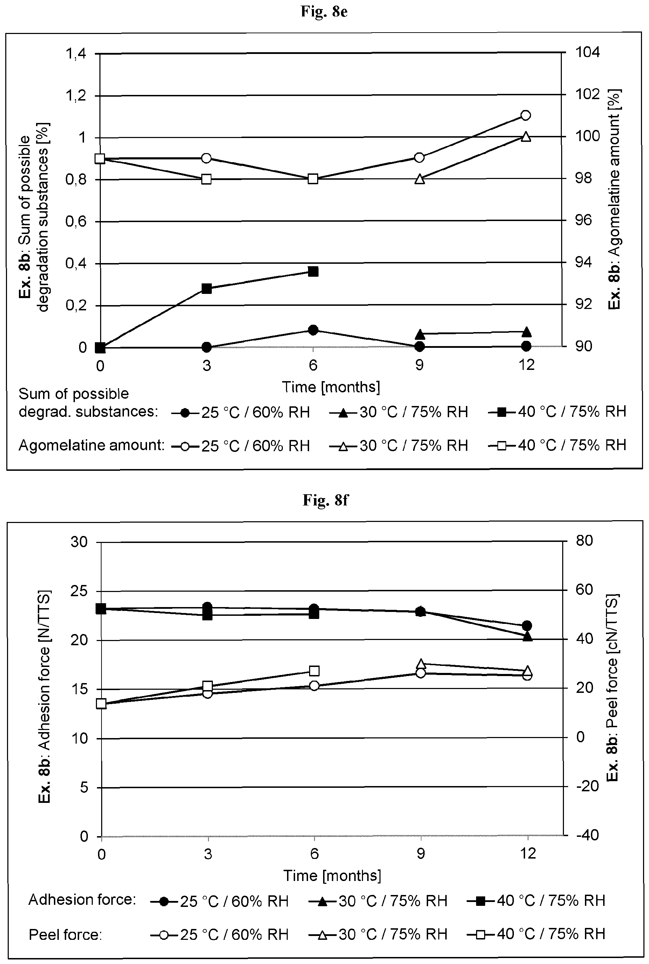

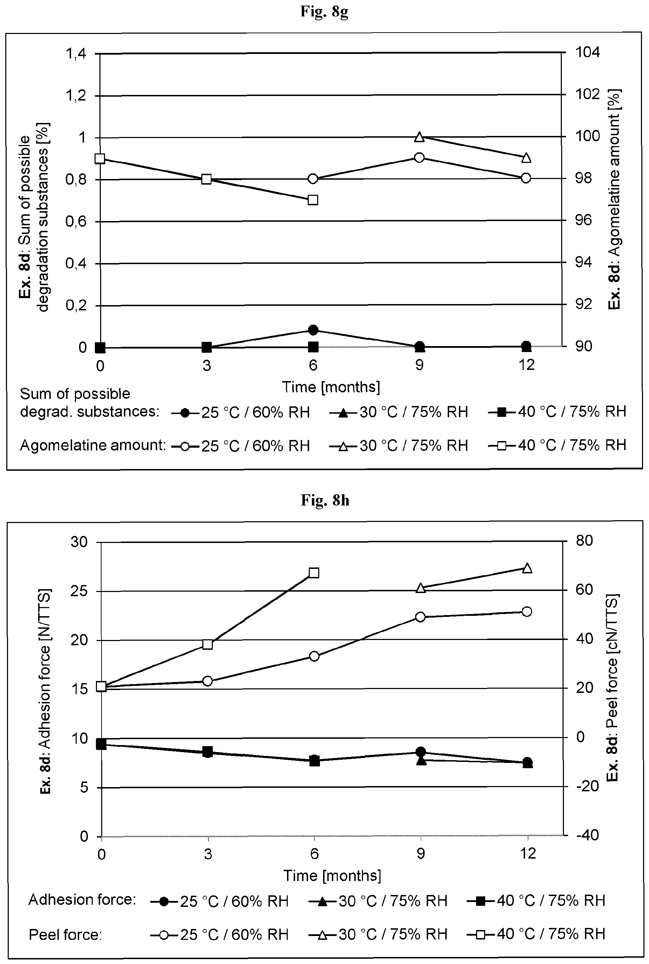

The agomelatine-containing layer of Examples 8b and 8d were observed using a microscope (Leica DM6000M microscope with digital camera DFC450 and Leica Application Suite Version 4.5) after 4 to 5 months of storage. These layers showed droplets and thus were determined to be of microreservoir-type. The droplet size was relatively homogeneous and of few micrometers in size (maximum droplet size of about 10 µm). Fig. 8c and 8d show a microscopic picture of the agomelatine-containing layers of Examples 8b and 8d, respectively

### Preparation of the TTS

See Example 1.

### Measurement of skin permeation rate

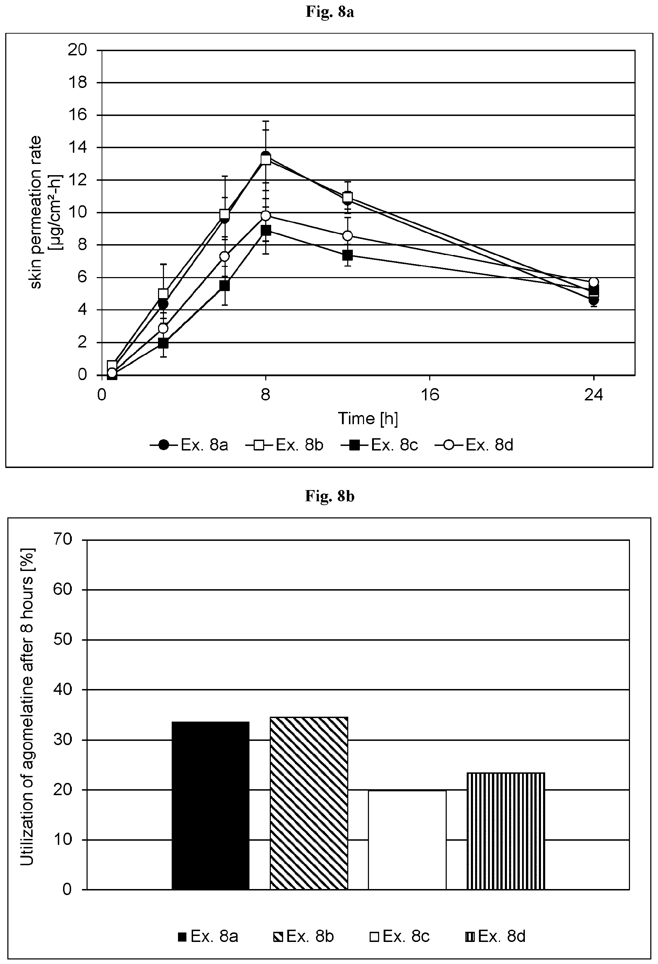

The permeated amount and the corresponding skin permeation rates of TTS prepared according to Example 8a to 8d were determined as in Example 1 above. Split thickness human skin from cosmetic surgeries (female abdomen, date of birth 1976) was used. A dermatome was used to prepare skin to a thickness of 500 µm, with an intact epidermis for all TTS. Diecuts with an area of 1.157 cm² were punched from the TTS. The results are shown in Table 8.2 and Figure 8a.

**Table 8.2**

| **Skin permeation rate with SD [µg/(cm² h)]** | | | | | | | | |
|---|---|---|---|---|---|---|---|---|
| **Elapsed time [h]** | **Ex. 8a (n = 3)** | | **Ex. 8b (n = 3)** | | **Ex. 8c (n = 3)** | | **Ex. 8d (n = 3)** | |
| | **Rate** | **SD** | **Rate** | **SD** | **Rate** | **SD** | **Rate** | **SD** |
| **0.5** | 0.35 | 0.07 | 0.57 | 0.27 | 0.03 | 0.02 | 0.13 | 0.15 |
| **3** | 4.35 | 0.87 | 4.99 | 1.83 | 1.94 | 0.83 | 2.85 | 0.98 |
| **6** | 9.63 | 1.30 | 9.90 | 2.35 | 5.49 | 1.20 | 7.29 | 1.21 |
| **8** | 13.46 | 1.63 | 13.26 | 2.39 | 8.90 | 1.45 | 9.80 | 1.56 |
| **12** | 10.75 | 0.53 | 10.93 | 0.98 | 7.36 | 0.66 | 8.56 | 1.13 |
| **24** | 4.60 | 0.16 | 5.08 | 0.86 | 5.24 | 0.05 | 5.69 | 0.23 |

| | | | | | | | | |
|---|---|---|---|---|---|---|---|---|
| *: Standard deviation in this Example was, as in all other Examples, calculated based on the n-method. | | | | | | | | |

### Utilization of agomelatine

The utilization of agomelatine at 8 hours was calculated based on the cumulative permeated amount at 8 hours and the initial agomelatine content. The results are shown in Table 8.3 and in Figure 8b.

**Table 8.3**

| **Utilization of agomelatine after 8 hours [%]** | | | |
|---|---|---|---|
| **Example 8a (n = 3)** | **Example 8b (n = 3)** | **Example 8c (n = 3)** | **Example 8d (n = 3)** |
| **33.5** | **34.5** | **19.9** | **23.4** |

The *in vitro* experiments show a good skin permeation rate and utilization for both formulations, but the addition of levulinic acid in Examples 8a/8b seem to be of more advantage when compared to the formulation of Examples 8c/8d, comprising higher amounts of PVP but no solubilizer.

### Storage stability measurements

A long term storage stability test was conducted for Examples 8b and 8d under different test conditions, i.e. storage at 25 °C and 60 % relative humidity (RH), at 30 °C and 75 % RH, and at 40 °C and 75 % RH. Samples were taken from the TTS after 3, 6, 9 and 12 months storage at 25 °C and 60 % RH, after 9 and 12 months storage at 30 °C and 75 % RH, and after 3 and 6 months storage at 40 °C and 75 % RH, and the amount of agomelatine, as well as various possible degradation substances was determined by a specific quantitative HPLC method based on the agomelatine content calculated from the (actual) area weight of the tested TTS. The adhesion force of the adhesive layer on steel plate as well as the peel force of the adhesive layer from the release liner was measured using a 45.0 mm x 45.0 mm sample, at an angle of 90 °, and a test speed of 300 mm/min for the adhesion force and 150 mm/min for the peel force. It was further tested whether removability of the adhesive layer from the release liner was assured and the compliance noted. In addition, possible occurrences of cold flow was visually inspected and compliance with still acceptable extent of cold flow noted. The results are shown in Tables 8.4 to 8.9 as well as in Figures 8e to 8h.

**Table 8.4**

| **Ex. 8b** - **25 °C / 60 % RH** | Initial | 3 months | 6 months | 9 months | 12 months |
|---|---|---|---|---|---|
| Detected amounts: | 99 | 99 | 98 | 99 | 101 |
| Agomelatine [%] | | | | | |
| Detected amounts: | n.d. | n.d. | 0.08 | n.d. | n.d. |
| Sum of possible degradation substances [%] | | | | | |
| Adhesion force [N/TTS] | 23.2 | 23.3 | 23.1 | 22.8 | 21.3 |
| Peel force [cN/TTS] | 14 | 18 | 21 | 26 | 25 |
| Removability from release liner | complies | complies | complies | complies | complies |
| Cold Flow | complies | complies | complies | complies | complies |

| | | | | | |
|---|---|---|---|---|---|
| * n.d. = not detected | | | | | |

**Table 8.5**

| **Ex. 8d** - **25 °C** / **60 % RH** | Initial | 3 months | 6 months | 9 months | 12 months |
|---|---|---|---|---|---|
| Detected amounts: | 99 | 98 | 98 | 99 | 98 |
| Agomelatine [%] | | | | | |
| Detected amounts: | n.d. | n.d. | 0.08 | n.d. | n.d. |
| Sum of possible degradation substances [%] | | | | | |
| Adhesion force [N/TTS] | 9.40 | 8.50 | 7.70 | 8.50 | 7.4 |
| Peel force [cN/TTS] | 21 | 23 | 33 | 49 | 51 |
| Removability from release liner | complies | complies | complies | complies | complies |
| Cold Flow | complies | complies | complies | complies | complies |

| | | | | | |
|---|---|---|---|---|---|
| * n.d. = not detected | | | | | |

**Table 8.6**

| **Ex. 8b** - **30 °C / 75 % RH** | Initial | 3 months | 6 months | 9 months | 12 months |
|---|---|---|---|---|---|
| Detected amounts: | 99 | N/A | N/A | 98 | 100 |
| Agomelatine [%] | | | | | |
| Detected amounts: | n.d. | N/A | N/A | 0.06 | 0.07 |
| Sum of possible degradation substances [%] | | | | | |
| Adhesion force [N/TTS] | 23.2 | N/A | N/A | 22.9 | 20.3 |
| Peel force [cN/TTS] | 14 | N/A | N/A | 30 | 27 |
| Removability from release liner | complies | N/A | N/A | complies | complies |
| Cold Flow | complies | N/A | N/A | complies | complies |

| | | | | | |
|---|---|---|---|---|---|
| * n.d. = not detected, N/A = No data | | | | | |

**Table 8.7**

| **Ex. 8d** - **30 °C** / **75 % RH** | Initial | 3 months | 6 months | 9 months | 12 months |
|---|---|---|---|---|---|
| Detected amounts: | 99 | N/A | N/A | 100 | 99 |
| Agomelatine [%] | | | | | |
| Detected amounts: | n.d. | N/A | N/A | n.d. | n.d. |
| Sum of possible degradation substances [%] | | | | | |
| Adhesion force [N/TTS] | 9.4 | N/A | N/A | 7.7 | 7.4 |
| Peel force [cN/TTS] | 21 | N/A | N/A | 61 | 69 |
| Removability from release liner | complies | N/A | N/A | complies | complies |
| Cold Flow | complies | N/A | N/A | complies | complies |

| | | | | | |
|---|---|---|---|---|---|
| * n.d. = not detected, N/A = No data | | | | | |

**Table 8.8**

| **Ex. 8b - 40 °C / 75 % RH** | Initial | 3 months | 6 months | 9 months | 12 months |
|---|---|---|---|---|---|
| Detected amounts: | 99 | 98 | 98 | N/A | N/A |
| Agomelatine [%] | | | | | |
| Detected amounts: | n.d. | 0.07 | 0.09 | N/A | N/A |
| Sum of possible degradation substances [%] | | | | | |
| Adhesion force [N/TTS] | 23.2 | 22.5 | 22.6 | N/A | N/A |
| Peel force [cN/TTS] | 14 | 21 | 27 | N/A | N/A |
| Removability from release liner | complies | complies | complies | N/A | N/A |
| Cold Flow | complies | complies | complies | N/A | N/A |

| | | | | | |
|---|---|---|---|---|---|
| * n.d. = not detected, N/A = No data | | | | | |

**Table 8.9**

| **Ex. 8d - 40 °C / 75 % RH** | Initial | 3 months | 6 months | 9 months | 12 months |
|---|---|---|---|---|---|
| Detected amounts: | 99 | 98 | 97 | N/A | N/A |
| Agomelatine [%] | | | | | |
| Detected amounts: | n.d. | n.d. | n.d. | N/A | N/A |
| Sum of possible degradation substances [%] | | | | | |
| Adhesion force [N/TTS] | 9.4 | 8.6 | 7.6 | N/A | N/A |
| Peel force [cN/TTS] | 21 | 38 | 67 | N/A | N/A |
| Removability from release liner | complies | complies | complies | N/A | N/A |
| Cold Flow | complies | complies | complies | N/A | N/A |

| | | | | | |
|---|---|---|---|---|---|
| * n.d. = not detected, N/A = No data | | | | | |

The stability data show that the initial as well as storage stability is excellent for Examples 8b and 8d, both in terms of the amount of agomelatine (in particular with respect to the amount of agomelatine remaining after storage) as well as the sum of possible degradation substances.

The adhesion force is also maintained at a good level over time with only a slight decrease.

Although peel force slightly increases over time, the increase is well within an acceptable range and removability from the release liner was assured at all time. No blocking of the release liner or delamination of the adhesive layer is expected.

All in all, a long shelf life of e.g. 12 or 24 months or longer can be assumed.

### EXAMPLES 9A-9E

### Coating composition

The formulations of the agomelatine-containing coating compositions of Examples 9a to 9g are summarized in Table 9.1 below. The formulations are based on weight percent, as also indicated in Table 9.1.

**Table 9.1 (Examples 9a, 9b, 9d and 9e are not according to the claims)**

| **Ingredient (Trade Name)** | **Ex. 9a** | | **Ex. 9b** | | **Ex. 9c** | | **Ex. 9d** | | **Ex. 9e** | |
|---|---|---|---|---|---|---|---|---|---|---|
| | **Amt [g]** | **Solids [%]** | **Amt [g]** | **Solids [%]** | **Amt [g]** | **Solids [%]** | **Amt [g]** | **Solids [%]** | **Amt [g]** | **Solids [%]** |
| Agomelatine | 0.2 | 4.0 | 0.2 | 3.8 | 0.2 | 2.0 | 0.4 | 4.0 | 0.8 | 2.1 |
| Silicone-based PSA in n-heptane. Solids content of about 72.7 % by weight (Bio-PSA Q7-4301) | - | - | - | - | - | - | - | - | 48.5 | 92.9 |
| Silicone-based PSA in ethyl acetate. Solids content of about 62.1 % by weight (Bio-PSA Q7-4202) | - | - | - | - | 15.4 | 95.5 | - | - | - | - |
| Silicone acrylic hybrid PSA adhesive in n-heptane. Solid content of about 51.0 % by weight (SilAc PSA 7-6301) | 8.9 | 91.0 | - | - | - | - | - | - | - | - |
| Polyisobutylene adhesive in petroleum benzine, bp 80 110 °C . Solid content of about 41.0 % by weight (Oppanol B10/B100 85/15) | - | - | 11.8 | 91.4 | - | - | - | - | - | - |
| Styrene isoprene styrene adhesive in n-heptane. Solids content of about 35 % (SIS-Arkon) | - | - | - | - | - | - | 26.2 | 91.1 | - | - |
| Polyvinylpyrrolidone (Povidone K90) | 0.3 | 5.0 | 0.3 | 4.8 | 0.3 | 2.5 | 0.5 | 5.0 | - | - |
| Dipropylene glycol | - | - | - | - | - | - | - | - | 1.9 | 5.0 |
| Ethanol denat. (1 % (v/v) methyl ethyl ketone) | 1.5 | - | 0.8 | - | 0.8 | - | 2.0 | - | - | - |
| Ethyl acetate | - | - | 0.8 | - | - | - | - | - | 2.7 | - |
| n-heptane | - | - | - | - | - | - | 2.0 | - | - | - |
| Total | 10.9 | 100.0 | 13.9 | 100.0 | 16.7 | 100.0 | 31.1 | 100.1 | 53.9 | 100.0 |

### Preparation of the coating composition

For Example 9a, a beaker was loaded with agomelatine. The ethanol and the polyvinylpyrrolidone were added and the mixture was allowed to stand for 3 hours. The silicone acrylic hybrid PSA adhesive was added and the mixture stirred at 1000 rpm. A white homogeneous mixture was obtained.

For Example 9b, a beaker was loaded with agomelatine. The ethanol and the pressure sensitive adhesive (Q7-4202) were added and the mixture stirred. The polyvinylpyrrolidone was added under stirring and the mixture further stirred. A turbid homogeneous solution was obtained.

For Example 9c, a beaker was loaded with agomelatine. The polyisobutylene adhesive and the ethyl acetate were added and the mixture was allowed to stand for 1 hour. The polyvinylpyrrolidone was added, the mixture stirred at 250 rpm, and the ethanol added after 0.5 hours. A clear solution with visible small bubbles was obtained.

For Example 9d, a beaker was loaded with agomelatine and dissolved in 1.5 g of the ethanol. The polyvinylpyrrolidone was added and the mixture was allowed to stand for ~ 1 hour. The styrene isoprene styrene adhesive was added and the mixture stirred at 200 rpm. Finally, the remainder of the ethanol and the n-heptane were added and the mixture stirred at 200 rpm. A slightly turbid solution without visible crystals was obtained.

For Example 9e, a beaker was loaded with agomelatine. The dipropylene glycol was added and the mixture stirred. The ethyl acetate and the pressure sensitive adhesive (Q7-4301) were added. The mixture was then further stirred. A clear solution was obtained after about 2.5 hours of stirring.

### Coating of the coating composition

The resulting agomelatine-containing coating composition of Examples 9a to 9d was coated on a polyester film (74 µm thickness, which may function as fluoropolymer coated release liner) and dried for approx. 10 min at room temperature and 10 min at 70 °C. The coating thickness gave an area weight of 45.5 /m² (Ex. 9a), 47.0 g/m² (Ex. 9b), 57.6 g/m² (Ex. 9c), and 50.4 g/m² (Ex. 9d), respectively. The dried film was laminated with a polyethylene terephthalate backing layer (23 µm thickness for Ex. 9a, 9b, 9c and 9d) to provide an agomelatine-containing self-adhesive layer structure.

### Microscopic observation

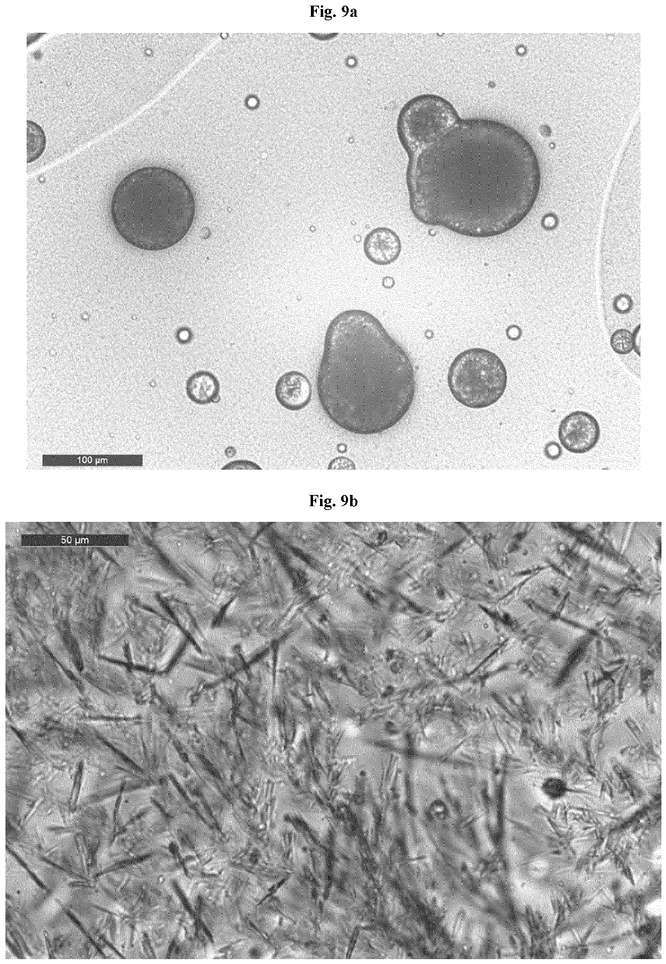

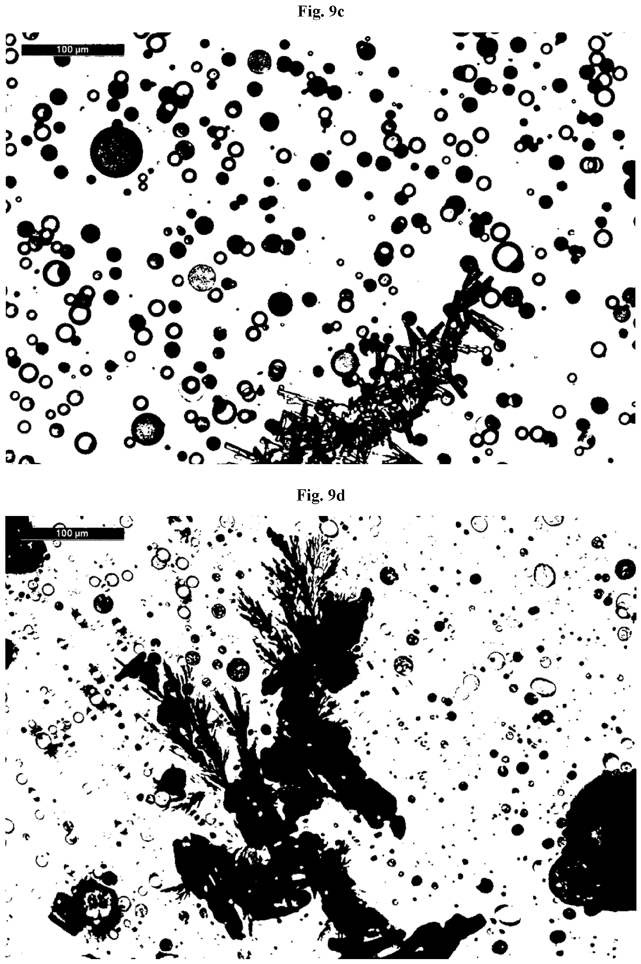

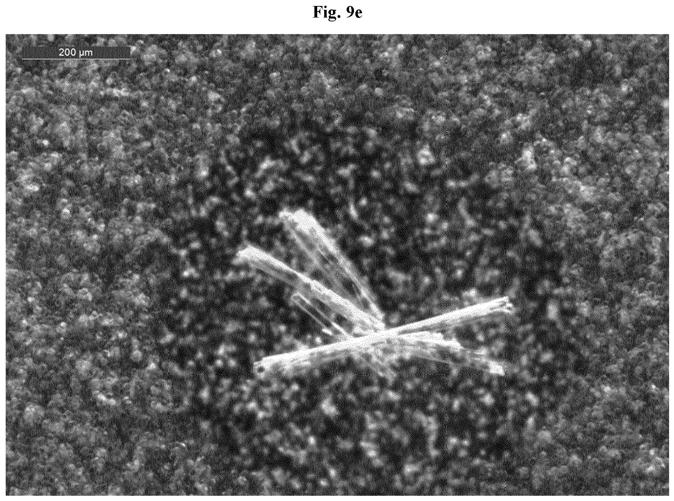

The agomelatine-containing layer of Examples 9a to 9e were observed using a microscope (Leica DM6000M microscope with digital camera DFC450 and Leica Application Suite Version 4.5) directly after (1 to 3 days) manufacture (Examples 9a and 9b) or upon about 2 weeks (Example 9c), 3 weeks (Example 9d) or 2.5 months (Example 9e) of storage. All these layers contained crystals. Fig. 9a to 9e show a microscopic picture of the agomelatine-containing layers of Examples 9a to 9e, respectively

### Preparation of the TTS

See Example 1.

## Claims

1. Transdermal therapeutic system for the transdermal administration of agomelatine comprising a self-adhesive layer structure containing a therapeutically effective amount of agomelatine, said self-adhesive layer structure comprising:
A) a backing layer; and
B) an agomelatine-containing layer comprising:
i) agomelatine;
ii) a hydrophobic polymer; and
iii) at least 1 wt-% of a crystallization inhibitor selected from the group consisting of polyvinylpyrrolidone and polyvinylpyrrolidone-polyvinylacetate copolymer;
wherein
the hydrophobic polymer is selected from pressure-sensitive adhesives based on polysiloxanes.

2. Transdermal therapeutic system according to claim 1,
wherein the agomelatine-containing layer is of a microreservoir-type.

3. Transdermal therapeutic system according to claim 1 or 2,
wherein the agomelatine-containing layer comprises at least 1.5 wt-%, at least 2.5 wt-%, at least 4 wt-%, or at least 5 wt-% of the crystallization inhibitor, and/or
wherein the crystallization inhibitor is polyvinylpyrrolidone, and/or
wherein the crystallization inhibitor is selected from soluble polyvinylpyrrolidones.

4. Transdermal therapeutic system according to any one of claims 1 to 3,
wherein the agomelatine-containing layer comprises a solubilizer selected from the group consisting of dipropylene glycol, lauryl lactate, mixtures of propylene glycol monoesters and diesters of fatty acids, levulinic acid, polyethylene glycol ethers and diethylene glycol monoethyl ether.

5. Transdermal therapeutic system according to claim 4,
wherein the agomelatine-containing layer comprises at least 1.5 wt-%, at least 2.5 wt-%, at least 4 wt-% or at least 5 wt-% of the solubilizer.

6. Transdermal therapeutic system according to any one of claims 1 to 3, wherein the agomelatine-containing layer does not comprise a solubilizer selected from the group consisting of dipropylene glycol, lauryl lactate, mixtures of propylene glycol monoesters and diesters of fatty acids, levulinic acid, polyethylene glycol ethers and diethylene glycol monoethyl ether.

7. Transdermal therapeutic system according to any one of claims 1 to 6,
wherein the agomelatine-containing layer comprises a crystallization inhibitor and the total amount of crystallization inhibitor and solubilizer present in the agomelatine-containing layer is at least 2.5 wt-%, at least 3.5 wt-%, at least 4 wt-% or at least 5 wt-%, and/or
wherein the ratio of the total amount of crystallization inhibitor and solubilizer present in the agomelatine-containing layer to the amount of agomelatine present in the agomelatine-containing layer is at least 1 : 2, at least 1 : 1, or at least 2:1.

8. Transdermal therapeutic system according to any one of claims 1 to 7,
wherein the agomelatine-containing layer comprises from 0.5 to less than or equal to 8 wt-% agomelatine, from 1 to less than or equal to 6 wt-% agomelatine, or from 1.5 to less than or equal to 5 wt-% agomelatine, and/or
wherein the agomelatine-containing layer is free of agomelatine crystals, and/or
wherein the agomelatine-containing layer has an area weight of at least 25 g/m², at least 35 g/m², or at least 40 g/m², or has an area weight of less than or equal to 150 g/m², less than or equal to 120 g/m², or less than or equal to 90 g/m², or has an area weight of from 25 to 150 g/m², from 35 to 120 g/m², or from 40 to 90 g/m².

9. Transdermal therapeutic system according to any one of claims 1 to 8, wherein the amount of the hydrophobic polymer is at least 75 wt-%, at least 80 wt-% or at least 75 wt-%, or the amount of the hydrophobic polymer is less than or equal to 98 wt-%, less than or equal to 94 wt-% or less than or equal to 90 wt-%, or the amount of the hydrophobic polymer ranges from 75 to 98 wt-%, from 80 to 94 wt-%, or from 85 to 90 wt-% of the agomelatine-containing layer.

10. Transdermal therapeutic system according to any one of claims 1 to 9,
wherein the agomelatine-containing layer is a dried biphasic layer having
a) an outer phase having a pressure-sensitive adhesive composition comprising the hydrophobic polymer, and
b) an inner phase having a composition comprising the agomelatine,
wherein the inner phase forms dispersed deposits in the outer phase.

11. Transdermal therapeutic system according to any one of claims 1 to 10,
providing a skin permeation rate of agomelatine as measured in a Franz diffusion cell with dermatomed human skin of
0.5 µg/cm²-hr to 15 µg/cm²-hr at hour 2,
1 µg/cm²-hr to 20 µg/cm²-hr at hour 4,
2 µg/cm²-hr to 25 µg/cm²-hr at hour 8, and
1 µg/cm²-hr to 15 µg/cm²-hr at hour 16,
and/or
providing a cumulative permeated amount of agomelatine as measured in a Franz diffusion cell with dermatomed human skin of at least 0.01 mg/cm², at least 0.015 mg/cm² or at least 0.02 mg/cm², or less than or equal to 0.2 mg/cm², less than or equal to 0.15 mg/cm², or less than or equal to 0.1 mg/cm², or of from 0.01 mg/cm² to 0.2 mg/cm², from 0.015 mg/cm² to 0.15 mg/cm² or from 0.02 mg/cm² to 0.1 mg/cm² at hour 8.

12. Transdermal therapeutic system according to any one of claims 1 to 11 for use in a method of treatment.

13. Transdermal therapeutic system for use according to claim 12 in a method of treating major depression,
wherein the transdermal therapeutic system is applied to the skin of a human patient and maintained on the skin for at least 2 hours, at least 4 hours or at least 6 hours, or for less than or equal to 24 hours, less than or equal to 18 hours, or less than or equal to 14 hours, or for 2 to 24 hours, for 4 to 18 hours, or for 6 to 14 hours.

14. Process of manufacture of an agomelatine-containing layer comprising the steps of:
i) combining at least agomelatine, a hydrophobic polymer, and a crystallization inhibitor selected from the group consisting of polyvinylpyrrolidone and polyvinylpyrrolidone-polyvinylacetate copolymer in a solvent to obtain a coating composition;
ii) coating the coating composition onto a backing layer or a release liner or any intermediate liner; and
iii) drying the coated coating composition to form the agomelatine-containing layer,
wherein
the hydrophobic polymer is selected from pressure-sensitive adhesives based on polysiloxanes.

15. Transdermal therapeutic system for the transdermal administration of agomelatine comprising a self-adhesive layer structure containing a therapeutically effective amount of agomelatine, said self-adhesive layer structure comprising:
A) a backing layer;
and
B) an agomelatine-containing layer comprising:
i) 2 to 6 wt-% agomelatine;
ii) a hydrophobic polymer;
iii) from 2 to 7 wt-% polyvinylpyrrolidone; and
iv) from 2 to 7 wt-% of a permeation enhancer selected from levulinic acid and polyethylene glycol ethers;
or
B) an agomelatine-containing layer comprising:
i) 2 to 6 wt-% agomelatine;
ii) a hydrophobic polymer; and
iii) from 7 to 15 wt-% polyvinylpyrrolidone;
wherein
the hydrophobic polymer is selected from pressure-sensitive adhesives based on polysiloxanes and
wherein the area weight of the agomelatine-containing layer ranges from 35 to 70 g/m²

## Patentansprüche

1. Transdermales therapeutisches System zur transdermalen Verabreichung von Agomelatin, umfassend eine selbstklebende Schichtstruktur, die eine therapeutisch wirksame Menge von Agomelatin enthält, wobei die genannte selbstklebende Schichtstruktur umfasst:
A) eine Rückschicht; und
B) eine agomelatinhaltige Schicht, umfassend:
i) Agomelatin;
ii) ein hydrophobes Polymer; und
iii) mindestens 1 Gew.-% eines Kristallisationsinhibitors, ausgewählt aus der Gruppe bestehend aus Polyvinylpyrrolidon und Polyvinylpyrrolidon-Polyvinylacetat-Copolymer;
wobei
das hydrophobe Polymer aus Haftklebstoffen auf der Basis von Polysiloxanen ausgewählt ist.

2. Das transdermale therapeutische System nach Anspruch 1,
wobei die agomelatinhaltige Schicht vom Typ eines Mikroreservoirs ist.

3. Das transdermale therapeutische System nach Anspruch 1 oder 2,
wobei die agomelatinhaltige Schicht mindestens 1,5 Gew.-%, mindestens 2,5 Gew.-%, mindestens 4 Gew.-% oder mindestens 5 Gew.-% des Kristallisationsinhibitors umfasst, und/oder
wobei der Kristallisationsinhibitor Polyvinylpyrrolidon ist, und/oder wobei der Kristallisationsinhibitor aus löslichen Polyvinylpyrrolidonen ausgewählt ist.

4. Das transdermale therapeutische System nach einem der Ansprüche 1 bis 3, wobei die agomelatinhaltige Schicht einen Lösungsvermittler enthält, ausgewählt aus der Gruppe bestehend aus Dipropylenglykol, Lauryllactat, Mischungen von Propylenglykolmonoestern und -diestern von Fettsäuren, Levulinsäure, Polyethylenglykolethern und Diethylenglykolmonoethylether.

5. Das transdermale therapeutische System nach Anspruch 4,
wobei die agomelatinhaltige Schicht mindestens 1,5 Gew.-%, mindestens 2,5 Gew.-%, mindestens 4 Gew.-% oder mindestens 5 Gew.-% des Lösungsvermittlers umfasst.

6. Das transdermale therapeutische System nach einem der Ansprüche 1 bis 3, wobei die agomelatinhaltige Schicht keinen Lösungsvermittler enthält, der ausgewählt ist aus der Gruppe bestehend aus Dipropylenglykol, Lauryllactat, Mischungen von Propylenglykolmonoestern und Diestern von Fettsäuren, Levulinsäure, Polyethylenglykolethern und Diethylenglykolmonoethylether.

7. Das transdermale therapeutische System nach einem der Ansprüche 1 bis 6, wobei die agomelatinhaltige Schicht einen Kristallisationsinhibitor umfasst und die Gesamtmenge an Kristallisationsinhibitor und Lösungsvermittler, die in der agomelatinhaltigen Schicht vorhanden ist, mindestens 2,5 Gew.-%, mindestens 3,5 Gew.-%, mindestens 4 Gew.-% oder mindestens 5 Gew.-% beträgt, und/oder wobei das Verhältnis der Gesamtmenge an Kristallisationsinhibitor und Lösungsvermittler, die in der agomelatinhaltigen Schicht vorhanden ist, zu der Menge an Agomelatin, die in der agomelatinhaltigen Schicht vorhanden ist, mindestens 1 : 2, mindestens 1 : 1 oder mindestens 2 : 1 beträgt.

8. Das transdermale therapeutische System nach einem der Ansprüche 1 bis 7, wobei die agomelatinhaltige Schicht von 0,5 bis weniger als oder gleich 8 Gew.-% Agomelatin, von 1 bis weniger als oder gleich 6 Gew.-% Agomelatin oder von 1,5 bis weniger als oder gleich 5 Gew.-% Agomelatin umfasst, und/oder wobei die agomelatinhaltige Schicht frei von Agomelatin-Kristallen ist, und/oder wobei die agomelatinhaltige Schicht ein Flächengewicht von mindestens 25 g/m², mindestens 35 g/m² oder mindestens 40 g/m² oder ein Flächengewicht von weniger als oder gleich 150 g/m², weniger als oder gleich 120 g/m² oder weniger als oder gleich 90 g/m² oder ein Flächengewicht von 25 bis 150 g/m², von 35 bis 120 g/m² oder von 40 bis 90 g/m² aufweist.

9. Das transdermale therapeutische System nach einem der Ansprüche 1 bis 8, wobei die Menge des hydrophoben Polymers mindestens 75 Gew.-%, mindestens 80 Gew.-% oder mindestens 75 Gew.-% beträgt, oder die Menge des hydrophoben Polymers weniger als oder gleich 98 Gew.-%, weniger als oder gleich 94 Gew.-% oder weniger als oder gleich 90 Gew.-% beträgt, oder die Menge des hydrophoben Polymers im Bereich von 75 bis 98 Gew.-%, von 80 bis 94 Gew.-% oder von 85 bis 90 Gew.-% der agomelatinhaltigen Schicht liegt.

10. Das transdermale therapeutische System nach einem der Ansprüche 1 bis 9, wobei die agomelatinhaltige Schicht eine getrocknete zweiphasige Schicht ist, die
a) eine äußere Phase mit einer das hydrophobe Polymer enthaltenden Haftklebstoffzusammensetzung enthält, und
b) eine innere Phase mit einer das Agomelatin enthaltenden Zusammensetzung enthält,
wobei die innere Phase dispergierte Depots in der äußeren Phase bildet.

11. Das transdermale therapeutische System nach einem der Ansprüche 1 bis 10, bereitstellend eine Hautpermeationsrate von Agomelatin, gemessen in einer Franz-Diffusionszelle mit dermatomierter menschlicher Haut von
0,5 µg/cm²-h bis 15 µg/cm²-h bei Stunde 2,
1 µg/cm²-h bis 20 µg/cm²-h bei Stunde 4,
2 µg/cm²-h bis 25 µg/cm²-h bei Stunde 8 und
1 µg/cm²-Stunde bis 15 µg/cm²-Stunde bei Stunde 16,
und/oder
bereitstellend eine kumulative Permeationsmenge von Agomelatin, gemessen in einer Franz-Diffusionszelle mit dermatomierter menschlicher Haut, von mindestens 0,01 mg/cm², mindestens 0,015 mg/cm² oder mindestens 0,02 mg/cm², oder weniger als oder gleich 0.2 mg/cm², weniger als oder gleich 0,15 mg/cm² oder weniger als oder gleich 0,1 mg/cm² oder von 0,01 mg/cm² bis 0,2 mg/cm², von 0,015 mg/cm² bis 0,15 mg/cm² oder von 0,02 mg/cm² bis 0,1 mg/cm² bei Stunde 8.

12. Das transdermale therapeutische System nach einem der Ansprüche 1 bis 11 zur Anwendung in einem Verfahren zur Behandlung.

13. Das transdermale therapeutische System zur Anwendung nach Anspruch 12 in einem Verfahren zur Behandlung einer schweren Depression,
wobei das transdermale therapeutische System auf die Haut eines menschlichen Patienten aufgebracht wird und mindestens 2 Stunden, mindestens 4 Stunden oder mindestens 6 Stunden oder weniger als oder gleich 24 Stunden, weniger als oder gleich 18 Stunden oder weniger als oder gleich 14 Stunden oder 2 bis 24 Stunden, 4 bis 18 Stunden oder 6 bis 14 Stunden auf der Haut verbleibt.

14. Verfahren zur Herstellung einer agomelatinhaltigen Schicht, umfassend die folgenden Schritte:
i) Zusammengeben von mindestens Agomelatin, einem hydrophoben Polymer und einem Kristallisationsinhibitor, ausgewählt aus der Gruppe bestehend aus Polyvinylpyrrolidon und Polyvinylpyrrolidon-Polyvinylacetat-Copolymer in einem Lösungsmittel, um eine Beschichtungszusammensetzung zu erhalten;
ii) Auftragen der Beschichtungszusammensetzung auf eine Rückschicht, eine abziehbare Schicht oder irgendeine intermediäre Schicht; und
iii) Trocknen der beschichteten Beschichtungszusammensetzung zur Bildung der agomelatinhaltigen Schicht,
wobei
das hydrophobe Polymer aus Haftklebstoffen auf der Basis von Polysiloxanen ausgewählt ist.

15. Transdermales therapeutisches System zur transdermalen Verabreichung von Agomelatin, umfassend eine selbstklebende Schichtstruktur, die eine therapeutisch wirksame Menge von Agomelatin enthält, wobei die selbstklebende Schichtstruktur umfasst:
A) eine Rückschicht;
und
B) eine agomelatinhaltige Schicht, umfassend:
i) 2 bis 6 Gew.-%- Agomelatin;
ii) ein hydrophobes Polymer;
iii) von 2 bis 7 Gew.-% Polyvinylpyrrolidon; und
iv) von 2 bis 7 Gew.-% eines Permeationsverstärkers, ausgewählt aus Levulinsäure und Polyethylenglykolethern;
oder
B) eine agomelatinhaltige Schicht, umfassend:
i) 2 bis 6 Gew.-% Agomelatin;
ii) ein hydrophobes Polymer; und
iii) von 7 bis 15 Gew.-% Polyvinylpyrrolidon;
wobei
das hydrophobe Polymer aus Haftklebstoffen auf der Basis von Polysiloxanen ausgewählt ist und
wobei das Flächengewicht der agomelatinhaltigen Schicht zwischen 35 und 70 g/m² liegt.

## Revendications

1. Système thérapeutique transdermique pour l'administration transdermique d'agomélatine comprenant une structure de couches auto-adhésives contenant une quantité efficace du point de vue thérapeutique d'agomélatine, ladite structure de couches auto-adhésives comprenant :
A) une couche support, et
B) une couche contenant de l'agomélatine comprenant:
i) agomélatine
ii) un polymère hydrophobe, et
iii) au moins 1% en poids d'un inhibiteur de cristallisation sélectionné dans le groupe consistant en polyvinylpyrrolidone et copolymère de polyvinylpyrrolidone-acétate de polyvinyl ;
dans lequel le polymère hydrophobe est sélectionné parmi les adhésifs sensibles à la pression à base de polysiloxanes.

2. Système thérapeutique transdermique selon la revendication 1, dans lequel la couche contenant l'agomélatine est du type microréservoir.

3. Système thérapeutique transdermique selon la revendication 1 ou 2, dans lequel la couche contenant l'agomélatine comprend au moins 1,5% en poids, au moins 2,5% en poids, au moins 4% en poids, ou au moins 5% en poids d'un inhibiteur de cristallisation et/ou dans lequel l'inhibiteur de cristallisation consiste en polyvinylpyrrolidone et/ou dans lequel l'inhibiteur de cristallisation est sélectionné parmi les polyvinylpyrrolidones solubles.

4. Système thérapeutique transdermique selon l'une quelconque des revendications 1 à 3, dans lequel la couche contenant l'agomélatine comprend un solubilisant sélectionné dans le groupe consistant en dipropylène glycol, lactate de lauryl, mélanges de monoesters de propylène glycol et de diesters d'acides gras, acide lévulinique, éthers de polyéthylène glycol et monoéthyl éther de diéthylène glycol.

5. Système thérapeutique transdermique selon la revendication 4, dans lequel la couche contenant l'agomélatine comprend au moins 1,5% en poids, au moins 2,5% en poids, au moins 4% en poids ou au moins 5% en poids de solubilisant.

6. Système thérapeutique transdermique selon l'une quelconque des revendications 1 à 3, dans lequel la couche contenant l'agomélatine est exempte de solubilisant sélectionné dans le groupe consistant en dipropylène glycol, lactate de lauryl, mélanges de monoesters de propylène glycol et de diesters d'acides gras, acide lévulinique, éthers de polyéthylène glycol et monoéthyl éther de diéthylène glycol.

7. Système thérapeutique transdermique selon l'une quelconque des revendications 1 à 6, dans lequel la couche contenant l'agomélatine comprend un inhibiteur de cristallisation et la quantité totale d'inhibiteur de cristallisation et de solubilisant présents dans la couche contenant l'agomélatine est d'au moins 2,5% en poids, d'au moins 3,5% en poids, d'au moins 4% en poids ou d'au moins 5% en poids, et/ou dans lequel le rapport quantité totale d'inhibiteur de cristallisation et de solubilisant présents dans la couche contenant l'agomélatine/quantité d'agomélatine présente dans la couche contenant l'agomélatine est d'au moins 1/2, au moins 1/l ou au moins 2/1.

8. Système thérapeutique transdermique selon l'une quelconque des revendications 1 à 7,
dans lequel la couche contenant l'agomélatine contient de 0,5% à moins de 8% ou à 8% en poids d'agomélatine, de 1 à moins de 6% ou à 6% en poids d'agomélatine, de 1,5 à moins de 5% ou à 5% en poids d'agomélatine, et/ou dans lequel la couche contenant l'agomélatine est exempte de cristaux d'agomélatine et/ou
dans lequel la couche contenant l'agomélatine présente un poids surfacique d'au moins 25 g/m², d'au moins 35 g/m², ou d'au moins 40 g/m² ou un poids surfacique inférieur ou égal à 150 g/m² ou inférieur ou égal à 120 g/m², ou inférieur ou égal à 90 g/m² ou présente un poids surfacique compris entre 25 et 150 g/m², entre 35 et 120 g/m² ou entre 40 et 90 g/m².

9. Système thérapeutique transdermique selon l'une quelconque des revendications 1 à 8, dans lequel la quantité de polymère hydrophobe est d'au moins 75% en poids, d'au moins 80% en poids ou d'au moins 75 % en poids ou la quantité de polymère hydrophobe est inférieure ou égale à 98% en poids, inférieure ou égale à 94% en poids ou inférieure ou égale à 90% en poids, ou la quantité de polymère hydrophobe est comprise entre 75 et 98% en poids, entre 80 et 94% en poids, ou entre 85 et 90% en poids de la couche contenant l'agomélatine.

10. Système thérapeutique transdermique selon l'une quelconque des revendications 1 à 9, dans lequel la couche contenant l'agomélatine est une couche biphasique séchée comportant :
a) une phase externe ayant une composition adhésive sensible à la pression comprenant le polymère hydrophobe, et
b) une phase interne ayant une composition comprenant l'agomélatine, dans laquelle la phase interne forme des dépôts dispersés dans la phase externe.

11. Système thérapeutique transdermique selon l'une quelconque des revendications 1 à 10, fournissant un taux de pénétration cutanée de l'agomélatine tel que mesuré par une cellule de diffusion de Franz sur une peau humaine dermatomée de:
0.5 µg/cm²-hr à 15 µg/cm²-hr après 2 heures,
1 µg/cm²-hr à 20 µg/cm²-hr après 4 heures,
2 µg/cm²-hr à 25 µg/cm²-hr après 8 heures, et
1 µg/cm²-hr à 15 µg/cm²-hr après 16 heures,
et/ou
fournissant un taux de pénétration cumulatif d'agomélatine tel que mesuré par une cellule de diffusion de Franz sur une peau humaine dermatomée d'au moins 0,01 mg/cm², d'au moins 0,015 mg/cm² ou d'au moins 0,02 mg/cm² , ou inférieure ou égale à 0,2 mg/cm², inférieure ou égale à 0,15 mg/cm², ou inférieure ou égale à 0,1 mg/cm², ou de 0,01 mg/cm² à 0,2 mg/cm², de 0,015 mg/cm² à 0,15 mg/cm² ou de 0,02 mg/cm² à 0,1 mg/cm² après 8 heures.

12. Système thérapeutique transdermique selon l'une quelconque des revendications 1 à 11, pour utilisation dans un procédé de traitement.

13. Système thérapeutique transdermique pour utilisation selon la revendication 12, dans un procédé de traitement d'une dépression majeure,
dans lequel le système thérapeutique transdermique est appliqué sur la peau d'un patient humain et maintenu sur la peau pendant au moins 2 heures, au moins 4 heures ou au moins 6 heures, ou pendant une durée égale ou inférieure à 24 heures, inférieure ou égale à 18 heures, ou inférieure ou égale à 14 heures, ou de 2 à 24 heures, 4 à 18 heures, ou 6 à 14 heures.

14. Procédé de fabrication d'une couche contenant de l'agomélatine comprenant les étapes consistant à :
i) combiner au moins de l'agomélatine, un polymère hydrophobe, et un inhibiteur de cristallisation sélectionné dans le groupe consistant en polyvinylpyrrolidone et copolymère polyvinylpyrrolidone-polyvinylacétate dans un solvant pour obtenir une composition de revêtement;
ii) revêtir d'une couche support ou d'un revêtement antiadhésif ou de tout autre revêtement intermédiaire; et
iii) sécher la composition de revêtement revêtue pour former la couche contenant l'agomélatine, le polymère hydrophobe étant sélectionné parmi les adhésifs sensibles à la pression à base de polysiloxanes.

15. Système thérapeutique transdermique pour l'administration transdermique d'agomélatine comprenant une structure de couches auto-adhésives contenant une quantité efficace du point de vue thérapeutique d'agomélatine, ladite structure de couches auto-adhésives comprenant :
A) une couche support,
et
B) une couche contenant de l'agomélatine comprenant:
i) 2 à 6% en poids d'agomélatine
ii) un polymère hydrophobe
iii) de 2 à 7% en poids de polyvinylpirroolidone, et
iv) de 2 à 7% en poids d'un amplificateur de perméation choisi parmi l'acide levulinique et les éthers de polyéthylène glycol
ou
B) une couche contenant de l'agomélatine comprenant:
i) 2 à 6% en poids d'agomélatine
ii) un polymère hydrophobe
iii) de 7 à 15 % en poids de polyvinylpyrrolidone,
dans lequel le polymère hydrophobe est sélectionné parmi les adhésifs à base de polysiloxanes sensibles à la pression et
dans lequel le poids surfacique de la couche contenant l'agomélatine est compris entre 35 et 70 g/m².
